# EUROPEAN PATENT APPLICATION

(11) **EP 2 100 965 A1**
(43) Date of publication of application: **16.09.2009**
(21) Application number: 08290237.0
(22) Date of filing: 12.03.2008
(51) Int. Cl.: C12P 19/12, C12P 19/26, C07H 15/10

(54) **Mutants of glycoside hydrolases and uses thereof for synthesizing complex oligosaccharides and disaccharide intermediates**

(71) Applicant: Institut Pasteur, 75724 Paris Cedex 15 (FR)
(72) Inventor: Mulard, Laurence, 94270 Le Kremlin Bicetre (FR); Andre, Isabelle, 31400 Toulouse (FR); Champion, Elise, 31400 Toulouse (FR); Moulis, Claire, 31380 Garidech (FR); Morel, Sandrine, 31320 Auzeville (FR); Monsan, Pierre, 31700 Mondonville (FR); Remaud-Siméon, Magali, 31520 Ramonville (FR); Descroix, Karine, 92240 Malakoff (FR)
(74) Representative: Rançon, Xavier Lucien Abel

(57) **Abstract**

Method for preparing the disaccharide α-D-glucopyranosyl-(1→4)-2-*N*-acetyl-2-deoxy-α-D-glucopyranoside, comprising the step of using a mutant of a wild type glycoside hydrolase.

SEQUENCE LISTING

<110> Institut Pasteur

<120> MUTANTS OF GLYCOSIDE HYDROLASES AND USES THEREOF FOR SYNTHESIZING
 COMPLEX OLIGOSACCHARIDES AND DISACCHARIDE INTERMEDIATES

<130> F226EP151

<160> 27

<170> PatentIn version 3.3

<210> 1
<211> 11
<212> PRT
<213> Artificial

<220>
<223> Consenus motif (1)

<400> 1

Leu Gly Val Asn Tyr Leu His Leu Met Pro Leu
1               5                   10

<210> 2
<211> 6
<212> PRT
<213> Artificial

<220>
<223> Consensus motif (2)

<400> 2

Asp Gly Gly Tyr Ala Val
1               5

<210> 3
<211> 6
<212> PRT
<213> Artificial

<220>
<223> Consenus motif (3)

<400> 3

Asp Phe Val Phe Asn His
1               5

<210> 4
<211> 13
<212> PRT
<213> Artificial

<220>
<223> Consenus motif (4)

<400> 4

Leu Arg Glu Ile Phe Pro Asp Thr Ala Pro Gly Asn Phe
1               5                   10

<210>  5
<211>  9
<212>  PRT
<213>  Artificial

<220>
<223>  Consensus motfi (5)

<400>  5

Phe Asn Ser Tyr Gln Trp Asp Leu Asn
1               5


<210>  6
<211>  12
<212>  PRT
<213>  Artificial

<220>
<223>  Consensus motif (6)

<400>  6

Ile Leu Arg Leu Asp Ala Val Ala Phe Leu Trp Lys
1               5                   10


<210>  7
<211>  4
<212>  PRT
<213>  Artificial

<220>
<223>  Consenus motif (7)

<400>  7

Glu Ala Ile Val
1


<210>  8
<211>  8
<212>  PRT
<213>  Artificial

<220>
<223>  Consenus motif (8)

<400>  8

Tyr Val Arg Cys His Asp Asp Ile
1               5


<210>  9
<211>  11
<212>  PRT
<213>  Artificial

<220>
<223>  Consensus motif (9)

<400>  9

Arg Ile Ser Gly Thr Leu Ala Ser Leu Ala Gly

1                   5                   10


<210>  10
<211>  10
<212>  PRT
<213>  Artificial

<220>
<223>  Consensus motif (9)

<400>  10

Gly Ile Pro Leu Ile Tyr Leu Gly Asp Glu
1               5                   10


<210>  11
<211>  6
<212>  PRT
<213>  Artificial

<220>
<223>  Consenus motif (10)

<400>  11

Arg Trp Val His Arg Pro
1               5


<210>  12
<211>  85
<212>  PRT
<213>  Artificial

<220>
<223>  Consensus sequence

<400>  12

Leu Gly Val Asn Tyr Leu His Leu Met Pro Leu Asp Gly Gly Tyr Ala
1               5                   10                  15

Val Asp Phe Val Phe Asn His Leu Arg Glu Ile Phe Pro Asp Thr Ala
            20                  25                  30

Pro Gly Asn Phe Phe Asn Ser Tyr Gln Trp Asp Leu Asn Ile Leu Arg
        35                  40                  45

Leu Asp Ala Val Ala Phe Leu Trp Lys Glu Ala Ile Val Tyr Val Arg
    50                  55                  60

Cys His Asp Asp Ile Gly Ile Pro Leu Ile Tyr Leu Gly Asp Glu Arg
65                  70                  75                  80

Trp Val His Arg Pro
                85


<210>  13
<211>  628
<212>  PRT

<213>  Neisseria polysaccharea

<400>  13

Ser Pro Asn Ser Gln Tyr Leu Lys Thr Arg Ile Leu Asp Ile Tyr Thr
1               5                   10                  15

Pro Glu Gln Arg Ala Gly Ile Glu Lys Ser Glu Asp Trp Arg Gln Phe
            20                  25                  30

Ser Arg Arg Met Asp Thr His Phe Pro Lys Leu Met Asn Glu Leu Asp
        35                  40                  45

Ser Val Tyr Gly Asn Asn Glu Ala Leu Leu Pro Met Leu Glu Met Leu
    50                  55                  60

Leu Ala Gln Ala Trp Gln Ser Tyr Ser Gln Arg Asn Ser Ser Leu Lys
65                  70                  75                  80

Asp Ile Asp Ile Ala Arg Glu Asn Asn Pro Asp Trp Ile Leu Ser Asn
                85                  90                  95

Lys Gln Val Gly Gly Val Cys Tyr Val Asp Leu Phe Ala Gly Asp Leu
            100                 105                 110

Lys Gly Leu Lys Asp Lys Ile Pro Tyr Phe Gln Glu Leu Gly Leu Thr
        115                 120                 125

Tyr Leu His Leu Met Pro Leu Phe Lys Cys Pro Glu Gly Lys Ser Asp
    130                 135                 140

Gly Gly Tyr Ala Val Ser Ser Tyr Arg Asp Val Asn Pro Ala Leu Gly
145                 150                 155                 160

Thr Ile Gly Asp Leu Arg Glu Val Ile Ala Ala Leu His Glu Ala Gly
                165                 170                 175

Ile Ser Ala Val Val Asp Phe Ile Phe Asn His Thr Ser Asn Glu His
            180                 185                 190

Glu Trp Ala Gln Arg Cys Ala Ala Gly Asp Pro Leu Phe Asp Asn Phe
        195                 200                 205

Tyr Tyr Ile Phe Pro Asp Arg Arg Met Pro Asp Gln Tyr Asp Arg Thr
    210                 215                 220

Leu Arg Glu Ile Phe Pro Asp Gln His Pro Gly Gly Phe Ser Gln Leu
225                 230                 235                 240

Glu Asp Gly Arg Trp Val Trp Thr Thr Phe Asn Ser Phe Gln Trp Asp
                245                 250                 255

Leu Asn Tyr Ser Asn Pro Trp Val Phe Arg Ala Met Ala Gly Glu Met
            260                 265                 270

Leu Phe Leu Ala Asn Leu Gly Val Asp Ile Leu Arg Met Asp Ala Val
        275                 280                 285

Ala Phe Ile Trp Lys Gln Met Gly Thr Ser Cys Glu Asn Leu Pro Gln
    290                 295                 300

Ala His Ala Leu Ile Arg Ala Phe Asn Ala Val Met Arg Ile Ala Ala
305                 310                 315                 320

Pro Ala Val Phe Phe Lys Ser Glu Ala Ile Val His Pro Asp Gln Val
                325                 330                 335

Val Gln Tyr Ile Gly Gln Asp Glu Cys Gln Ile Gly Tyr Asn Pro Leu
            340                 345                 350

Gln Met Ala Leu Leu Trp Asn Thr Leu Ala Thr Arg Glu Val Asn Leu
        355                 360                 365

Leu His Gln Ala Leu Thr Tyr Arg His Asn Leu Pro Glu His Thr Ala
    370                 375                 380

Trp Val Asn Tyr Val Arg Ser His Asp Asp Ile Gly Trp Thr Phe Ala
385                 390                 395                 400

Asp Glu Asp Ala Ala Tyr Leu Gly Ile Ser Gly Tyr Asp His Arg Gln
                405                 410                 415

Phe Leu Asn Arg Phe Phe Val Asn Arg Phe Asp Gly Ser Phe Ala Arg
            420                 425                 430

Gly Val Pro Phe Gln Tyr Asn Pro Ser Thr Gly Asp Cys Arg Val Ser
        435                 440                 445

Gly Thr Ala Ala Ala Leu Val Gly Leu Ala Gln Asp Asp Pro His Ala
    450                 455                 460

Val Asp Arg Ile Lys Leu Leu Tyr Ser Ile Ala Leu Ser Thr Gly Gly
465                 470                 475                 480

Leu Pro Leu Ile Tyr Leu Gly Asp Glu Val Gly Thr Leu Asn Asp Asp
                485                 490                 495

Asp Trp Ser Gln Asp Ser Asn Lys Ser Asp Asp Ser Arg Trp Ala His
            500                 505                 510

Arg Pro Arg Tyr Asn Glu Ala Leu Tyr Ala Gln Arg Asn Asp Pro Ser
        515                 520                 525

Thr Ala Ala Gly Gln Ile Tyr Gln Asp Leu Arg His Met Ile Ala Val
    530                 535                 540

Arg Gln Ser Asn Pro Arg Phe Asp Gly Gly Arg Leu Val Thr Phe Asn
545                 550                 555                 560

Thr Asn Asn Lys His Ile Ile Gly Tyr Ile Arg Asn Asn Ala Leu Leu
                565                 570                 575

Ala Phe Gly Asn Phe Ser Glu Tyr Pro Gln Thr Val Thr Ala His Thr
            580                 585                 590

Leu Gln Ala Met Pro Phe Lys Ala His Asp Leu Ile Gly Gly Lys Thr
        595                 600                 605

Val Ser Leu Asn Gln Asp Leu Thr Leu Gln Pro Tyr Gln Val Met Trp
    610                 615                 620

Leu Glu Ile Ala
625

<210>  14
<211>  29
<212>  DNA
<213>  Artificial

<220>
<223>  primer

<220>
<221>  misc_feature
<222>  (13)..(15)
<223>  n is a, c, g, or t

<400>  14
accctgcgcg aannnttccc cgaccagca                                          29

<210>  15
<211>  29
<212>  DNA
<213>  Artificial

<220>
<223>  primer

<220>
<221>  misc_feature
<222>  (15)..(17)
<223>  n is a, c, g, or t

<400>  15
tgctggtcgg ggaannnttc gcgcagggt                                          29

<210>  16
<211>  33
<212>  DNA
<213>  Artificial

<220>
<223>  primer


<220>
<221>  misc_feature
<222>  (14)..(16)
<223>  n is a, c, g, or t

<400>  16
tatggatgcg gttnnnttta tttggaaaca aat                                    33


<210>  17
<211>  33
<212>  DNA
<213>  Artificial

<220>
<223>  primer


<220>
<221>  misc_feature
<222>  (18)..(20)
<223>  n is a, c, g, or t

<400>  17
atttgtttcc aaataaannn aaccgcatcc ata                                    33


<210>  18
<211>  33
<212>  DNA
<213>  Artificial

<220>
<223>  primer


<220>
<221>  misc_feature
<222>  (17)..(19)
<223>  n is a, c, g, or t

<400>  18
tatggatgcg gttgccnnna tttggaaaca aat                                    33


<210>  19
<211>  33
<212>  DNA
<213>  Artificial

<220>
<223>  primer


<220>
<221>  misc_feature
<222>  (15)..(17)
<223>  n is a, c, g, or t

<400>  19
atttgtttcc aaatnnnggc aaccgcatcc ata                                    33

<210>  20
<211>  34
<212>  DNA
<213>  Artificial

<220>
<223>  primer


<220>
<221>  misc_feature
<222>  (15)..(17)
<223>  n is a, c, g, or t

<400>  20
tcaaatccga agccnnngtc caccccgacc aagt                                    34


<210>  21
<211>  34
<212>  DNA
<213>  Artificial

<220>
<223>  primer


<220>
<221>  misc_feature
<222>  (21)..(23)
<223>  n is a, c, g, or t

<400>  21
acttggtcgg ggtggacgat nnnttcggat ttga                                    34


<210>  22
<211>  34
<212>  DNA
<213>  Artificial

<220>
<223>  primer


<220>
<221>  misc_feature
<222>  (18)..(20)
<223>  n is a, c, g, or t

<400>  22
tcaaatccga agccatcnnn caccccgacc aagt                                    34


<210>  23
<211>  34
<212>  DNA
<213>  Artificial

<220>
<223>  primer


<220>
<221>  misc_feature
<222>  (15)..(17)
<223>  n is a, c, g, or t

<400>  23
acttggtcgg ggtgnnngat ggcttcggat ttga                                34

<210>  24
<211>  32
<212>  DNA
<213>  Artificial

<220>
<223>  primer

<220>
<221>  misc_feature
<222>  (15)..(17)
<223>  n is a, c, g, or t

<400>  24
tccgcagcca cgacnnnatc ggctggacgt tt                                32

<210>  25
<211>  32
<212>  DNA
<213>  Artificial

<220>
<223>  primer

<220>
<221>  misc_feature
<222>  (16)..(18)
<223>  n is a, c, g, or t

<400>  25
aaacgtccag ccgatnnngt cgtggctgcg ga                                32

<210>  26
<211>  27
<212>  DNA
<213>  Artificial

<220>
<223>  primer

<220>
<221>  misc_feature
<222>  (13)..(15)
<223>  n is a, c, g, or t

<400>  26
acaggcgact gcnnngtcag tggtaca                                27

<210>  27
<211>  27
<212>  DNA
<213>  Artificial

<220>
<223>  primer

<220>

<221>  misc_feature
<222>  (13)..(15)
<223>  n is a, c, g, or t

<400>  27
tgtaccactg acnnngcagt cgcctgt                                          27

## Description

The present invention relates to mutants of glycoside hydrolases and uses thereof in chemo-enzymatic synthesis of complex oligosaccharides, in particular fragments of *S*. *flexneri* 1a and 1b O-antigen.

Carbohydrates displayed at the surface of cells and pathogens are involved in a wide range of biological processes, among which several intercellular recognition events, as well as host-pathogen interactions possibly resulting in microbial or viral infections. The understanding of the molecular events involved in carbohydrate-mediated interactions has long been impaired by the difficult access to relevant oligosaccharides and glycoconjugates in pure form and sufficient amounts. The exquisite diversity of possible structures, varying in monosaccharide composition, linkage and branching pattern (ref. 1), is indeed a major roadblock to easy availability. In recent years however, important developments in the preparation of carbohydrate derivatives, based on (i) multistep chemical synthesis, (ii) enzymatic strategies using recombinant glycosyltransferases (i.e., enzymes catalyzing the transfer of a monosaccharide residue from an activated sugar phosphate to an acceptor molecule; EC 2.4) with *in situ* regeneration of sugar nucleotides, (iii) combinations thereof, or (iv) biosynthesis using metabolically engineered cell-factories (ref. 2), have opened the way to significant progress in the fields of glycobiology and glycotherapeutics (ref. 3-5). A number of efficient and elegant synthetic methods such as one-pot oligosaccharide synthesis (ref. 6) or automated synthetic tools (ref. 7) have been developed to provide more straightforward access to structurally-defined carbohydrates. The use of lightly protected precursors and the regioselective one-pot protection of monosaccharides were recently emphasized (ref. 8).

Nevertheless, despite accomplished advancements, chemical approaches towards specific usable microbial oligosaccharides still need considerable effort. They remain, for the most part, highly dependent on the design of appropriate combinations of multiple protection, deprotection, and efficient glycosylation steps, which often involve numerous tedious chromatographic separations (ref. 9). Avoiding the need for protecting groups, organisms engineered to express several glycosyltransferase genes have been used to produce a nice range of biologically active complex carbohydrates, but they remain to date limited to the synthesis of short oligosaccharides which can passively cross the cell membrane (ref. 2, 10).

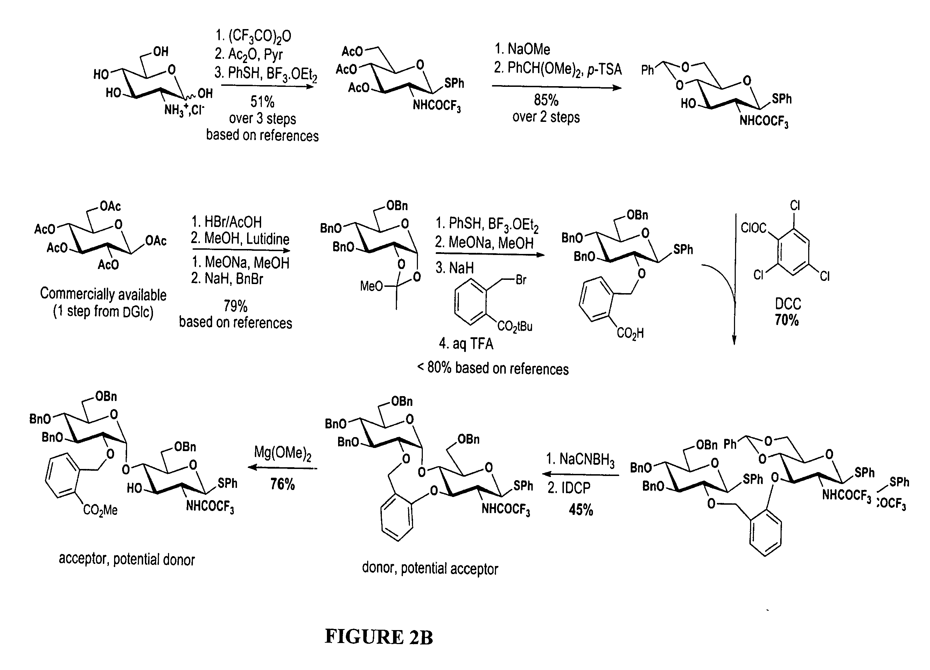

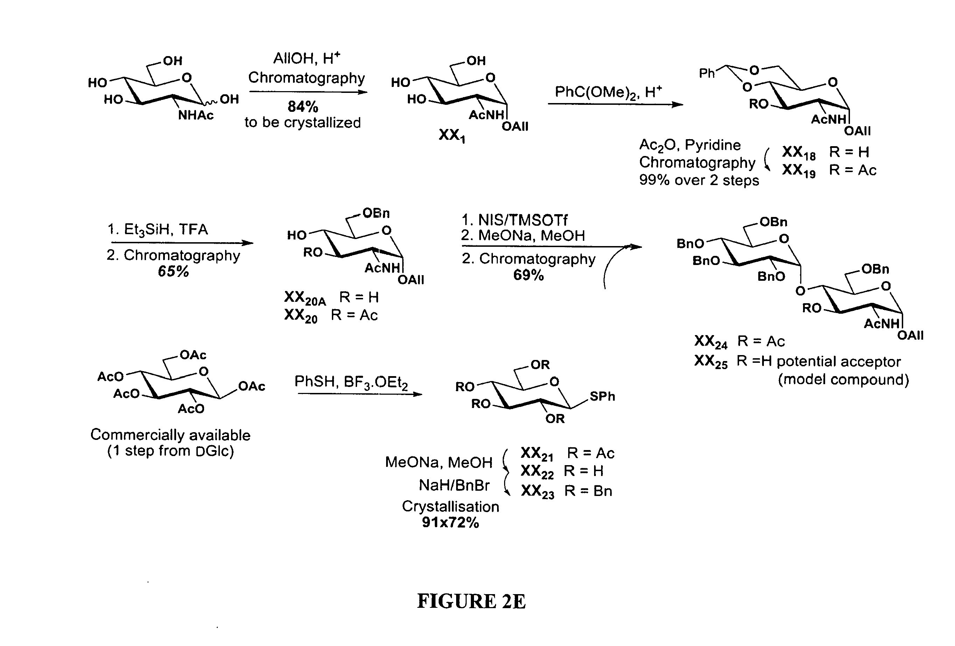

Following the early success of polysaccharide vaccines in the second half of the 20^{th} century, polysaccharide-protein conjugate vaccines were seen as a major progress in antibacterial vaccination (ref. 11, 12). Indeed, made from bacterial polysaccharides purified from pathogen cell cultures, eventually shortened following partial-chemical hydrolysis or enzymatic depolymerisation of the native antigen, and subsequently covalently coupled to a protein carrier, these second generation carbohydrate vaccines are suitable for use in human (ref. 12). Potential extrapolations are numerous since for a large number of pathogens, surface carbohydrates behave as key "protective antigens". Interestingly, this long known property of a range of bacterial polysaccharides was extended in recent years to other microbial carbohydrates of fungal (ref. 13) and parasitic origin (ref. 14). Besides, the disclosure that cancer cells could among other features be differentiated from healthy ones by the presence of surface glycoconjugates, often termed tumor-associated carbohydrate antigens, contributed to additional interest in carbohydrate antigens (ref. 15). Overall, interest in synthetic carbohydrate-based vaccines has emerged as one amongst the many exploding fields of carbohydrate medical applications (ref. 15, 16). The development of synthetic microbial carbohydrate-protein conjugates, thus termed third generation carbohydrate vaccines, was proposed as an alternative to conventional polysaccharide vaccines, compatible with the increasingly demanding requirements in terms of safety, efficiency, product definition, and needs for multivalent vaccines (ref. 17). Most interestingly, use of the natural antigen, and consequently risks associated to materials of biological origin, are avoided. However, chemical synthesis of carbohydrate-protein conjugates, more precisely of appropriate carbohydrate haptens can be seen as a drawback. By way of example, Figure 2B shows the first steps of a known chemical synthetic route to *S*. *flexneri* 1a O-antigen (ref. 66). In order to control the 1,2-cis glycosidic linkage involved in the key disaccharide motif α-D-glucopyranosyl-(1→4)-β-D-2-*N*-acetyl-2-deoxy-glucopyranosyl, the synthesis relies on the recently developed method of intramolecular glycosylation through prearranged unsymmetrically tethered glycosides. This attractive strategy involves a thioethyl glucopyranoside bearing an hydroxyl group at position 2 differentiated from the others in order to allow introduction of the tether, next linked at position 3 of the glucosamine acceptor. Subsequent deblocking at position 4 of the glucosamine residue provided the donor/acceptor bis(glycoside). Intramolecular glycosylation gave the (1→4)-α-D-glucosidic linkage only, providing a suitable disaccharide donor in 45% yield over two steps. Next, transesterification at position 3 of the glucosamine residue provided a disaccharide acceptor in 34% yield over three steps. As expected, the glycosylation step was both highly stereoselective and high yielding (72%, α-anomer only). A limitation of the strategy, however, is the 8 step-synthesis of the required tethered donor from glucose, added to the 5 steps synthesis of the acceptor. More recently, an alternative was proposed on a model disaccharide (K. Descroix & L. Mulard, unpublished). In this case (Figure 2E), the construction of the α-D-glucopyranosyl-(1→)-α-D-2-*N*-acetyl-2-deoxy-glucopyranosyl motif involves a more conventional glycosylation step between the phenyl tetra-*O*-benzyl-1thio-β-D-glucopyranoside donor **(XX₂₁)** and allyl 2-acetamido-3-*O*-acetyl-6-*O*-benzyl-2-deoxy-α-D-glucopyranoside **(XX₂₀)** as acceptor. Interestingly, the disaccharide acceptor **XX₂₅,** bearing the required 1,2-cis stereochemistry was obtained in 69% yield over two steps indicating a good stereoselectivity of the glucosylation step despite the absence of any participating group at position 2 of the donor. Nevertheless, preparation of disaccharide **XX₂₅** from the free monosaccharide precursors required a total of 10 synthetic steps combined to 3 purifications.

The use of enzymes as catalysts has then emerged as a practical alternative to a number of limitations encountered in chemical synthesis (ref. 18, 19). Leloir-type glycosyltransferases and transglycosidases constitute the two major classes of enzymes that can be used for the synthesis of glycosidic linkages. Both are enzymes transferring a glycosyl group from a donor to an acceptor. Glycosyltransferases require nucleotide sugar as donor substrate whereas transglycosidases usually employ monoand/or oligosaccharides as donor substrates.

The term "donor" refers to a molecule that provides a glycosyl moiety which will be transferred to an acceptor molecule.

The term "acceptor" refers to a molecule that will receive the glycosyl moiety through the formation of a chemical bond, preferentially C-O-linkage.

However, despite the increasing number of available transglycosidases and glycosyltransferases (Leloir type), the lack of appropriate enzymatic tools with requisite substrate specificity has prevented extensive exploration of the chemo-enzymatic strategies when dealing with complex bacterial carbohydrate antigens. The use of multiple overexpressed native glycosyltransferases was shown to be highly rewarding for the synthesis of the upstream pentasaccharide terminus of the *Neisseria meningitidis* lipo-oligosaccharide (ref. 20), but examples of enzymatic synthesis of complex carbohydrate remain scarce (ref. 21). Indeed, certain membrane Leloir-type glycosyltransferases are not easily available. Their nucleotide activated sugar substrates are expensive. They may be generated *in situ*, but the process necessitates additional enzymes (ref. 21).

Replacement of glycosyltransferases by transglycosidases has been proposed to proceed from different types of glycosyl donors, and to be compatible with a larger variety of acceptors (ref. 22). Interestingly, modified donors were occasionally used successfully (ref. 23). Nonetheless, the availability of these enzymes is often critical, especially when considering the appropriate regio- and stereospecificity required for a given target (ref. 22, 24 and 25).

Protein engineering based on rational, semi-rational or fully combinatorial approaches (directed evolution) has also proven to be extremely useful to generate catalysts with improved natural properties but also to create new substrate specificities (ref. 26, 27). In the field of carbohydrate-enzymes, glycosyltransferase substrate specificity has been successfully modified by site-directed mutagenesis assisted by computational modelling or directed evolution for the synthesis of biologically relevant carbohydrate structure (ref. 27). Promiscuous β-glycosidases showing altered and new specificities towards the donor or the acceptor sugar have been generated (ref. 28, 29, 31). Engineering of new glycosynthases (*i*.*e*., enzymes catalyzing the condensation of sugar residues for synthesizing a glycoside) from β-glycosidases (classified into EC 3.2.1) also emerged as a powerful way to generate modified transferases, even if they use only fluoride donors (ref. 32). In addition, this methodology has never been shown to be successful for α-retaining enzyme. However, one case of active glycosynthase derived from invertase enzymes has been described up to now (ref. 33).

Interestingly, the use of intermediates issued from enzymatic glycosylation in the subsequent generation of glycoconjugates of higher complexity has also been reported (ref. 36-38). In all cases, the building blocks, conceived by action of native glycosyltransferases, were converted to donors and used as such, following peracylation.

The term "building block" refers to a suitably protected carbohydrate intermediate occurring in the chemical pathway of synthesis of complex oligosaccharides, e.g., said carbohydrate can be a disaccharide.

The term "intermediate" refers to a compound, protected or not, issued from an enzymatic and/or synthetic step, and involved in the multi-step synthesis of a specific target, e.g., said compound can be a disaccharide.

The design of an appropriate enzymatic glycosylation tool that would allow an optimal combination of the chemical and enzymatic steps involved in the synthesis of complex oligosaccharides has never been attempted although it would be of major interest to develop new synthetic pathways.

The Inventors have thus investigated the applicability of enzymatic glycosylation for the synthesis of building blocks compatible with chemical chain extension both at the reducing and non-reducing ends, which is compatible with subsequent conversion into donors as well as into acceptors. In the course of their investigation, the Inventors have surprisingly demonstrated the applicability of engineered amylosucrases (AS) - which are glycoside hydrolases - to the synthesis of disaccharide intermediates to synthetic oligosaccharide fragments of *Shigella flexneri* 1a and/or 1b lipopolysaccharide by *in vitro* chemo-enzymatic synthetic methodologies, implicating an enzymatic step at an early stage in the synthesis.

Amylosucrases (EC 2.4.1.4) as well as sucrose hydrolases (EC 3.2.1.-) belong to the family 13 of the glycoside hydrolases (GH13), and more particularly the subfamily 4 (GH13.4) as defined per the CAZY nomenclature (ref. 62-65). Amylosucrases and sucrose hydrolases operate on the same substrate (sucrose) with the same molecular mechanism (ref. 67). The difference between the amylosucrases and the sucrose hydrolases is only different transglycosylation abilities (ref. 65).

The structure of amylosucrase from *Neisseria polysaccharea* is the only known structure of enzymes from family GH13.4 (ref. 68). The single polypeptide chain (628 amino acid residues) of amylosucrase from *Neisseria polysaccharea* is folded into a tertiary structure consisting of five domains named N (residues 1-90), A (residues 98-184; 261-395; 461-550), B (residues 185-260), B' (residues395-460) and C (residues 555-628). Domains A, B and C are common domains found in family GH13. Domains N and B' are specific to family GH13.4. Domain N is the N-terminal domain composed of 6 α-helices. Domain A is made up of eight alternating β-sheets (β1- β8) and α-helices (α1-α8) building up the catalytic core: the (β/α)₈ barrel common to family GH13. It contains also eight loops connecting helices to strands (labeled loop1 to loop8). Domain B, or loop 3, is an extension of domain A, containing two short antiparallel β-sheets flanked by two α-helices. Domain B', or loop 7, is another extension of domain A, composed of two α -helices followed by a β-sheet and another short α -helice. Domain C is an eight-stranded β-sandwich found C-terminal to the (β/α)₈ barrel.

Unexpectedly, the Inventors have now found eleven consensus amino acid sequences to characterize glycoside hydrolases: eight consensus motifs defined hereafter (SEQ ID NO: 1; SEQ ID NO: 2; SEQ ID NO: 3; SEQ ID NO: 6; SEQ ID NO: 7; SEQ ID NO: 8; SEQ ID NO: 10; SEQ ID NO: 11) are localised in said β-sheets (6) or said loops (2) constituting domain A, two consensus motifs (SEQ ID NO: 4; SEQ ID NO: 5) are found in said domain B and one consensus motifs (SEQ ID NO: 9) is found is said domain B'.

*Shigella* is the causal agent of shigellosis, or bacillary dysentery. In developing countries, it induces about 1 million deaths per year, most of which involve children under five years of age (ref. 39). In countries where disease is endemic, a number of *S*. *flexneri* serotypes and to a lesser extent *S*. *sonnei* are isolated, emphasizing the need for a multivalent vaccine. Noteworthy, despite numerous clinical trials (ref. 40), no vaccine is available so far. Epidemiological as well as experimental data point to the polysaccharide part, or O-antigen (O-Ag), of the bacterial lipopolysaccharide as an important virulence factor (ref. 41) and the major target of protective humoral response against reinfection (ref. 42). *S*. *flexneri* is divided into at least 14 serotypes based on known O-Ag structures. Interestingly, protein-conjugates of short synthetic oligosaccharides mimicking *S*. *flexneri* 2a O-Ag induced in mice a potent anti-O-Ag humoral immune response, which was shown to be protective against homologous challenge (ref. 43). The diversity, associated to a close resemblance in composition, of the known *S. flexneri* O-Ag repeating units was found of utmost interest to challenge the investigation. Indeed, except for serotype 6, all *S. flexneri* O-Ag repeating units share a linear tetrasaccharide backbone (ref. 41). Diversity resides in the branching pattern, which involves *O*-acetyl and/or α-D-glucopyranosyl decorations (ref. 41, 44). Interestingly, at least 4 different patterns of α-D-glucosylation, have been characterized for this family of bacterial polysaccharides. *N*-acetyl-D-glucopyranosamine residue can be implicated as branching acceptor. By way of example, serotypes 1a and 1b of *S*. *flexneri* share the α-D-glucopyranosyl-(1→4)-*N*-acetyl-β-D-glucopyranosaminyl (ED) branching pattern.

Within the framework of research that has led to the present invention, the Inventors have demonstrated the chemo-enzymatic synthesis of disaccharide building blocks to *S. flexneri* 1a and 1b serotype-specific oligosaccharides by selecting a 2-acetamido-2-deoxy-D-glucopyranoside residue as substrate acceptor, and using as enzyme a recombinant amylosucrase (an α-retaining transglucosidase from family 13 of glycoside-hydrolases that uses sucrose as glucosyl donor, (ref. 45, 46)), at an earlier stage of a multi-step synthesis. New amylosucrase specificities were then surprisingly generated to glucosylate efficiently and regiospecifically allyl 2-acetamido-2-deoxy-α-D-glucopyranoside to provide building blocks compatible with chemical chain elongation as exemplified (Schemes 2A, 2C, and 2D, Examples 2, 3, and 4).

Here-under are examples of repeating units and/or cores of bacterial surface polysaccharides containing the disaccharide motives synthesized by glucansucrases (ref. 39, 41):

| Target disaccharide | Organism | Structure |
|---|---|---|
| α-D-Glc*p*-(1→4)-D- Glc*p*NAc | *E. coli* O18B1 | →6)-α-D-Glcp-(1→4)-[β-D-Glcp- (1→3)]-α-D-Galp(1→3)-[α-D-Glcp- (1→4)]-α-D-GlcpNAc-(1→2)-α-L-Rhap- (1→ →6)-α-D-Glcp-(1→4)-[?]-D-GlcpNAc- (1→ as core fragment |
| | *H pantelleriensis* | |

Accordingly, the present invention provides a method for preparing a building block corresponding to the disaccharide α-D-glucopyranosyl-(1→4)-2-*N-*acetyl-2-deoxy-α-D-glucopyranoside of formula (I): 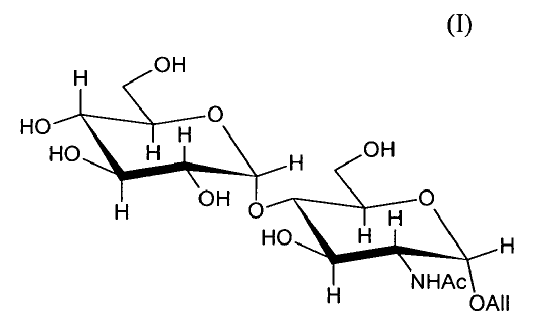 said method being **characterized in that** it comprises the step of using a mutant of a wild-type glycoside hydrolase, wherein said wild type glycoside hydrolase has 450 to 850 amino acids, preferably 580 to 735 amino acids, and comprises, preferably from the N- to C-terminus, eleven motifs defined by the following consensus motifs:
(1) the amino acid sequence LGVNYLHLMPL (SEQ ID NO: 1), which is located in the β-strand 2 of said wild type glycoside hydrolase;
(2) the amino acid sequence DGGYAV (SEQ ID NO: 2), which is located in the loop 2 of the (β/α)₈-barrel of said wild type glycoside hydrolase;
(3) the amino acid sequence DFVFNH (SEQ ID NO: 3) which is located in the β-strand 3 of said wild type glycoside hydrolase;
(4) the amino acid sequence LREIFPDTAPGNF (SEQ ID NO: 4), which is located in the domain B of said wild type glycoside hydrolase;
(5) the amino acid sequence FNSYQWDLN (SEQ ID NO: 5), which is located in the C-terminal part of the domain B of said wild type glycoside hydrolase;
(6) the amino acid sequence ILRLDAVAFLWK (SEQ ID NO: 6), which is located in the β-strand 4 of said wild type glycoside hydrolase;
(7) the amino acid sequence EAIV (SEQ ID NO: 7), which is located in the β-strand 5 of said wild type glycoside hydrolase;
(8) the amino acid sequence YVRCHDDI (SEQ ID NO: 8), which is located in the β-strand 7 of said wild type glycoside hydrolase;
(9) the amino acid sequence RISGTLASLAG (SEQ ID NO: 9), which is located in the domain B' of said wild type glycoside hydrolase;
(10) the amino acid sequence GIPLIYLGDE (SEQ ID NO: 10), which is located in the β-strand 8 of said wild type glycoside hydrolase;
(11) the amino acid sequence RWVHRP (SEQ ID NO: 11), which is located in the loop 8 of the (β/α)₈-barrel,
   and the sequence formed by said eleven motifs joined end-to-end from motif (1) to motif (11) of said wild type glycoside hydrolase has at least 65%, preferably at least 70%, and by order of increasing preference, at least 75%, 80%, 85%, 90%, 95%, 95%, 97%, 98%, and 99%, or 100% sequence identity or at least 80%, preferably at least 85%, and by order of increasing preference, at least 90%, 95%, 95%, 97%, 98%, and 99%, or 100% sequence similarity with the amino acid sequence SEQ ID NO: 12, which is formed by the concatenation of the eleven consensus motifs ordered from (1) to (11);
wherein said mutant has a mutation consisting of:
- the substitution of the amino acid residue at position 4 in said motif (4) with any amino acid selected from the group consisting of alanine (A), cysteine (C), glutamic acid (E), glycine (G), histidine (H), leucine (L), methionine (M), asparagine (N), proline (P), glutamine (Q), serine (S), threonine (T), valine (V) with the provisio that said wild type glycoside hydrolase does not contain a valine at this position, tryptophan (W) and tyrosine (Y), or
- the substitution of the amino acid residue at position 8 in said motif (6) with any amino acid selected from the group consisting of glutamic acid (E), phenylalanine (F), glycine (G), lysine (K), leucine (L), methionine (M), proline (P), glutamine (Q), arginine (R) and valine (V), or
- the substitution of the amino acid residue at position 9 in said motif (6) with any amino acid selected from the group consisting of alanine (A), cysteine (C), aspartic acid (D), glutamic acid (E), glycine (G), histidine (H), isoleucine (I), lysine (K), leucine (L), methionine (M), proline (P), glutamine (Q), arginine (R), serine (S), threonine (T), valine (V) and tryptophan (W), or
- the substitution of the amino acid residue at position 4 in said motif (7) with any amino acid selected from the group consisting of alanine (A), cysteine (C), aspartic acid (D), glycine (G), histidine (H), isoleucine (I), leucine (L), methionine (M), asparagine (N), serine (S), threonine (T) and tyrosine (Y), or
- the substitution of the amino acid residue at position 7 in said motif (8) with any amino acid selected from the group consisting of alanine (A) and valine (V), or
- the substitution of the amino acid residue at position 1 in said motif (9) with any amino acid selected from the group consisting of alanine (A), cysteine (C), phenylalanine (F), glycine (G) with the provisio that said wild type glycoside hydrolase does not contain a glycine at this position, lysine (K), asparagine (N), glutamine (Q), serine (S) with the provisio that said wild type glycoside hydrolase does not contain a serine at this position, threonine (T) and tryptophan (W).

According to a preferred embodiment of said mutant of a wild type glycoside hydrolase, the amino acid residue at position 9 in said motif (6) is substituted with any amino acid selected from the group consisting of cysteine (C), aspartic acid (D), isoleucine (I), lysine (K) and glutamine (Q), and more preferably with any amino acid selected from the group consisting of aspartic acid (D) and lysine (K).

A "wild type glycoside hydrolase" refers to an amylosucrase (EC 2.4.1.4) or a sucrose hydrolase (EC 3.2.1.-), preferably an amylosucrase. A wild type glycoside hydrolase belongs to the family 13, subfamily 4, of the glycoside hydrolases (GH 13.4) as defined per the CAZY nomenclature (ref. 66-69, http://www.cazy.org).

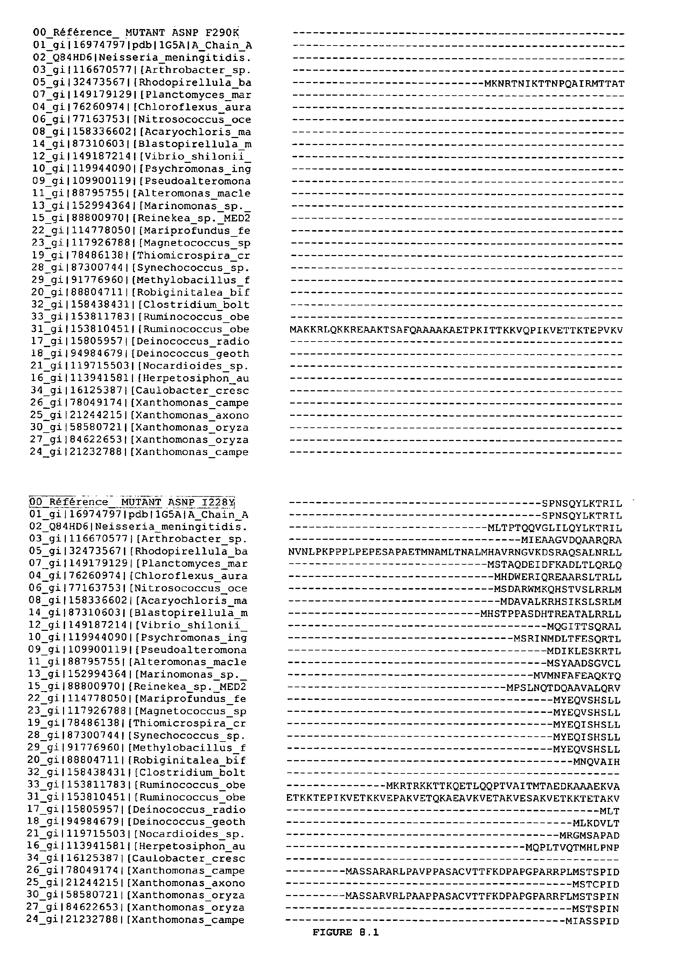

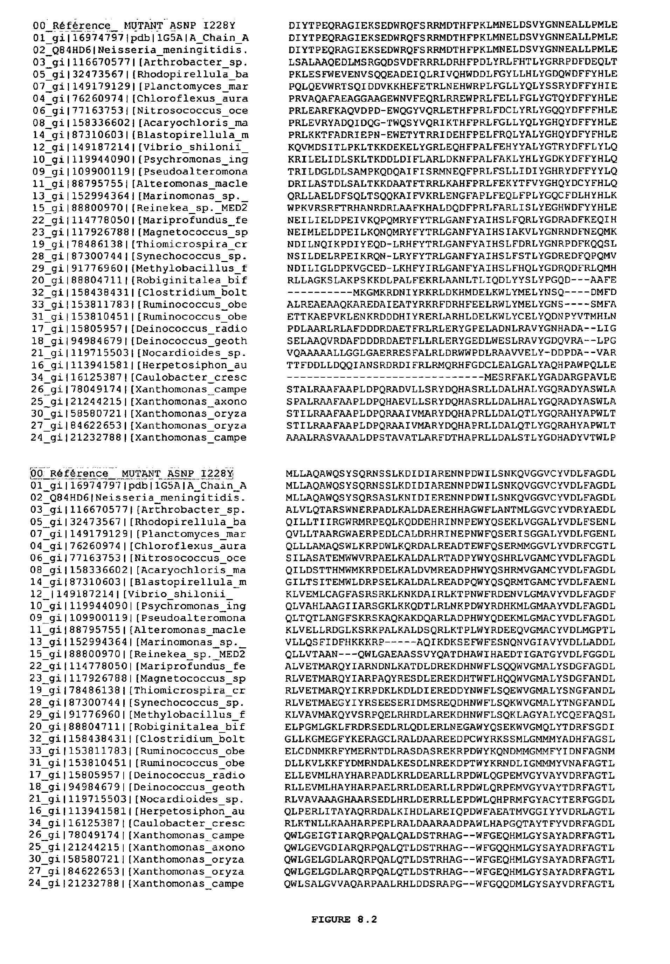

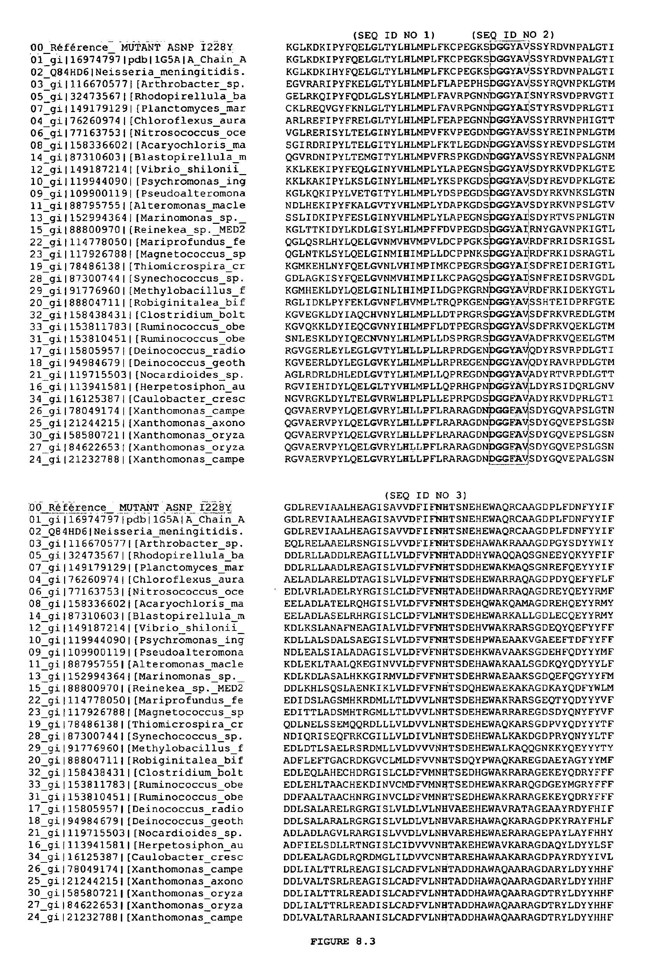

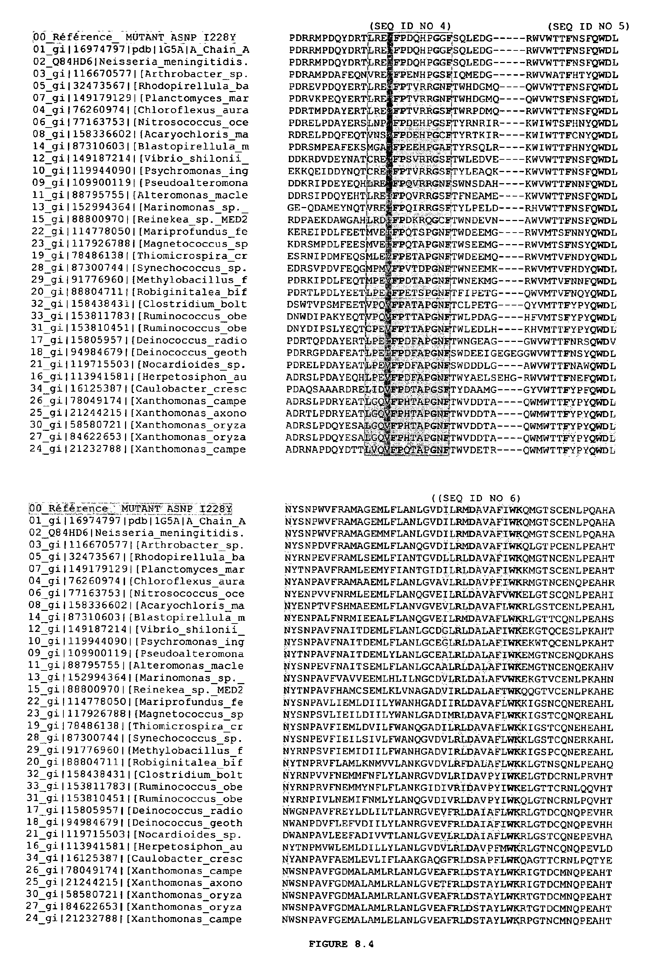

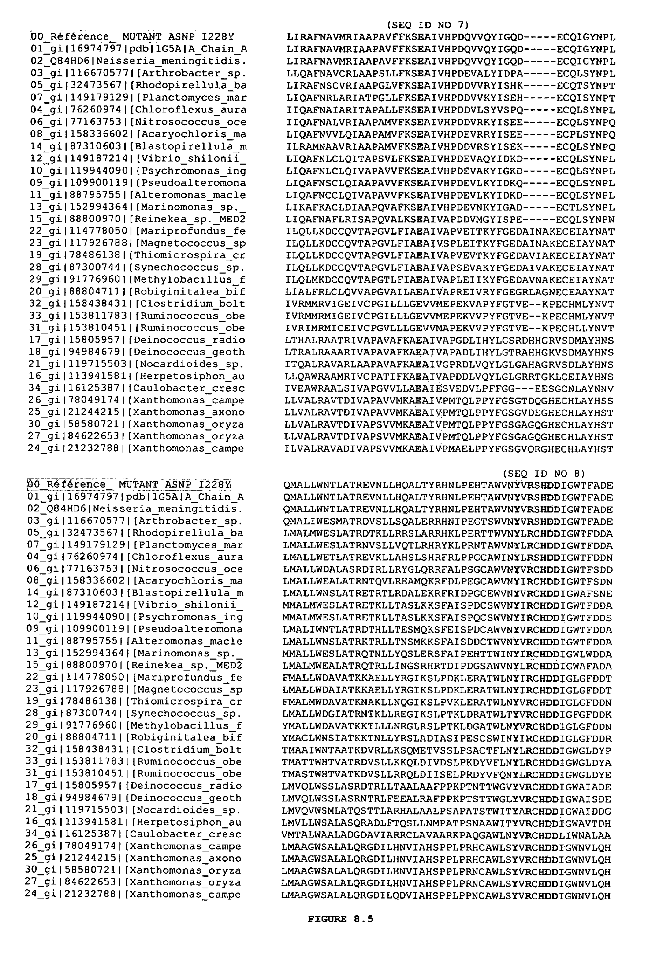

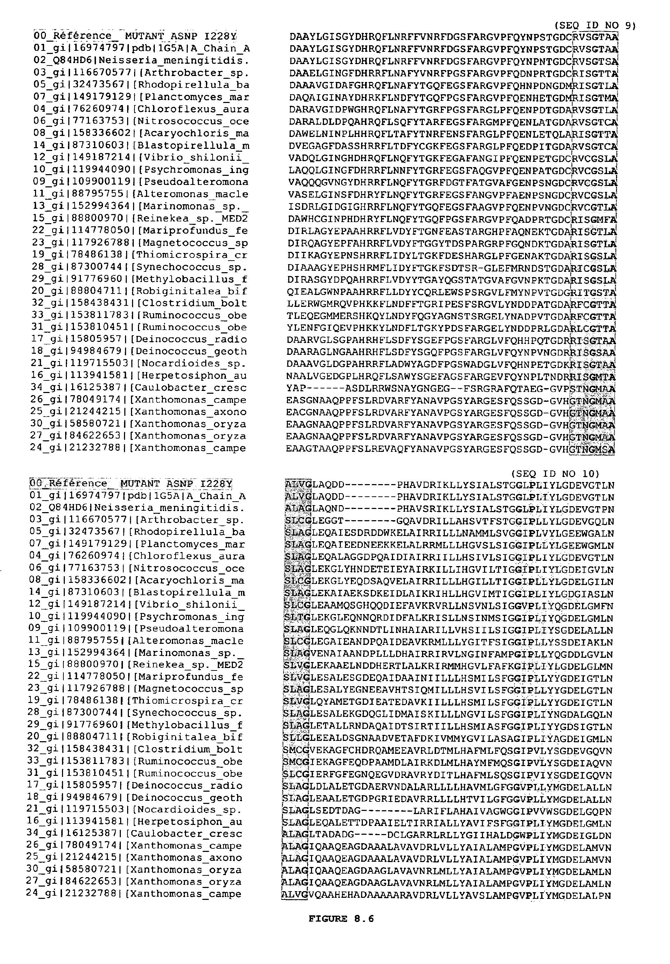

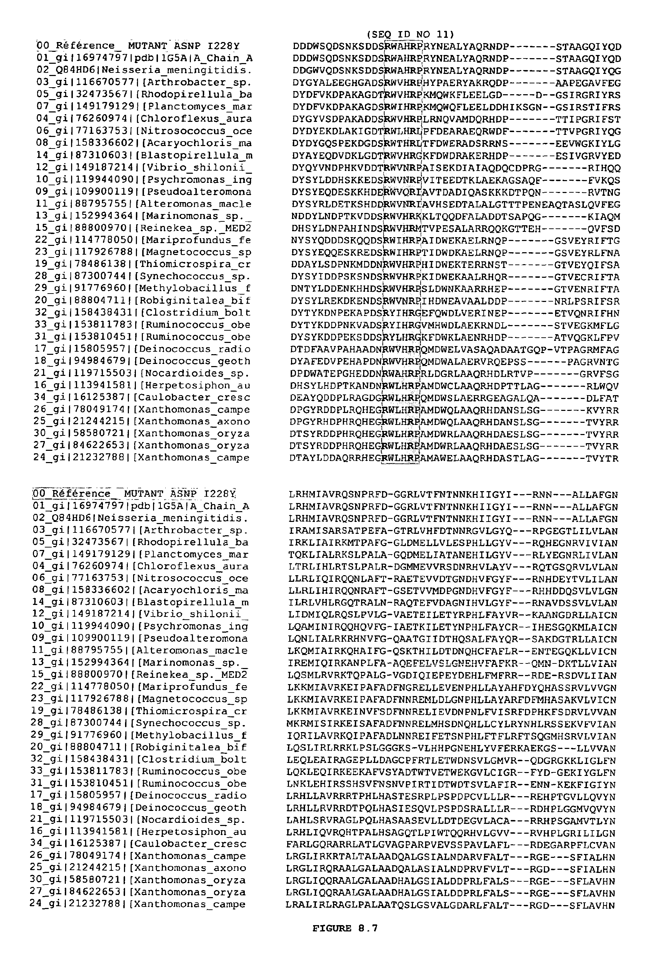

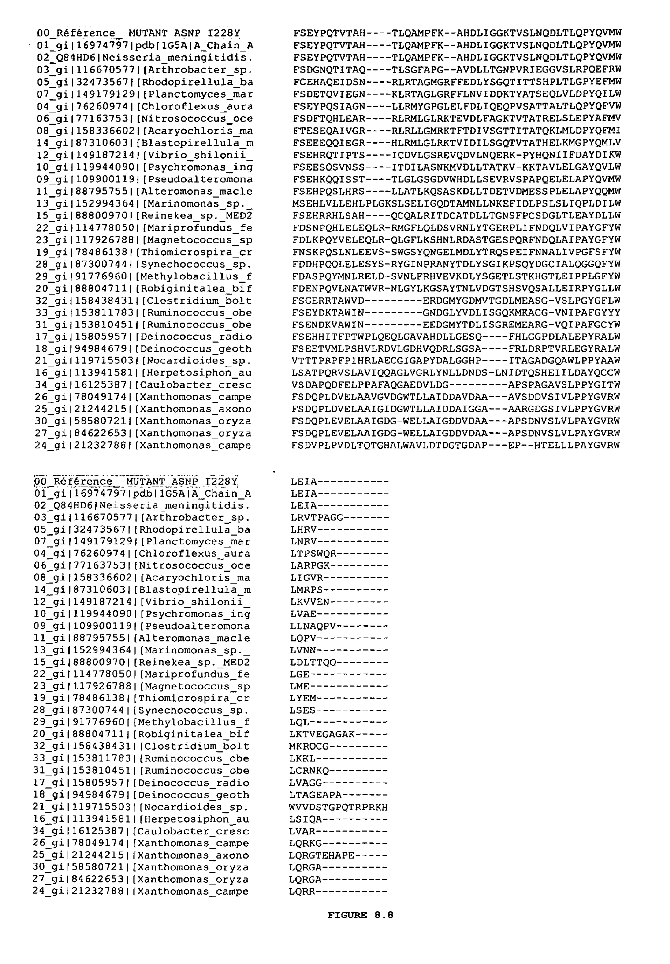

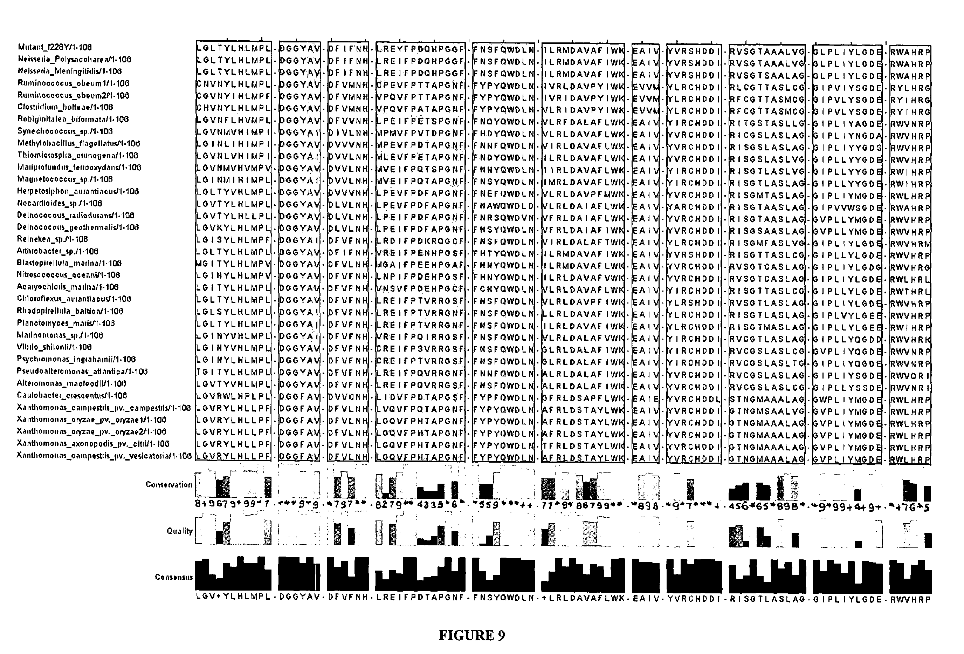

The eleven consensus motifs of the wild type glycoside hydrolases have been found by the Inventors by aligning 34 wild type glycoside hydrolases as shown in Figures 8 and 9.

By way of example, the glycoside hydrolase 1G5A (gi|16974797, SEQ ID NO: 13) comprises, from the N- to C-terminus, the eleven following motifs: (1) 125-134, (2) 144-149, (3) 182-187, (4) 225-237, (5) 250-258, (6) 282-293, (7) 328-331, (8) 388-395, (9) 446-456, (10) 480-489 and (11) 509-514 of SEQ ID NO: 13. These eleven motifs joined end-to-end form motif (1) to motif (11) form an amino acid sequence which has 83% sequence identity and 92% sequence similarity with the sequence SEQ ID NO: 12.

In order to identify the eleven motifs from a wild type glycoside hydrolase, one of skilled in the art can align the amino acid sequence of this wild type glycoside hydrolase against the amino acid sequence of the glycoside hydrolase 1G5A (SEQ ID NO: 13) for example, and therefore identify the eleven motifs thereof matching the eleven consensus motifs as described above.

Unless otherwise specified, sequence alignments are performed using the well-known MUSCLE program under default parameters (http://phylogenomics.berkeley.edu/cgi-bin/muscle/input_muscle.py). Jalview software can be used for visualizing the alignment and generating the eleven motifs joined end-to-end. The sequence identity and similarity values provided herein are calculated using the Vector NTI AlignX program (V9.1.0, Invitrogen, USA) on a comparison window including the whole set of eleven consensus motifs ordered from 1 to 10 as defined above.

In a particular embodiment of said wild type glycoside hydrolase, it is an amylosucrase selected from the group consisting of the proteins available in the GENBANK database under the following accession number: gi|16974797 (named 1G5A and also reproduced herein as SEQ ID NO: 13), gi|99031739 (named 1ZS2), gi|27574003 (1MVY), gi|27574004 (named 1MW0), gi|47169012 (named 1S46), gi|16974938 (named 1JGI), gi|27574006 (named 1MW2), gi|27574007 (named 1MW3), gi|27574005 (named 1MW1), gi|16974937 (named 1JG9), gi|27728142 (named Q84HD6), gi|116670577, gi|32473567, gi|149179129, gi|76260974, gi|7163753, gi|158336602, gi|87310603, gi|149187214, gi|119944090, gi|109900119, gi|88795755, gi|152994364, gi|88800970, gi|114778050, gi|117926788, gi|78486138, gi|87300744, gi|91776960, gi|88804711, gi|158438431, gi|153811783, gi|153810451, gi|15805957, gi|94984679, gi|119715503, gi|113941581, gi|16125387, gi|119477809 and gi|89092061.

In a more preferred embodiment of said wild type glycoside hydrolase, it is an amylosucrase from *Neisseria polysaccharea,* and is preferably selected from the group consisting of 1G5A, 1ZS2, 1MVY, 1MW0, 1S46, 1JGI, 1MW2, 1MW3, 1MW1 and 1JG9 proteins.

In another preferred embodiment of said wild type glycoside hydrolase, it is a sucrose hydrolase from *Xanthomonas*, and is preferably selected from the group consisting of the proteins available in the GENBANK database under the following accession number: gi|78049174, gi|21244215, gi|58580721, gi|84622653, gi|21232788.

The Table I below shows the sequence identity and similarity percent of the eleven motifs joined end-to-end for each of 34 glycoside hydrolases as described above with the sequence SEQ ID NO: 12.

**Table I:**

| GI number in the GenBank Database | Organisms | % identity | % similarity |
|---|---|---|---|
| gi\|16974797 | *Neisseria polysaccharea* | 83 | 92 |
| gi\|27728142 | *Neisseria meningitidis* | 84 | 92 |
| gi\|116670577 | *Arthrobacter sp. FB24* | 83 | 90 |
| gi\|76260974 | *Chloroflexus aurantiacus J-10-fl* | 85 | 93 |
| gi\|32473567 | *Rhodopirellula baltica SH 1* | 86 | 91 |
| gi\|77163753 | *Nitrosococcus oceani ATCC 19707* | 85 | 92 |
| gi\|149179129 | *Planctomyces maris DSM8797* | 85 | 91 |
| gi\|158336602 | *Acaryochloris marina MBIC11017* | 81 | 93 |
| gi\|109900119 | *Pseudoalteromonas atlantica T6c* | 81 | 93 |
| gi\|119944090 | *Psychromonas ingrahamii 37* | 81 | 91 |
| gi\|88795755 | *Alteromonas macleodii 'Deep ecotype'* | 79 | 90 |
| gi\|149187214 | *Vibrio shilonii AK1* | 79 | 88 |
| gi\|152994364 | *Marinomonas sp. MWYL1* | 79 | 86 |
| gi\|87310603 | *Blastopirellula marina DSM3645* | 80 | 91 |
| gi\|88800970 | *Reinekea sp. MED297* | 80 | 91 |
| gi\|113941581 | *Herpetosiphon aurantiacus ATCC 23779* | 82 | 92 |
| gi\|15805957 | *Deinococcus radiodurans R1* | 83 | 93 |
| gi\|94984679 | *Deinococcus geothermalis DSM 11300* | 85 | 93 |
| gi\|78486138 | *Thiomicrospira crunogena XCL-2* | 83 | 91 |
| gi\|88804711 | *Robiginitalea biformata HTCC2501* | 82 | 90 |
| gi\|119715503 | *Nocardioides sp. JS614* | 81 | 91 |
| gi\|114778050 | *Mariprofundus ferrooxydans PV-1* | 82 | 91 |
| gi\|117926788 | *Magnetococcus sp. MC-1* | 84 | 91 |
| gi\|21232788 | *Xanthomonas campestris pv. campestris str.* | 73 | 88 |
| gi\|21244215 | *Xanthomonas axonopodis pv. citri str. 306* | 74 | 88 |
| gi\|78049174 | *Xanthomonas campestris pv. vesicatoria str. 85-10* | 74 | 88 |
| gi\|84622653 | *Xanthomonas oryzae pv. oryzae MAFF 311018* | 74 | 88 |
| gi\|87300744 | *Synechococcus sp. WH 5701* | 79 | 91 |
| gi\|91776960 | *Methylobacillus flagellatus KT* | 82 | 88 |
| gi\|58580721 | *Xanthomonas oryzae pv. oryzae KACC10331* | 74 | 88 |
| gi\|153810451 | *Ruminococcus obeum ATCC 29174* | 73 | 88 |
| gi\|158438431 | *Clostridium bolteae ATCC BAA-613* | 68 | 84 |
| gi\|153811783 | *Ruminococcus obeum ATCC 29174* | 67 | 83 |
| gi\|16125387 | *Caulobacter crescentus CB15* | 68 | 86 |

According to a preferred embodiment of said wild type glycoside hydrolase, it contains an isoleucine (I) or valine (V) residue at position 4 in said motif (4), preferably an isoleucine.

According to another preferred embodiment of said wild type glycoside hydrolase, it contains an alanine (A) or proline (P) residue at position 8 in said motif (6), preferably an alanine.

According to another preferred embodiment of said wild type glycoside hydrolase, it contains a phenylalanine (F) or tyrosine (Y) residue at position 9 in said motif (6), preferably a phenylalanine.

According to another preferred embodiment of the said wild type glycoside hydrolase, it contains a valine (V), methionine (M) or glutamic acid (E) residue at position 4 in said motif (7), preferably a valine.

According to another preferred embodiment of said wild-type glycoside hydrolase, it contains an aspartic acid (D) residue at position 7 in said motif (8).

According to another preferred embodiment of said wild-type glycoside hydrolase, it contains a glycine (G), arginine (R) or serine (S) residue at position 1 in said motif (9), preferably a glycine.

In a preferred embodiment of said mutant of a glycoside hydrolase, it is a mutant of the 1G5A amylosucrase from *Neisseria polysaccharea* having the amino acid sequence SEQ ID NO: 13, wherein said mutant, has in reference to SEQ ID NO: 13, a mutation consisting of:
- the substitution of the isoleucine (I) residue at position 228 (I228), corresponding to position 4 in said motif (4), with any amino acid selected from the group consisting of alanine (A), cysteine (C), glutamic acid (E), glycine (G), histidine (H), leucine (L), methionine (M), asparagine (N), proline (P), glutamine (Q), serine (S), threonine (T), valine (V), tryptophan (W) and tyrosine (Y), or
- the substitution of the alanine (A) residue at position 289 (A289), corresponding to position 8 in said motif (6), with any amino acid selected from the group consisting of glutamic acid (E), phenylalanine (F), glycine (G), lysine (K), leucine (L), methionine (M), proline (P), glutamine (Q), arginine (R) and valine (V), or
- the substitution of the phenylalanine (F) residue at position 290 (F290), corresponding to position 9 in said motif (6), with any amino acid selected from the group consisting of alanine (A), cysteine (C), aspartic acid (D), glutamic acid (E), glycine (G), histidine (H), isoleucine (I), lysine (K), leucine (L), methionine (M), proline (P), glutamine (Q), arginine (R), serine (S), threonine (T), valine (V) and tryptophan (W), preferably with any amino acid selected from the group consisting of cysteine (C), aspartic acid (D), isoleucine (I), lysine (K) and glutamine (Q), and more preferably with any amino acid selected from the group consisting of aspartic acid (D) and lysine (K), or
- the substitution of the valine (V) residue at position 331 (V331), corresponding to position 4 in said motif (7), with any amino acid selected from the group consisting of alanine (A), cysteine (C), aspartic acid (D), glycine (G), histidine (H), isoleucine (I), leucine (L), methionine (M), asparagine (N), serine (S), threonine (T) and tyrosine (Y), or
- the substitution of the aspartic acid (D) residue at position 394 (D394), corresponding to position 7 in said motif (8), with any amino acid selected from the group consisting of alanine (A) and valine (V), or
- the substitution of the arginine (R) residue at position 446 (R446), corresponding to position 1 in said motif (9), with any amino acid selected from the group consisting of cysteine (C), phenylalanine (F), glycine (G), lysine (K), asparagine (N), glutamine (Q), serine (S), threonine (T) and tryptophan (W).

Unexpectedly, the mutants of a glycoside hydrolase according to the present invention present a specific activity toward D-Glc*p*NHTCA (NHTCA is N-trichloroacetyl) or/and substantially improve the glucosylation rate of D-Glc*p*NAc and α-D-Glc*p*NAc-OAll. The use of an appropriate combination of a mutant of a glycoside hydrolaseof the present invention with a donor and an acceptor as defined in the present invention at an earlier stage of a multi-step synthesis leads to the synthesis of complex oligosaccharides, such as *S. flexneri* 1a and 1b O-antigens.

Especially, the invention is directed to a method for preparing the building block corresponding to a disaccharide α-D-glucopyranosyl-(1→4)-2-amino-2-deoxy-α-D-glucopyranoside of formula and/or α-D-glucopyranosyl-(1→4)-2-*N*-acyl-2-deoxy-α-D-glucopyranoside of formula (Ia): 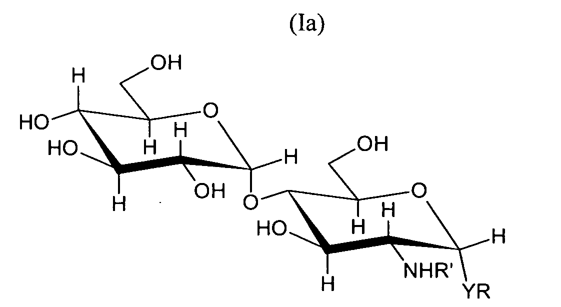 advantageously the disaccharide allyl α-D-glucopyranosyl-(1→4)-2-*N-*acetyl-2-deoxy-α-D-glucopyranoside of formula (I): 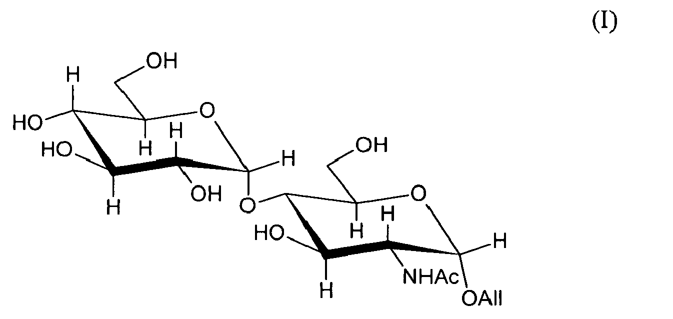 said method being **characterized in that** it comprises the step of reacting a mutant of a glycoside hydrolase as above disclosed, with the acceptor of formula (II), preferably of formula (IIa): 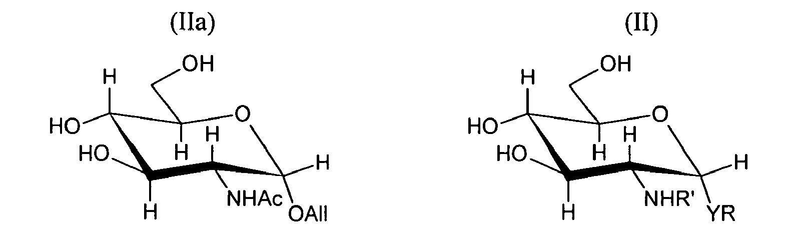 wherein Y is selected from -O- and -S- and R is selected from the group consisting of: C₁-C₆ alkyl, C₁-C₆ alkenyl, aryl, allyl, -CO-alkyl (C₁-C₆), -CO-alkenyl (C₁-C₆), -CO-aryl,
R' designates a group selected from: acetyl, trichloroacetyl (TCA), trifluoroacetyl (TFA),
wherein aryl designates an aromatic group like phenyl, benzyl, possibly substituted by one or several of the following groups: C₁-C₄ alkyl, -NO₂, a halogen atom, -O-alkyl (C₁-C₆),
with a donor of formula (IIIa) : 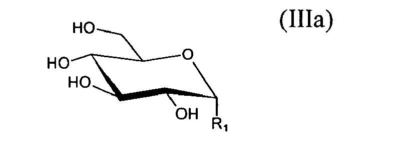 wherein R₁ represents a group selected from: 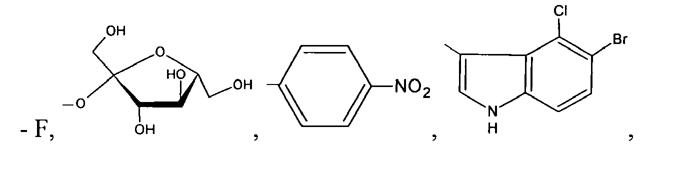 and preferably with the donor of formula (III), sucrose:

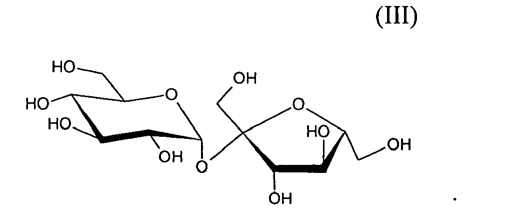

Another object of the invention is a method for the preparation of the building block corresponding to the disaccharide of formula (XX_{3B}) in which R₂ represents a group selected from AcBn and Ac. 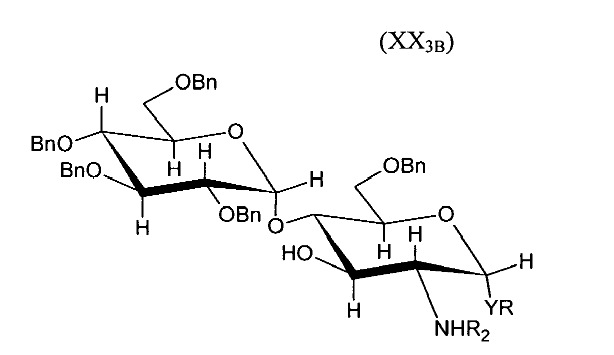 and preferably corresponding to the disaccharide of formula (XX_{3A}) 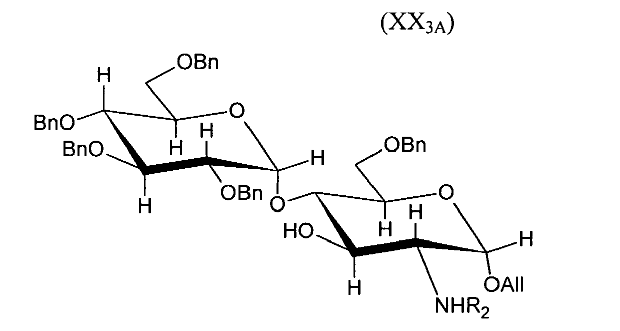 comprising at least one step wherein the acceptor of formula (IIa), advantageously (II), is reacted with a mutant of a glycoside hydrolase as above disclosed to give the disaccharide of formula (Ia) 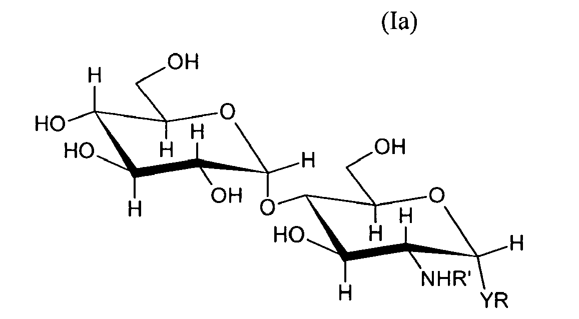 advantageously of formula (I)

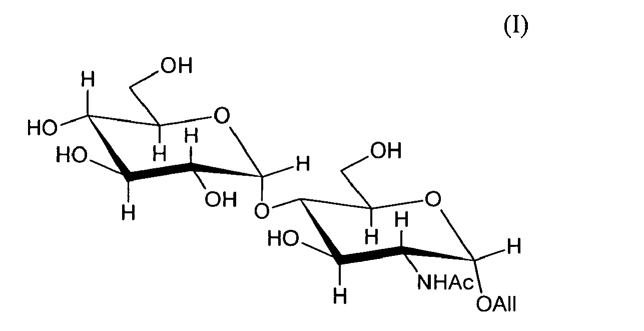

Some of the molecules obtained by the method of the invention are new and as such are another object of the invention. Their list is given here-under:
- Allyl α-D-glucopyranosyl-(1→4)-α-D-glucopyranosyl-(1→4)-2-acetamido-2-deoxy-α-D-glucopyranoside (XX₂),
- Allyl α-D-glucopyranosyl-(1→4)-2-acetamido-2-deoxy-α-D-glucopyranoside (XX₃),
- Allyl 2,3,4,6-tetra-O-acetyl-α-D-glucopyranosyl-(1→4)-2-acetamido-3,6-di-*O*-acetyl-2-deoxy-α-D-glucopyranoside (XX₄),
- Allyl α-D-glucopyranosyl-(1→4)-2-amino-2-deoxy-α-D-glucopyranoside (XX₅),
- Allyl α-D-glucopyranosyl-(1→4)-2-amino-2-*N*,3-O-carbonyl-2-deoxy-α-D-glucopyranoside (XX₆),
- Allyl 2,4,6-tri-*O*-benzyl-α-D-glucopyranosyl-(1→4)-6-*O*-benzyl-2-benzylamino-2-*N*,3-*O*-carbonyl-2-deoxy-α-D-glucopyranoside (XX₇),
- Allyl 2,3,4,6-tetra-*O*-benzyl-α-D-glucopyranosyl-(1→4)-6-*O*-benzyl-2-benzylamino-2-*N*,3-*O*-carbonyl-2-deoxy-α-D-glucopyranoside (XX₈),
- Allyl 2,3,4,6-tetra-*O*-benzyl-α-D-glucopyranosyl-(1→4)-6-*O*-benzyl-2-amino-2-*N*,3-*O*-carbonyl-2-deoxy-α-D-glucopyranoside (XX_{8A}),
- Allyl 2,3,4,6-tetra-*O*-benzyl-α-D-glucopyranosyl-(1→4)-6-*O*-benzyl-2-benzylamino-2-deoxy-α-D-glucopyranoside (XX₉),
- Allyl 2,3,4,6-tetra-*O*-benzyl-α-D-glucopyranosyl-(1→4)-6-O-benzyl-2-benzylacetamido-2-deoxy-α-D-glucopyranoside (XX₁₀),
- Allyl 2-*O*-benzoyl-4-*O*-benzyl-3-*O*-chloroacetyl-α-L-rhamnopyranosyl-(1→3)-[2,3,4,6-tetra-*O*-benzyl-α-D-glucopyranosyl-(1→4)]-6-*O-*benzyl-2-benzylacetamido-2-deoxy-α-D-glucopyranoside (XX₁₁),
- Allyl 2-*O*-acetyl-3,4-di-*O*-benzyl-α-L-rhamnopyranosyl-(1→3)-[2,3,4,6-tetra-*O*-benzyl-α-D-glucopyranosyl-(1→4)]-6-*O*-benzyl-2-benzylacetamido-2-deoxy-α-D-glucopyranoside (XX₁₂),
- Allyl α-D-glucopyranosyl-(1→4)-2-deoxy-2-trichloroacetamido-α-D-glucopyranoside (XX₁₃),
- Allyl 2,3,4,6-tetra-*O*-acetyl-α-D-glucopyranosyl-(1→4)-3,6-di-(*O-*acetyl-2-deoxy-2-trichloroacetamido-α-D-glucopyranoside (XX₁₄),
- 2,3,4,6-tetra-O-acetyl-α-D-glucopyranosyl-(1→4)-3,6-di-*O*-acetyl-2-deoxy-2-trichloroacetamido-α-D-glucopyranose (XX₁₅),
- 2,3,4,6-Tetra-*O*-acetyl-α-D-glucopyranosyl-(1→4)-3,6-di-O-acetyl-2-deoxy-2-trichloroacetamido-α-D-glucopyranosyl trichloroacetimidate (XX₁₆),
- Allyl 2,3,4,6-tetra-*O*-acetyl-α-D-glucopyranosyl-(1→4)-3,6-di-*O-*acetyl-2-deoxy-2-trichloroacetamido-α-D-glucopyranosyl-(1→2)-3,4-di-O-benzyl-α-L-rhamnopyranoside (XX₁₇),
- Allyl 2,3,4,6-tetra-*O*-benzyl-α-D-glucopyranosyl-(1→4)-2-acetamido-3-*O*-acetyl-6-*O*-benzyl-2-deoxy-α-D-glucopyranoside (XX₂₄),
- Allyl 2,3,4,6-tetra-*O*-benzyl-α-D-glucopyranosyl-(1→4)-2-acetamido-6-*O*-benzyl-2-deoxy-α-D-glucopyranoside (XX₂₅).

Another object of the invention is a mutant of a wild-type glycoside hydrolase, said wild-type glycoside hydrolase being defined as above, and said mutant having a mutation consisting of:
- the substitution of the amino acid residue at position 4 in said motif (4) with any amino acid selected from the group consisting of alanine (A), cysteine (C), glutamic acid (E), glycine (G), histidine (H), leucine (L), methionine (M), asparagine (N), proline (P), glutamine (Q), serine (S), threonine (T), valine (V) with the provisio that said wild type glycoside hydrolase does not contain a valine at this position, tryptophan (W) and tyrosine (Y), or
- the substitution of the amino acid residue at position 8 in said motif (6) with any amino acid selected from the group consisting of glutamic acid (E), phenylalanine (F), glycine (G), lysine (K), leucine (L), methionine (M), proline (P), glutamine (Q), arginine (R) and valine (V), or
- the substitution of the amino acid residue at position 9 in said motif (6) with any amino acid selected from the group consisting of alanine (A), cysteine (C), aspartic acid (D), glutamic acid (E), glycine (G), histidine (H), isoleucine (I), lysine (K), leucine (L), methionine (M), proline (P), glutamine (Q), arginine (R), serine (S), threonine (T), valine (V) and tryptophan (W), preferably selected from the group consisting of cysteine (C), aspartic acid (D), isoleucine (I), lysine (K) and glutamine (Q), and more preferably with any amino acid selected from the group consisting of aspartic acid (D) and lysine (K), or
- the substitution of the amino acid residue at position 4 in said motif (7) with any amino acid selected from the group consisting of alanine (A), cysteine (C), aspartic acid (D), glycine (G), histidine (H), isoleucine (I), leucine (L), methionine (M), asparagine (N), serine (S), threonine (T) and tyrosine (Y), or
- the substitution of the amino acid residue at position 7 in said motif (8) with any amino acid selected from the group consisting of alanine (A) and valine (V), or
- the substitution of the amino acid residue at position 1 in said motif (9) with any amino acid selected from the group consisting of alanine (A), cysteine (C), phenylalanine (F), glycine (G) with the provisio that said wild type glycoside hydrolase does not contain a glycine at this position, lysine (K), asparagine (N), glutamine (Q), serine (S) with the provisio that said wild type glycoside hydrolase does not contain a serine at this position, threonine (T) and tryptophan (W).

Advantageously, the mutants of a glycoside hydrolase according to the present invention having a mutation consisting of:
- the substitution of the amino acid residue at position 4 in said motif (4) with any amino acid selected from the group consisting of alanine (A), cysteine (C), glycine (G), histidine (H), asparagine (N), serine (S), threonine (T), tryptophan (W) and tyrosine (Y), or
- the substitution of the amino acid residue at position 9 in said motif (6) with any amino acid selected from the group consisting of cysteine (C), aspartic acid (D), glutamic acid (E), isoleucine (I) and valine (V),
   present also a specific activity toward L-Rha*p*, α-L-Rha*p*-OMe and α-L-Rha*p*-OAllyl. These mutants also catalyze the glucosylation of α-L-Rha*p*-OMe to give the disaccharide [α-D-Glcp(1→3)]-α-L-Rha*p*-OMe.

In a preferred embodiment of said mutant of a wild-type glycoside hydrolase, it is a mutant of the 1G5A amylosucrase from *Neisseria polysaccharea* having the amino acid sequence SEQ ID NO: 13, wherein said mutant, has in reference to SEQ ID NO: 13, a mutation consisting of:
- the substitution of the isoleucine (I) residue at position 228 (I228) with any amino acid selected from the group consisting of alanine (A), cysteine (C), glutamic acid (E), glycine (G), histidine (H), leucine (L), methionine (M), asparagine (N), proline (P), glutamine (Q), serine (S), threonine (T), valine (V), tryptophan (W) and tyrosine (Y), or
- the substitution of the alanine (A) residue at position 289 (A289) with any amino acid selected from the group consisting of glutamic acid (E), phenylalanine (F), glycine (G), lysine (K), leucine (L), methionine (M), proline (P), glutamine (Q), arginine (R) and valine (V), or
- the substitution of the phenylalanine (F) residue at position 290 (F290) with any amino acid selected from the group consisting of alanine (A), cysteine (C), aspartic acid (D), glutamic acid (E), glycine (G), histidine (H), isoleucine (I), lysine (K), leucine (L), methionine (M), proline (P), glutamine (Q), arginine (R), serine (S), threonine (T), valine (V) and tryptophan (W), or
- the substitution of the valine (V) residue at position 331 (V331) with any amino acid selected from the group consisting of alanine (A), cysteine (C), aspartic acid (D), glycine (G), histidine (H), isoleucine (I), leucine (L), methionine (M), asparagine (N), serine (S), threonine (T) and tyrosine (Y), or
- the substitution of the aspartic acid (D) residue at position 394 (D394) with any amino acid selected from the group consisting of alanine (A) and valine (V), or
- the substitution of the arginine (R) residue at position 446 (R446) with any amino acid selected from the group consisting of cysteine (C), phenylalanine (F), glycine (G), lysine (K), asparagine (N), glutamine (Q), serine (S), threonine (T) and tryptophan (W).

According to a more preferred embodiment of said mutant of a glycoside hydrolase, it is a mutant of the 1G5A amylosucrase from *Neisseria polysaccharea* has in reference to SEQ ID NO: 13, a mutation consisting of the substitution of the phenylalanine (F) residue at position 290 (F290) with any amino acid selected from the group consisting of cysteine (C), aspartic acid (D), isoleucine (I), lysine (K) and glutamine (Q), and more preferably with any amino acid selected from the group consisting of aspartic acid (D) and lysine (K). These mutants present a glucosylation rate of D-Glc*p*NAc of higher than 50%.

The present invention also provides polynucleotides encoding a mutant of a glycoside hydrolase according to the present invention.

Polynucleotides of the invention may be obtained by the well-known methods of recombinant DNA technology and/or of chemical DNA synthesis. These methods also allow introducing the desired mutations in a naturally occurring DNA sequence.

The invention also provides recombinant DNA constructs comprising a polynucleotide of the invention, such as expression cassettes wherein said polynucleotide is linked to appropriate control sequences allowing the regulation of its transcription and translation in a host cell and optionally to a sequence encoding a GST tag allowing a rapid purification of the mutant enzymes and recombinant vectors comprising a polynucleotide or an expression cassette of the invention.

Another object of the invention is a method for determining whether a wild type protein is a wild type glycoside hydrolase, said method comprising the steps of:
a) determining the amino acid sequence of said protein,
b) identifying in the amino acid sequence of said protein, preferably from the N- to C-terminus, eleven motifs defined by the following consensus motifs:
   (1) the amino acid sequence LGVNYLHLMPL (SEQ ID NO: 1);
   (2) the amino acid sequence DGGYAV (SEQ ID NO: 2);
   (3) the amino acid sequence DFVFNH (SEQ ID NO: 3);
   (4) the amino acid sequence LREIFPDTAPGNF (SEQ ID NO: 4);
   (5) the amino acid sequence FNSYQWDLN (SEQ ID NO: 5);
   (6) the amino acid sequence ILRLDAVAFLWK (SEQ ID NO: 6);
   (7) the amino acid sequence EAIV (SEQ ID NO: 7);
   (8) the amino acid sequence YVRCHDDI (SEQ ID NO: 8);
   (9) the amino acid sequence RISGTLASLAG (SEQ ID NO: 9);
   (10) the amino acid sequence GIPLIYLGDE (SEQ ID NO: 10);
   (11) the amino acid sequence RWVHRP (SEQ ID NO: 11);
c) determining the sequence identity percent or sequence similarity percent between the sequence formed by said eleven motifs joined end-to-end from motif (1) to motif (11) with the amino acid sequence SEQ ID NO: 12, and if the sequence identity percent is at least 65%, preferably at least 70%, and by order of increasing preference, at least 75%, 80%, 85%, 90%, 95%, 95%, 97%, 98%, and 99%, or 100% or if the sequence similarity is at least 80%, preferably at least 85%, and by order of increasing preference, at least 90%, 95%, 95%, 97%, 98%, and 99%, or 100%, then the wild type protein is a wild type glycoside hydrolase.

In addition to the preceding features, the invention further comprises other features which will emerge from the following description, which refers to examples illustrating the present invention, as well as to the appended figures.

Figure 1 shows the repeating unit of *S*. *flexneri* serotypes 1a and 1b O-Antigen. Rha*p* = rhamnopyranosyl - GlcNAc*p* = 2-*N*-acetyl-2-deoxy-glucopyranosyl - Glc*p* = glucopyranosyl - Ac = acetyl.

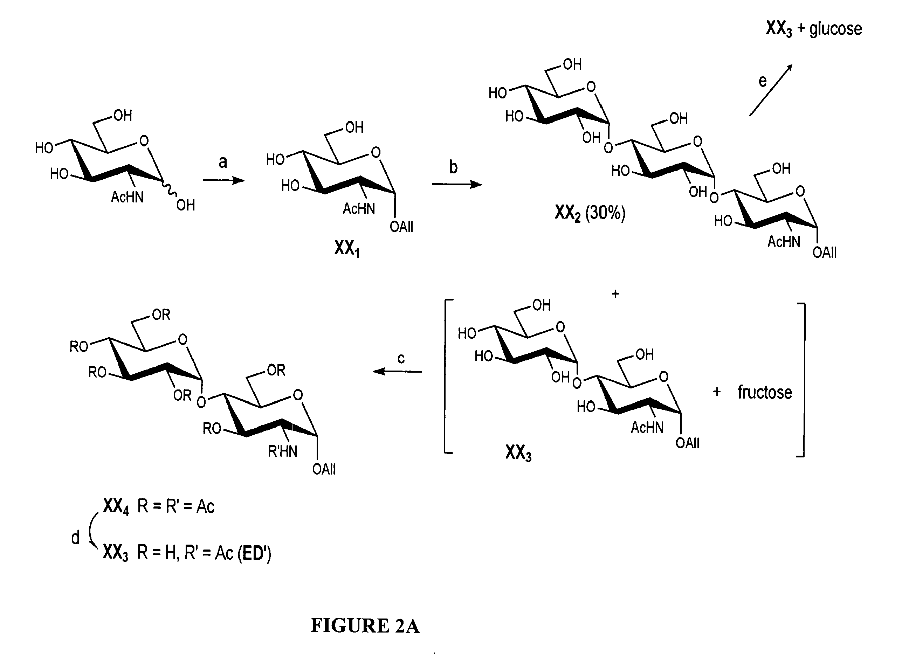

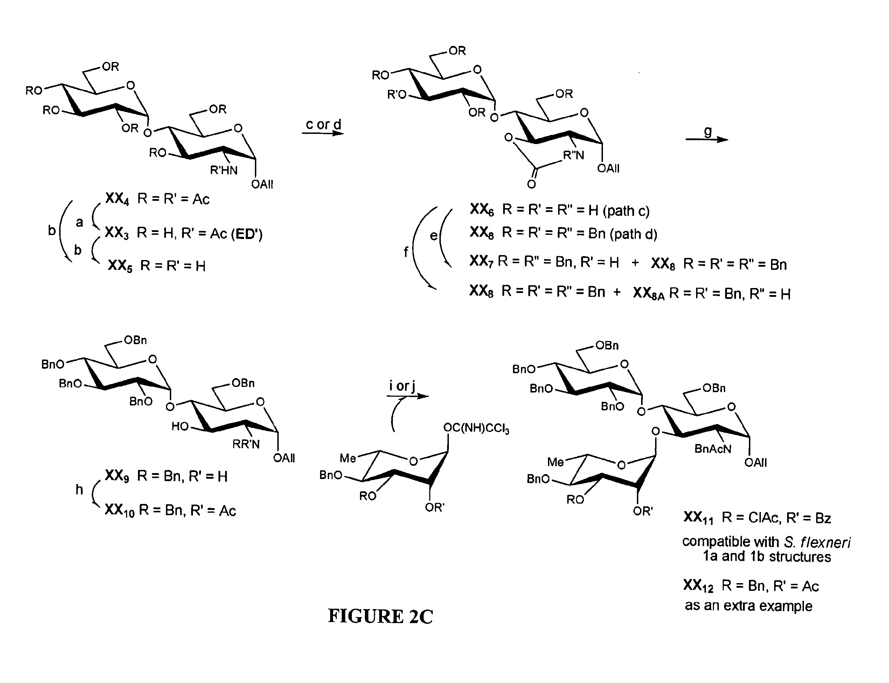

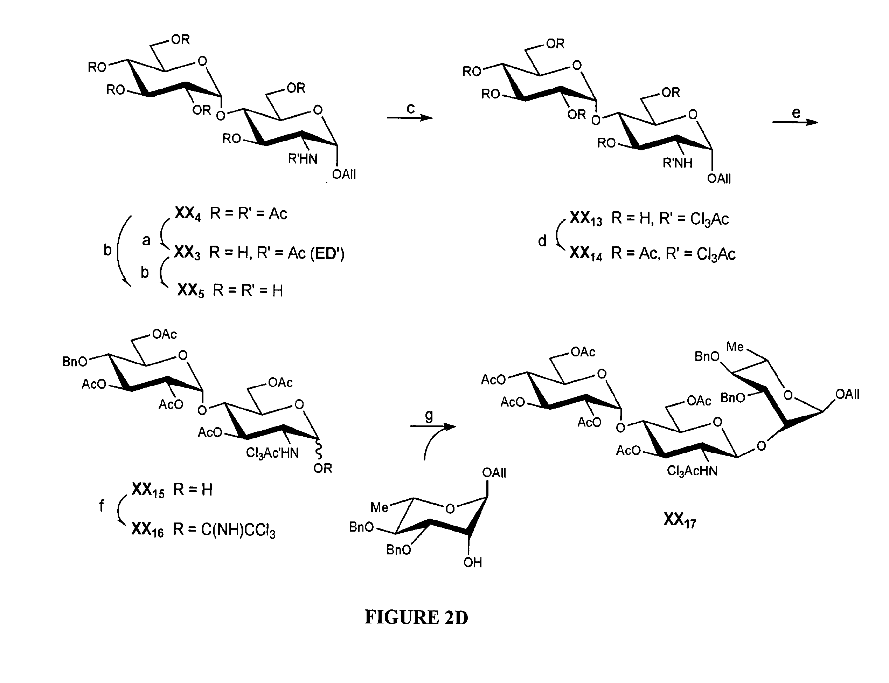

Figure 2 shows the first steps of chemo-enzymatic routes (A, C, D) to potential synthetic intermediates to oligosaccharide fragments of *S*. *flexneri* 1b and/or 1a O-antigens, a chemical synthetic route (B) to *S*. *flexneri* 1a pentasaccharides (ref. 66), and the chemical synthesis (E) of a model disaccharide intermediate to oligosaccharide fragments of *S. flexneri* 1b and/or 1a O-antigens. In Figure 2A: Chemo-enzymatic synthesis of disaccharide **XX₃**. a. AllOH, BF₃.OEt₂; b. non-purified F290K extract, sucrose; c. Ac₂O, Pyridine; d. MeONa, MeOH; e. Amyloglucosidase from *Aspergillus niger*, acetate buffer (pH 4.8). In Figure 2C: a. MeONa, MeOH; b. Ba(OH)₂.8H₂O, H₂O; c. *p*NO₂C₆H₄OCOCl, MeONa, MeOH; d. (i) Cl₃CH₂OCOCl , NaOMe, (ii) BnBr, NaH; e. (i) BnBr (6 eq), (ii) NaH; f. (i) NaH, (ii) BnBr (5.5 eq); g. 1M aq NaOH, 1,4-dioxane; h. Ac₂O, Pyridine; i. TMSOTf, MS 4Å, Toluene, 0°C; j. TMSOTf, MS 4Å, Toluene, -10°C. In Figure 2D: a. MeONa, MeOH; b. Ba(OH)₂.8H₂O, H₂O; c. (Cl₃Ac)₂O, NaOMe; b. Ac₂O, Pyridine; c. cat. [Ir(COD){PCH₃(C₆H₅)₂}₂]⁺PF₆⁻, THF; d. CCl₃CN, DBU; e. TMSOTf, MS 4Å, Toluene, -60°C.

Figure 3 shows the reaction catalyzed by glucansucrases. Glucansucrases follow a double displacement retaining mechanism, in which a β-glucosyl enzyme covalent intermediate is first formed from sucrose substrate. In a second step, the glucosyl moiety is transferred to an acceptor which depends on the conditions of reaction may be *(i)* water to give glucose *(ii)* fructose to form sucrose isomers *(iii)* glucose released from hydrolysis to form soluble oligosaccharides, or *(iv)* an exogeneous hydroxylated acceptor.

Figure 4 shows the architecture of the active site in complex with maltoheptaose (G7).

Figure 5 shows the comparison of docking modes: (A) Maltose moiety from the crystallographic maltoheptaose (PDB: 1MW0) occupying binding subsites (-1) and (+1) of amylosucrase from *Neisseria polysaccharea* and (B) α-D-Glc*p*-(1→4)-D-Glc*p*NAc in the active site of AS. The seven amino acid residues (I228, A289, F290, I330, V331, D394 and R446) selected for mutagenesis are shown on the figures. Hydrogen atoms have been omitted on the figures for clarity purpose.

Figure 6 shows the screening of the library for their ability to synthesize the desired disaccharide: α-D-Glc*p*-(1→4)-D-Gl*cp*NAc. Rows indicate the 7 mutated positions and columns represent the 20 possible amino acid mutations including the wild type amylosucrase.

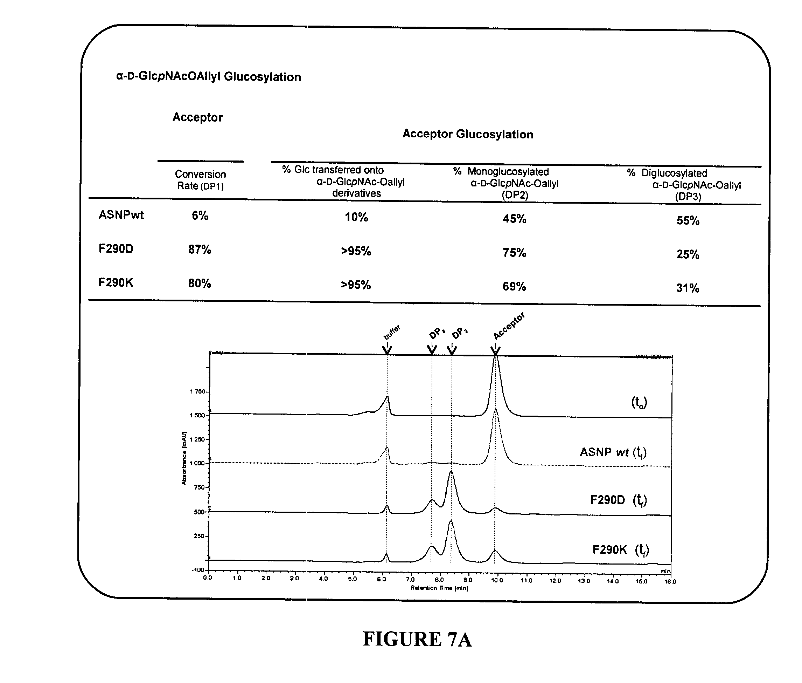

Figure 7A shows transglucosylation of α-D-Glc*p*NAc-OAll using the most improved 1G5A variant F290K and F290D and corresponding HPLC chromatogramm (with UV_{λ=220nm} detection) comparing F290K, F290D and ASNPwt. Initial reaction conditions: Sucrose=Acceptor=146mM. At final time, sucrose was fully consumed. Conversion Rate= (Q(Acceptor)ₜ₀-Q(Acceptor)_{tf})/Q(Acceptor)ₜ₀ where Q(X)= Quantity of X in moles. % Glc transferred onto acceptor derivatives = Q(Glucosyl units transferred onto acceptor derivatives)/Q(Glucosyl units transferrable from initial sucrose). % Monoglucosylated acceptor = Q(Monoglucosylated acceptor)/Q (acceptor derivatives). % Diglucosylated acceptor = Q(Diglucosylated acceptor)/ Q (acceptor derivatives). G=Glucose; F=Fructose; GF=Sucrose; DP₁= Acceptor; DP₂= monoglucosylated Acceptor; DP₃= diglucosylated Acceptor (to=initial time of the reaction - the medium contains only the donor and the acceptor, no products have been formed yet).

Figure 7B shows the comparison of Dionex HPAEC product profiles obtained at the end of the reaction (*t* = 24 h) with wild-type AS and the variant F290K using 146 mM sucrose.

Figure 7C shows the comparison of Dionex HPAEC product profiles obtained at the end of the reaction (*t* = 24 h) with wild-type AS and the variant F290K using 146 mM sucrose supplemented with 146 mM acceptor (α-D-Glc*p*NAc-OAll).

Figure 7D shows the comparison of kinetic parameters between ASNPwt and the variant F290K.

Figure 7E shows the determination of kinetic parameters for the variant F290K and ASNPwt catalyzed reactions: (a) varied acceptor (b) varied donor. Figure 7E(a), varied acceptor: α-allyl-N-Acetyl-glucopyranoside, constant donor: 250 mM. Figure 7E(b), varied donor, constant acceptor: α-allyl-N-Acetyl-glucopyranoside.

Figures 8.1 to 8.8 show the sequence alignment of 34 wild type glycoside hydrolases using the CLUSTALW program under default parameters.

Figure 9 shows the alignment of the eleven different motifs found in 34 wild type glycoside hydrolases.

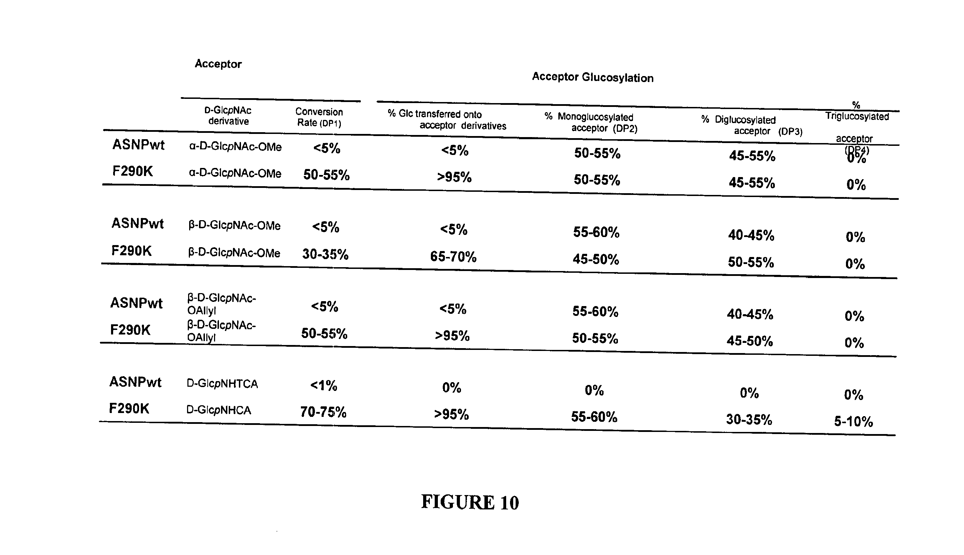

Figure 10 shows transglucosylation rates of D-Glc*p*NAc derivatives using the most improved variant F290K and ASNPwt. Initial reaction conditions: Sucrose=Acceptor=146mM. At final time, sucrose was fully consumed. Conversion Rate= (Q(Acceptor)ₜ₀-Q(Acceptor)_{tf})/Q(Acceptor)ₜ₀ where Q(X)= Quantity of X in moles. % Glc transferred onto acceptor derivatives = Q(Glucosyl units transferred onto acceptor derivatives)/Q(Glucosyl units transferrable from initial sucrose). % Monoglucosylated acceptor = Q(Monoglucosylated acceptor)/Q (acceptor derivatives). % Diglucosylated acceptor = Q(Diglucosylated acceptor)/ Q (acceptor derivatives). % Triglucosylated acceptor = Q(Triglucosylated acceptor)/ Q (acceptor derivatives).

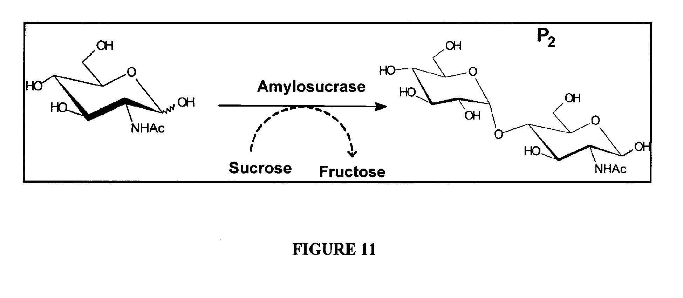

Figure 11 shows the structure of disaccharide α*-D-Glcp-(1*→*4)-D-GlcpNAc* (P2) obtained by AS-mediated glucosylation of D-GlcpNAc.

### EXAMPLE 1: Engineering transglucosidase for the synthesis the α-D-glucopyranosyl-(1→4)-N-acety-β-D-glucopyranosaminy disaccharide

### 1) Materials and Methods

### Bacterial strains, plasmids and chemicals

Plasmid pGST-AS, derived from the pGEX-6P-3 (GE Healthcare Biosciences) and containing the *N. polysaccharea* amylosucrase encoding gene (ref. 45) was used for the construction of the AS single mutant library.

*E. coli* JM109 was used as host for the plasmid library transformation, gene expression and large-scale production of the selected mutants.

Sucrose, *N*-acetyl-D-glucosamine and glycogen were purchased from Sigma-Aldrich (Saint-Louis, MO, USA). Known allyl 2-acetamido-2-deoxy-α-D-glucopyranoside (α-D-GlcNAc*p*-OAll) (ref. 69), allyl 2-acetamido-2-deoxy-β-D-glucopyranoside (ref. 70) (β-D-Glc*p*NAc-OAll), methyl 2-acetamido-2-deoxy-α-D-glucopyranoside (ref. 71) (α-D-Glc*p*NAc-OMe), methyl 2-acetamido-2-deoxy-β-D-glucopyranoside (ref. 70) (β-D-Glc*p*NAc-OMe), and 2-deoxy-2-trichloroacetamido -β-D-glucopyranose (ref. 72) (D-GlcNHTCA) were synthesized chemically.,

The reference disaccharides α-D-Glc*p*-(1→4)-D-Glc*p*NAc and Glc*p-*(1→6)-D-Glc*p*NAc were enzymatically synthesised and characterized (see Example 7).

Ampicillin, lysozyme and isopropyl-β-D-thiogalactopyranoside (IPTG) were purchased from Euromedex (Souffelweyersheim, France), and *Dpn*I restriction enzyme from New England Biolabs (Beverly, MA, USA).

Oligonucleotides were synthetised by Eurogenetec (Liege, Belgium). DNA extraction (QIASpin) and purification (QIAQuick) columns were purchased from Qiagen (Chatsworth, CA).

Wild type glycoside hydrolase: amylosucrase (ASNPwt) 1G5A of sequence SEQ ID NO: 13.

### Selection of mutation position by molecular modelling

*Starting models for the disaccharide and for AS:* The disaccharide α-D-Glc*p*-(1→4)-D-Glc*p*NAc was constructed with the monosaccharide obtained from a database of carbohydrate three-dimensional structures. All molecular modelling calculations were performed using the SYBYL 7.3 software. The coordinates of amylosucrase were taken from the 2.0 Å resolution crystal structures of amylosucrase from *N. polysaccharea* in complex with sucrose (PDB: 1JGI) and maltoheptaose, a reaction product (PDB: 1MW0). All hydrogen atoms were added to the enzyme and their position optimized with the Tripos force field.

*Systematic conformational search for the disaccharide:* Both anomeric forms of the α-D-Glcp-(1→4)-D-Glc*p*NAc, were subjected to a systematic grid search study of the glycosidic linkage conformation. Starting from minimized disaccharides, a two-dimensional systematic conformational search was performed by rotating the two torsion angles defining the glycosidic linkages, Φ and Ψ by 20°steps: Φ = O5'-C1'-O4-C4 and Ψ = C1'-O4-C4-C3 for α-D-Glc*p*-(1→4)-D-Glc*p*NAc. The MM3 force field implemented in SYBYL 7.3 software was used for this purpose together with the energy parameters appropriate for carbohydrates. Different maps were constructed with the dielectric constant set to 4.0 and 78.0 (to mimic gas phase and water environment, respectively). The geometries were optimized at each point of the grid with the driver option that keeps fixed the atoms defining the torsion angles. The solvent specific relaxed conformational maps obtained for all disaccharides were then used to locate the different energy minima that were subsequently fully relaxed.

*Docking of disaccharide in the binding site of AS:* The lowest energy conformations identified on the disaccharide potential energy maps were used as starting structures to be docked in the binding site of amylosucrase. This was performed by superimposing the glucosyl unit of the disaccharides at (-1) subsite onto the glucosyl unit of the crystallographic maltoheptaose. Each of these AS-disaccharide complexes was optimized by means of the appropriate energy parameters. The annealing method implemented in SYBYL 7.3 software was used to optimize the complexes. Two shells of amino acids were considered: a 12Å shell centred on the binding site was taken into account for the energy calculations. A 6Å shell region closest to the carbohydrate was defined as the hot region to be optimized. The position of all atoms included in this region was optimized using Powell's method.

### Construction of mutant library

Single mutagenesis, focused on +1 subsite amino acids retained from ligand docking, was carried out with the QuickChange Site-Directed Mutagenesis Kit (Stratagene, La Jolla, CA) according to the manufacturer's instructions, and using pGST-AS G537D as vector template. It was checked that this mutation had no impact on the native enzyme catalytic properties. The complementary primers listed below were used to obtain the single mutant library (Table II below). XXX codon indicates the bases which were used to obtain the replacement by the desired amino acids and are listed in Table III below.

**Table II: Primers used to generate the 19 monomutants for the positions 228, 289, 290, 330, 331, 394 and 446.**

| SEQ ID | Primer | Nucleotide |
|---|---|---|
| NO: | Name | Sequence |
| 14 | I228for | 5'-ACC CTG CGC GAA XXX TTC CCC GAC CAG CA-3' |
| 15 | I228rev | 5'-TG CTG GTC GGG GAA XXX TTC GCG CAG GGT-3' |
| 16 | A289for | 5'-T ATG GAT GCG GTT XXX TTT ATT TGG AAA CAA AT-3' |
| 17 | A289rev | 5'-AT TTG TTT CCA AAT AAA XXX AAC CGC ATC CAT A-3' |
| 18 | F290for | 5'-T ATG GAT GCG GTT GCC XXX ATT TGG AAA CAA AT-3' |
| 19 | F290rev | 5'-AT TTG TTT CCA AAT XXX GGC AAC CGC ATC CAT A-3' |
| 20 | I330for | 5'-TC AAA TCC GAA GCC XXX GTC CAC CCC GAC CAA GT-3' |
| 21 | I330rev | 5'-AC TTG GTC GGG GTG GAC GAT XXX TTC GGA TTT GA-3' |
| 22 | V331for | 5'-TC AAA TCC GAA GCC ATC XXX CAC CCC GAC CAA GT-3' |
| 23 | V331rev | 5'-AC TTG GTC GGG GTG XXX GAT GGC TTC GGA TTT GA-3' |
| 24 | D394for | 5'-TC CGC AGC CAC GAC XXX ATC GGC TGG ACG TTT-3' |
| 25 | D394rev | 5'-AAA CGT CCA GCC GAT XXX GTC GTG GCT GCG GA-3' |
| 26 | R446for | 5'-ACA GGC GAC TGC XXX GTC AGT GGT ACA-3' |
| 27 | R446rev | 5'-TGT ACC ACT GAC XXX GCA GTC GCC TGT-3' |

**Table III: Sequence of XXX codon used to replace each selected amino acids by the 19 other ones**

| | | Ala | Cys | Asp | Glu | Phe | Gly | His | Ile | Lys | Leu | Met | Asn | Pro | Gln | Arg | Ser | Thr | Val | Trp | Tyr |
|---|---|---|---|---|---|---|---|---|---|---|---|---|---|---|---|---|---|---|---|---|---|
| 228 | for | GCC | TGC | GAC | GAG | TTC | GGC | CAC | wt | AAG | CTC | ATG | AAC | CCC | CAG | CGC | AGC | ACC | GTC | TGG | TAC |
| | rev | GGC | GCA | GTC | CTC | GAA | GCC | GTG | wt | CTT | GAG | CAT | GTT | GGG | CTG | GCG | GCT | GGT | GAC | CCA | GTA |
| 289 | for | wt | TGC | GAC | GAA | TTC | GGC | CAC | ATC | AAA | CTC | ATG | AAC | CCC | CAA | CGC | AGC | ACC | GTC | TGG | TAC |
| | rev | wt | GCA | GTC | TTC | GAA | GCC | GTG | GAT | TTT | GAG | CAT | GTT | GGG | TTG | GCG | GCT | GGT | GAC | CCA | GTA |
| 290 | for | GCT | TGT | GAT | GAA | wt | GGT | CAT | ATT | AAA | CTT | ATG | AAT | CCT | CAA | CGT | TCT | ACT | GTT | TGG | TAT |
| | rev | AGC | ACA | ATC | TTC | wt | ACC | ATG | AAT | TTT | AAG | CAT | ATT | AGG | TTG | ACG | AGA | AGT | AAC | CCA | ATA |
| 330 | for | GCC | TGC | GAC | GAA | TTC | GGC | CAC | wt | AAA | CTC | ATG | AAC | CCC | CAA | CGT | TCT | ACC | GTC | TGG | TAC |
| | rev | GGC | GCA | GTC | TTC | GAA | GCC | GTG | wt | TTT | GAG | CAT | GTT | GGG | TTG | ACG | AGA | GGT | GAC | CCA | GTA |
| 331 | for | GCC | TGC | GAC | GAA | TTC | GGC | CAT | ATC | AAA | CTC | ATG | AAC | CCC | CAA | CGC | TCC | ACC | wt | TGG | TAC |
| | rev | GGC | GCA | GTC | TTC | GAA | GCC | ATG | GAT | TTT | GAG | CAT | GTT | GGG | TTG | GCG | GGA | GGT | wt | CCA | GTA |
| 394 | for | GCC | TGC | wt | GAG | TTC | GGC | CAC | ATC | AAA | CTC | ATG | AAC | CCC | CAA | CGC | AGC | ACC | GTC | TGG | TAC |
| | rev | GGC | GCA | wt | CTC | GAA | GCC | GTG | GAT | TTT | GAG | CAT | GTT | GGG | TTG | GCG | GCT | GGT | GAC | CCA | GTA |
| 446 | for | GCT | TGT | GAT | GGT | TTT | GGT | CAT | ATT | AAA | CTT | ATG | AAT | CCT | CAA | wt | AGT | ACT | GTT | TGG | TAT |
| | rev | AGC | ACA | ATC | ACC | AAA | ACC | ATG | AAT | TTT | AAG | CAT | ATT | AGG | TTG | wt | ACT | AGT | AAC | CCA | ATA |

PCR amplification was carried out with Pfu DNA polymerase (2.5 U) for 16 cycles (95°C, 30s; 55°C, 30s; 72°C, 12min). The DNA was digested with Dpn*I* to eliminate methylated parental template and purified using Qiaquick spin column, following manufacturer's recommendations. E. *coli* JM109 was transformed with the plasmid and plated on LB agar supplemented with 100µg/mL ampicillin. For each construction, two clones were isolated and their corresponding plasmids stored at -20°C. 17 mutants (I228A₁, I228V₁, I228Y1, A289D₁, F290D₁, F290K1, F290Q₁, I330A₁, I330D₁, I330E₁, I330F₁, I330T₁, I330W₁, V331A₁, V331S₁, D394V₁ and R446K₁) were sequenced on the entire gene and showed no other mutations by Millegen (Labège, France) or Cogenics (Meylan, France).

### Expression of mutant library

The protocol was established to enable the rapid identification of clones for which D-GlcNAc*p* glucosylation was improved. To obtain higher amounts of enzymes and facilitate detection of glucosylated compounds upon HPLC screening, mutants were produced in 96-DeepWell Format plates. Storage microplates containing monomutants were thawed and replicated to inoculate a starter culture in 96-well microplates containing, in each well, 150µL LB medium supplemented with ampicillin (100µg/mL). After growth for 24h at 30°C under agitation (200 rpm), plates were duplicated into 96-Deep Well plates containing, in each well, 1.1 mL LB medium supplemented with ampicillin (100µg/mL) and IPTG (1mM) to induce GST-AS expression. Cultures were then grown for 24h at 30°C under agitation (200 rpm). Plates were centrifuged (20 min, 3000g, 4°C) and the supernatant was removed. The cell pellet was resuspended in 200µL of lysozyme (0.5 mg/mL), followed by freezing at -80°C for 8 to 12h. After thawing at room temperature, 100µL of sucrose and 100µL of acceptor (each at a final concentration of 73 mM) were added to each well. Enzymatic reaction was incubated at 30°C during 24h under agitation. The DeepWell plates were then centrifuged (20 min, 3000g, 4°C) and 300µL of the supernatant was transferred to a filter micro-plate (PVDF 0.2 µm) to be clarified. Supernatant filtration was carried out by centrifugation of the filter micro-plate (5 min, 2000g, 4°C) into a novel microplate for screening.

### Screening of mutant library

Efficiency of the glucosylation reaction was evaluated by HPLC analysis of the acceptor reaction product synthesized when using D-GlcNAc*p* as acceptor using a Dionex P 680 series pump, a Shodex RI 101 series refractometer, a Dionex UVD 340 UV/Vis detector and an autosampler HTC PAL. HPLC analyses were performed using a Biorad HPLC Carbohydrate Analysis column (HPX-87K column (300x7.8 mm)) maintained at 65°C, using ultra-pure water as eluent with a flow rate of 0.6 mL/min HPX-87K column was used to determine sucrose consumption by RI detection and concomitant α-D-Glc*p*-(1→4)-D-Glc*p*NAc formation by UV₌₂₂₀ₙₘ detection.

### Production and Purification of the Selected Variants: F290D and F290K

Production and purification of AS variants were performed as previously described (ref. 45). Since pure GST/AS fusion protein possesses the same function and the same efficiency as pure AS, enzymes were purified to the GST/AS fusion protein stage (96 kDa). The enzymes were desalted by size exclusion chromatography using P6DG columns (GE Healthcare Biosciences) at +4°C and stored in elution buffer (50 mM Tris-HCl, pH 7.0, 150 mM NaCl ) at -80°C. The protein content was determined by micro-Bradford method, using bovine serum albumin as standard (ref. 56).

### Biochemical Characterization of the Selected 1G5A Variants: F290D and F290K

All assays were performed at 30°C in 50 mM Tris buffer, pH=7.0. For the acceptor reactions, F290D, F290K and ASNPwt (1G5A) were tested on α-D-GlcNAc*p*OAll.

*Standard activity determination.* Specific activity of the purified enzymes was determined by measuring the initial rate of released fructose under standard conditions (146mM sucrose). Fructose concentration was determined using the dinitrosalycilic acid (DNS) method (ref. 57). One unit of AS variant corresponds to the amount of enzyme that catalyses the production of 1µmole fructose per minute in the assay conditions.

### Comparison of products synthesized by wild-type and AS variants.

Reactions were performed in the presence of 146 mM sucrose alone or supplemented with 146 mM acceptor. The purified wild-type or mutated GST/AS were employed at 0.5 U/mL. The reactions were stopped by heating at 95°C for 5 min. The final mixture was centrifuged at 18 000g for 10 min, filtered on 0.22 µm membrane and analyzed by HPLC, as previously described.

Different carbohydrate analyses were performed to compare the product profiles synthesized by ASNPwt and AS variant (F290K): Soluble and insoluble oligosaccharides produced during the reaction were identified by HPAEC using a Dionex Carbo-Pack PA100 column at 30°C. Before analysis, the insoluble fraction was solubilized in KOH at a final total sugar concentration of 10g/kg. Mobile phase (150mM NaOH) was set at 1mL/min flow rate with a sodium acetate gradient (going from 6 to 500 mM within 120 min). Detection was performed using a Dionex ED40 module with a gold working electrode and an Ag/AgCl pH reference. Note that α-D-GlcNAc*p*OAllyl (acceptor) and its derivatives are not oxidable products and thus are not detectable by HPAEC. Sucrose, glucose, fructose, α-D-GlcNAc*p*OAllyl (acceptor) and its derivatives (glucosylation products) were quantified by HPLC, as previously described
*Kinetics studies*. (k_{cat},K_{M})

Enzyme assays were carried out in a total volume of 2 mL containing pure enzyme (0.115 mg and 0.106 mg when using ASNPwt and F290K, respectively). Catalytic efficiency (Eff= k_{cat}/K_{M}) of ASNPwt and F290K variant was determined with both sucrose (D=Donor) and α-D-GlcNAc*p*OAll (A=Acceptor) as variable substrates. For the determination of the catalytic efficiency for D, A was held constant at 250 mM and D was varied between 0 and 600 mM. For the determination of the catalytic efficiency for A, D was held constant at 250 mM and A was varied between 0 and 250 mM. Experiments were performed until A and D solubility limits were reached. For each experiment, the reaction velocity corresponding to the acceptor glucosylation was determined by the formation of α-D-Glc*p*-(1→4)-α-D-GlcNAc*p*OAll (called Vi(Dp2)), corresponding to the kinetics of the reaction of interest.

Initial velocities were fitted to the Michaelis-Menten equation using Sigma-Plot. As saturation was not achieved with the mutant, efficiency was calculated by linear regression analysis of the velocity versus substrate concentration plot. Aliquots (200µL) were removed between 0 and 60 min (at which time product formation was still linear with respect to time), heated at 95°C for 5 min and centrifuged at 18 000g for 5 min. The final mixtures were filtered on a 0.22 µm membrane and analyzed using HPLC material previously described. HPLC analyses were performed using a Biorad HPLC Carbohydrate Analysis column (HPX-87K column (300x7.8 mm)) maintained at 65°C, using ultra-pure water as eluent with a flow rate of 0.6 mL/min to determine the released fructose by RI detection and to detect the formation of α-D-Glc*p*-(1→4)-α-D-GlcNAc*p*OAll by UV₌₂₂₀ₙₘ detection.

### Preparative synthesis of the acceptor reaction product

In order to characterize the products of D-GlcNAc*p* glucosylation, a synthesis was carried out at the preparative scale. 10 ml mixture containing 146 mM sucrose, 146 mM D-GlcNAc*p* and 1.5 U/ml of purified F290K AS mutant were incubated at 30°C for 24h. Then, the reaction mixture was centrifuged (4800 rpm, 20 min, 4°C) to remove proteins and filtered on 0.22 µm membrane.

In the same way, oligosaccharides produced from α-D-GlcNAc*p*OAll glucosylation were produced in a 100 mL mixture reaction (146 mM sucrose + 146 mM α-D-GlcNAc*p*OAll), using 4 U/mL of non-purified F290K extract (sonication supernatant).

### Purification of the oligosaccharides

Concerning D-GlcNAc*p* glucosylation, monoglucosylated product was separated by preparative chromatography on C 18 reverse-phase chromatography column (Bischoff Chromatography). Ultra pure water was used as eluent at a constant flow rate of 50 mL/min. Glucosyl detection was carried out with a refractometer (Bischoff) and each peak was collected separately, concentrated and reinjected into the analytical HPLC system described above, to verify the compound's purity.

### Structural analysis of the acceptor reaction products

The structure of Dp2, which was synthesized using sucrose as donor and D-GlcNAc*p* as acceptor with AS mutant F290K was analyzed by HRMS and NMR. It corresponds to α-D-glucopyranosyl-(1→4)-*N*-acetyl-D-glucosamine and was found identical to Dp2 formed by ASNPwt.

HRMS (FAB): Anal. Calcd for C₁₄H₂₅NO₁₁Na: 406.1325 [MNa⁺], Found: 406.1356; ¹H and ¹³C listed in the Table IV below.

**Table IV. ¹H and ¹³C chemical shifts (ppm, D₂O) and ³J coupling constants (Hz) of P2 resulting from F290K-mediated glucosylation of D-GlcpNAc**

| Compounds | Residue | δ (ppm) | Carbohydrate Ring | | | | | | N-Acetyl group | |
|---|---|---|---|---|---|---|---|---|---|---|
| | | | 1 | 2 | 3 | 4 | 5 | 6 | C | CH₃ |
| **P2** (β anomer) | α-D-Glc*p* | ¹H | 5.39 | 3.56 | 3.66 | 3.40 | 3.70 | 3.82 | | |
| | | ¹³C | 99.87 | 72.06 | 73.27 | 69.78 | 73.14 | 60.97 | | |
| | | | | | | | | | | |
| | β-D- Glc*p*NAc | ¹H | 4.70 | 3.68 | 3.79 | 3.69 | 3.56 | 3.82 | | 2.02 |
| | | | (J_{1.2}: 8.0 Hz) | | | | | | | |
| | | ¹³C | 95.25 | 57.09 | 74.79 | 77.22 | 75.06 | 61.00 | 175.22 | 22.62 |
| **P2** (α anomer) | α-D-Glc*p* | ¹H | 5.40 | 3.56 | 3.66 | 3.40 | 3.70 | 3.82 | | |
| | | ¹³C | 100.15 | 72.17 | 73.30 | 69.78 | 73.14 | 60.97 | | |
| | | | | | | | | | | |
| | α-D- Glc*p*NAc | ¹H | 5.18 | 3.89 | 4.00 | 3.66 | 3.94 | 3.82 | | 2.02 |
| | | (J_{1.2}: 3.6 Hz) | | | | | | | | |
| | | ¹³C | 91.13 | 54.36 | 71.62 | 77.94 | 70.57 | 61.00 | 174.98 | 22.34 |

### 2) Results

### Choice of acceptor substrates

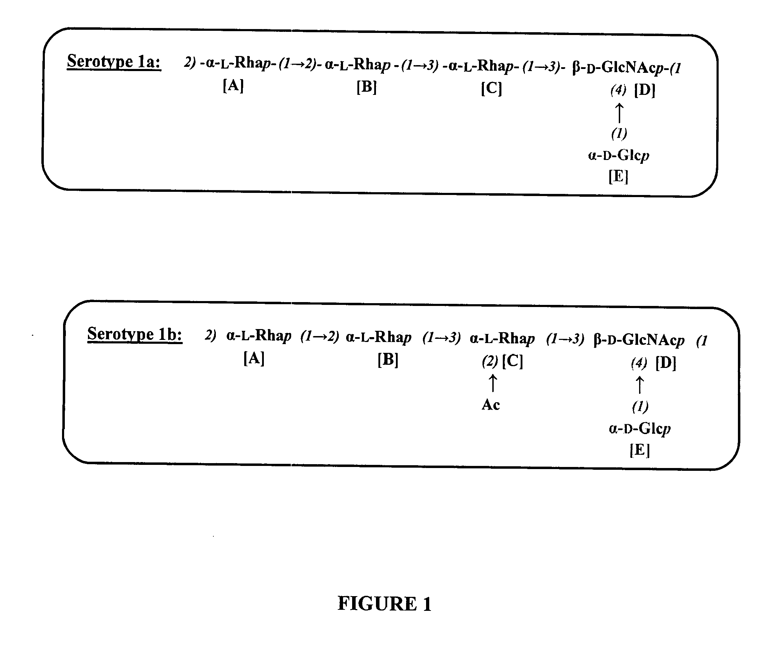

The choice of a suitable precursor to residue **D** (Figure 1) to be used as acceptors in the enzymatic steps to *S*. *flexneri* serotype 1a and 1b O-antigen was crucial. Three major features had to be taken into account: (i) light protecting pattern compatible with the limited ability of selected glucansucrases to modulate their acceptor binding site, (ii) easy synthetic access, and (iii) the possible conversion of the glucosylation product into a protected disaccharide building block known to be compatible with additional chemical elongation. The selection of an appropriate precursor to residue **D (D')** was made according to the same criteria. Allyl 2-*N*-acetyl-2-deoxy-α-D-glucopyranoside **(XX₁)** was selected based on the assumption that the 3_{D'}-OH group would be easily differentiated at the disaccharide level providing that a 2,3-oxazolidinone moiety could be introduced following *N*-deacetylation. Regioselective differentiation of the 3_{D'}-OH is indeed a pre-requirement to any specific chain elongation at this position as required in the synthesis of *S*. *flexneri* 1a and 1b oligosaccharides.

Thus, isolation of **ED' (XX₃)** was best performed following rough chromatography of the crude enzymatic glucosylation mixture issued from **D' (XX₁),** peracetylation into pure **XX₄,** then transesterification of intermediate **XX₄** (30% from **XX₁**) (Scheme 2A). To access *S. flexneri* oligosaccharides bearing the **ED** branching pattern, the product of **XX₁** enzymatic glucosylation, disaccharide **XX₃,** had to be turned into a donor allowing chain elongation at the reducing end **XX₁₀** (Scheme 2C) and/or an acceptor allowing chain extension at position 3_{D'} (**XX₁₆**) (Scheme 2D). Therefore, selective *N*-trichloroacetylation and per-*O*-acetylation of **XX₅**, resulting from extensive deaetylation of **XX₄,** gave **XX₁₄** (58%), which was converted, via hemiacetal **XX₁₃**, to trichloroacetimidate **XX₁₆** (26%) bearing a participating group at position 2_{D} as required. Alternatively, **XX₄** was saponified into **XX₅** (83%) (Scheme 2C), which was turned into the benzylated 2_{D},3_{D}-oxazolidinone **XX₈** via **XX₆** (34%). Oxazolidinone clivage and subsequent N-acetylation gave acceptor **XX₁₀** (80%) bearing a free hydroxyl group at position 3 and a participating group at position 2. Both disaccharide donor **XX₁₆** and disaccharide acceptor **XX₁₀** were converted to trisaccharides by reaction with a rhamnopyranoside acceptor or a rhamnopyranosyle donor.

### Screening for native transglucosidases able to synthesize the starting building block

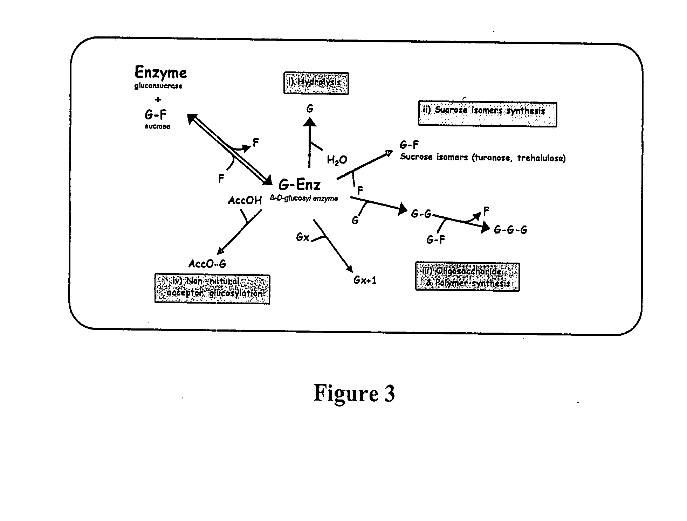

Selected α-retaining transglucosidases are glucansucrases belonging found in families 13 and 70 of glycoside-hydrolases (ref. 51). They catalyze the synthesis of α-glucan polymers by successive transfers of α-D-glucopyranosyl units from sucrose without any mediation of sugar nucleotides. Using the high energy of the sucrose bond to catalyze condensation reaction, they stand among the most efficient transglucosidases in the glycoside-hydrolase family. Depending on regiospecificity of the enzyme, distinct types of glucosidic linkage are found in the polymer formed. Notably, polymerization reaction can be redirected toward the glucosylation of exogenous acceptors, when the latter are well recognized (Figure 3). In addition, glucansucrases generally possess a broad acceptor spectrum, what indicates a certain plasticity of the acceptor recognition at the acceptor binding site. However, none of them had yet been tested for the glucosylation of the starting acceptor of interest. Glucosylation of D-Glc*p*NAc was thus attempted with four recombinant glucansucrases, which were selected for their very distinct specificities. For this first screening it has been preferred to start with commercially available D-GlcNAc*p* instead of the allyl-derivative, assuming that the modification with an allyl group at the anomeric position would not significantly affect the acceptor recognition as preliminary studies performed on D-Glc*p* and α-D-Glc*p*-OAlkyl derivatives with glucansucrases previously showed negligible differences. Enzymes specific for α-1,6 and α-1,3, α-1,2 or α-1,4 glucosidic bond formation were tested in the presence of the target acceptor. None of them was able to glucosylate this acceptor with the requisite regiospecificity and good yields.

Consequently, to overcome the limited substrate recognition by the enzymes, it was opted for the engineering of novel transglucosidases with altered regio and stereospecificities. The combination of combinatorial and rational enzyme engineering in the form of focused small size libraries was used. Further, the three-dimensional structure of AS (ref. 52) was available in complex with either the substrate or the natural reaction product.

### Engineering of amylosucrases able to glucosylate the target building block

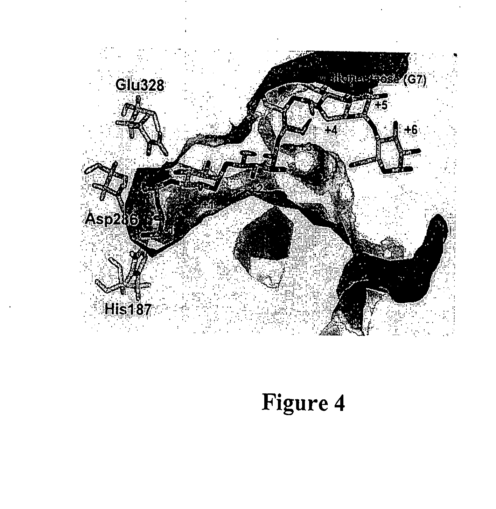

To modify enzyme specificity, an approach based on site-directed evolution targeted at the binding pocket was followed. The catalytic site pocket is defined by the subsites (-1) and (+1) according to the nomenclature earlier described for glycoside hydrolases (Figure 4). The subsite (-1) is responsible for the specificity towards sucrose and is occupied by the glucosyl unit which will be transferred whereas the subsite (+1) ensures a correct positioning of the acceptor and is also responsible for specificity of synthesis of the (α-1→4) glucan linkage (ref. 46)

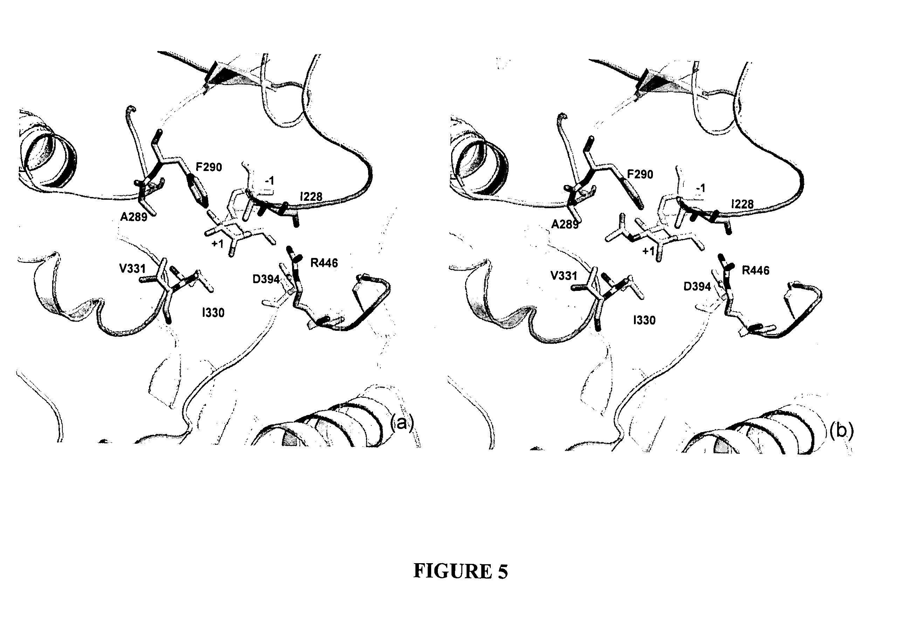

It was combined both the rational selection of mutation targets at the acceptor binding site (noted +1 subsite in Figure 4) and for each of the identified positions, a systematic modification of the residue by all 19 remaining possible amino acids. As (+1) subsite is responsible for the enzyme specificity toward acceptors, the approach consisted in *(i)* mapping the binding site residues important for functional plasticity and *(ii)* identifying the most promising positions to be modified to favour acceptor recognition. Starting from the crystallographic structure of AS in complex with sucrose (PDB: 1JGI) (ref. 54) and maltoheptaose (PDB: 1MW0) (ref. 55), the target disaccharide α-D-Glc*p*-(1→4)-D-Glc*p*NAc was docked in the AS active site (Figure 5).

### Structural analysis of α-D-Glcp-(1→4)-D-GlcpNAc [ED]: AS complex

The desired disaccharide α-D-Glc*p*-(1→4)-D-Glc*p*NAc was docked into the AS active site using the crystallographic maltose glucosyl units (*i*.*e*. α-D-Glc*p-*(1→4)-D-Glc*p*: native product) bound at (-1) and (+1) subsites (PDB: 1MW0) as a template for the starting location. As amylosucrase synthesizes naturally an α-(1-4) osidic linkage, the docking mode adopted by α-D-Glc*p*-(1→4)-D-Glc*p*NAc (target product) was close to the one observed for maltose glucosyl units at subsites (-1) and (+1) by crystallography. The unique difference between both disaccharides is the N-acetyl group at the C2 carbone of the D-Glc*p*NAc unit that substitutes the hydroxyl group carried by the D-Glc*p* in maltose. Modeling results indicated that to accommodate the D-Glc*p*NAc moiety at the (+1) subsite, several hydrophobic residues (Ala289, Phe290, Ile330 and Val331) surrounding the N-Acetyl group had to move away to provide enough fitting space. Monomutants for position Ile228, Asp394 and Arg446 have been also considered in the screen.

Out of the 18 residues identified as surrounding the (+1) subsite, 7 positions that were presumed to be not critical for sucrose binding but beneficial for target acceptor glucosylation have been selected for mutagenesis: Ile228, Ala289, Phe290, Ile330, Val331, Asp394 and Arg446. It was systematically mutated the selected amino acids by the 19 other possible residues to create a small size library focused on +1 subsite.

### Detection of mutants able to glucosylate the target acceptors

For each of the 7 selected positions, 19 single mutants were generated (corresponding to each possible amino acid change). Site-directed mutagenesis has been preferred to saturation mutagenesis, as each mono mutant generated is directly identified and can be easily isolated and characterized. A first library of 133 monomutants (7x19) was thus obtained and stored in cryotubes and 96-wells microplates. The mutants were tested for the glucosylation of the target acceptor D-Glc*p*NAc in microtiter format experiments. HPLC screening was performed to identify those able to form the desired disaccharide. Both sucrose consumption and disaccharide formation were determined in order to calculate the glucosylation rate defined as the molar ratio of monoglucosylated acceptor versus/sucrose consumed.

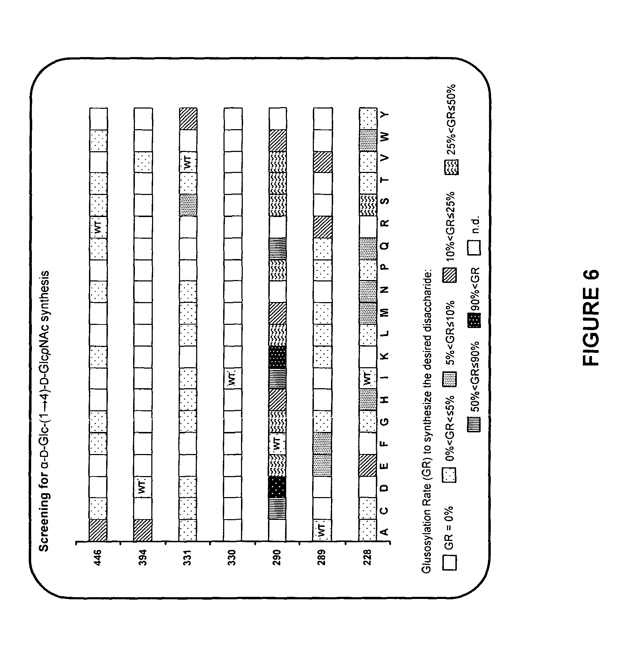

D-Glc*p*NAc was poorly recognized by the wild type AS (glucosylation rate=2%). The remodelling of the +1 subsite led to performant results. Indeed, 17 mutants catalyzed the formation of the α-D-Glc*p*-(1→4)-D-Glc*p*NAc with a glucosylation rate comprised between 10 and 50 % and 5 mutants with a glucosylation rate higher than 50 % (Figure 6). Position 290 is clearly a key position to improve the formation of α-D-Glc*p*-(1→4)-D-Glc*p*NAc. Of the 19 mutants, five synthesize the desired product with the correct regiospecificity and a glucosylation rate higher than 50%. Notably, F290D and F290K yielded the desired disaccharide with glucosylation rate of more than 90%, which represent a 45 fold increase compared to the wild type. Mutations at positions 228, 289, 331 and 446 also led to the improvement of the α-D-Glc*p*-(1→4)-D-Glc*p*NAc synthesis.

In overall, two mutants of interest for the chemo-enzymatic pathway were retained and further characterized F290D and F290K which are specific for the production of α-D-Glc*p*-(1→4)-D-Glc*p*NAc.

### Characterization of F290K and F290D 1G5A mutants

F290D and F290K were produced in a larger amount and purified to homogeneity for further characterization. Glucosylation reactions were performed using α-D-Glc*p*NAc-OAll as acceptor with F290D and F290K. The distribution of the acceptor reaction products is shown in Figure 7A. Regarding the variant F290K and F290D, both are highly specific for the glucosylation of αa-D-Glc*p*NAc-OAll. F290K and F290D yielded comparable amount of α-D-Glc*p*-(1-4)-α-D-Glc*p*NAc-OAll and are 10 times more efficient for α-D-Glc*p*NAc-OAll glucosylation; 20 times more efficient to form α-D-Glc*p*-(1→4)-D-Glc*p*NAc-OAll. The best catalytic efficiency is however obtained with the F290K mutant, which was thus selected to carry out the production of α-D-Glc*p*-(1→4)-D-Glc*p*NAc-OAll.

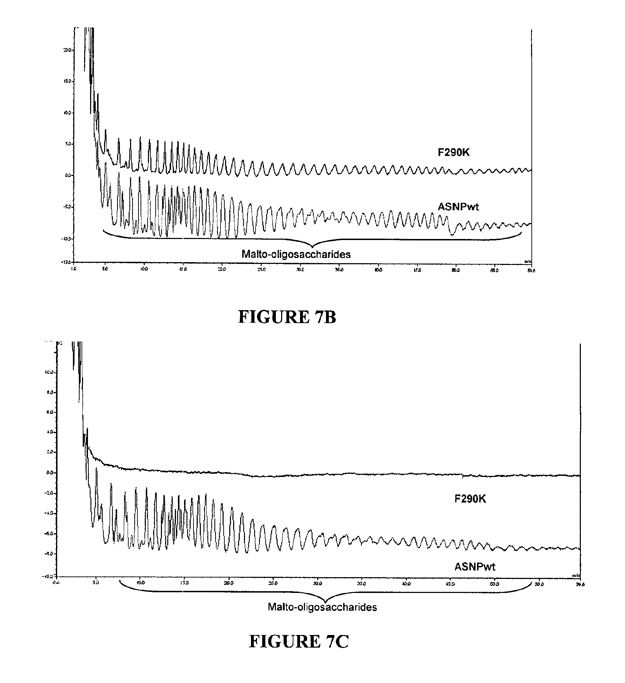

Regarding the characterization of the products formed by F290K mutant, we observed that addition of acceptor totally suppressed maltooligosaccharide formation (Figures 7B and 7C).Note that in the absence of acceptor, this mutant mainly catalyzed hydrolysis reaction but was still able to synthesize longer maltooligosaccharides (up to DP 20). Our assumption regarding the influence of the allyl aglycone in α-D-Glc*p*NAc-OAll on the glucosylation rate and regiospecificity of glucosaminyl acceptors was thus valid.

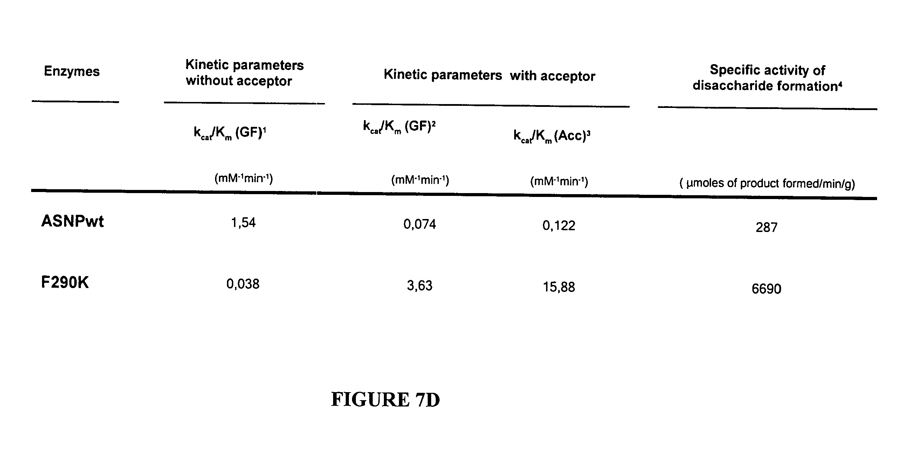

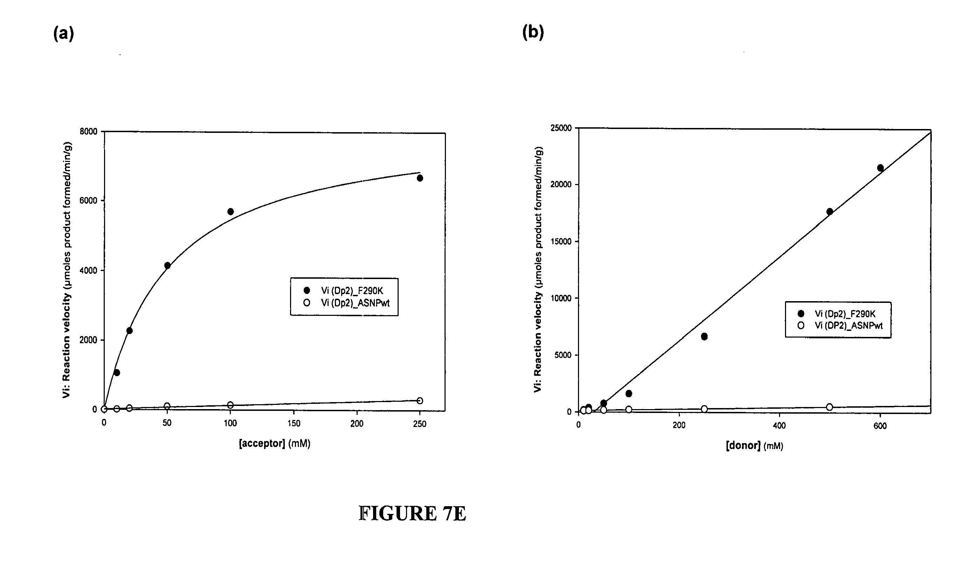

Kinetic measurements showed that F290K could not be saturated with sucrose, indicating thus a poor affinity for sucrose. k_{cat}/Km values were then determined by linear regression both in the presence and in the absence of acceptor (Figure 7D ). In the presence of sucrose alone, k_{cat}lKm values decreased by 40 fold for F290K mutant. In the presence of the acceptor, we observe for F290K mutant a formidable increase of catalytic efficiency towards both sucrose and the acceptor (Figures 7D and 7E). Such an improvement can be attributed to the high specificity of the mutant F290K for the α-D-Glc*p*-(1→4)-D-Glc*p*NAc-OAll formation. The adaptation of the acceptor binding site to the target acceptor enhanced the rate of the de-glucosylation step, which is no more the limiting step of the reaction. A remarkable 130 fold increase of the efficiency of the mutant compared to ASNPwt was observed and the expected regioselectivity was achieved.

### EXEMPLE 2: Enzymatic glucosylation of D-GlcpNAc derivatives

The mutant F290K and the ASNPwt were tested on other D-GlcNAc derivatives (α-D-GlcpNAc-OMe, β-D-GlcpNAc-OMe, β-D-GlcpNAc-Oallyl and D-GlcNHTCA ) used as potential acceptors. The enzymatic reaction was carried out using sucrose as donor and D-GlcNAc derivative as acceptor in a 1:1 ratio (146mM ) The distribution of the acceptor reaction products is shown in Figure 10. Comparing to ASNPwt, the variant F290K is highly specific for the glucosylation of all the other D-GlcNAc derivatives. (>95% for α-D-GlcpNAc-OMe, β-D-GlcpNAc-OAll and D-GlcNHTCA and 65-70% for β-D-GlcpNAc-OMe).

Efficiency of the glucosylation reaction was evaluated by HPLC analysis of the acceptor reaction product synthesized a Biorad HPLC Carbohydrate Analysis column (HPX-87K column (300x7.8 mm)) Percentages of glucosylation rates were estimated according to peak areas as no reference was available.

### EXAMPLE 3: Chemo-enzymatic synthesis of potential building blocks to oligosaccharide fragments of S. flexneri 1b and/or 1a O-antigens

**General methods.** All moisture sensitive reactions were carried out under an atmosphere of argon in oven-dried glassware. Anhydrous solvents sold on molecular sieves were used as such. 4 Å powder molecular sieves was kept under vacuum and activated before use by heating at 250 °C under vacuum. TLC were performed on precoated slides of Silica Gel 60 F₂₅₄ (Merck). Detection was effected with UV light, and/or by charring in 5% sulfuric acid in ethanol. Preparative chromatography was performed by elution from columns of Silica Gel 60 (particle size 0.040-0.063 mm). Products were routinely analyzed by ¹H and ¹³C NMR spectroscopy and by mass spectrometry (MS). NMR spectra were recorded at 25 °C (400 MHz for ¹H, 100 MHz for ¹³C). Proton-signal assignments were made by first-order analysis of the spectra, as well as analysis of 2D ¹H-¹H correlation maps (COSY). The ¹³C NMR assignments were supported by 2D ¹³C-¹H correlations maps (HSQC and HMBC). Signal assignments marked with a * are interchangeable assignments.

**Allyl 2-acetamido-2-deoxy-**α**-D-glucopyranoside (XX₁) (ref. 69).** A mixture of 2-acetamido-2-deoxy-D-glucose (50 g, 226 mmol), allyl alcohol (400 mL), and BF₃.OEt₂ (7.5 mL, 61 mmol) was heated at 90 °C. After 24 h, the reaction mixture was concentrated under reduced pressure, and the resulty gummy residue was purified by column chromatography (9:1 CH₂Cl₂-MeOH) to obtain compound **XX₁** as a white solid (49.6 g, 84%). Compound **XX₁** had Rf = 0.5 (85:15 CH₂Cl₂-MeOH); ¹H NMR (D₂O, 400 MHz) δ (ppm): 5.87 (m, 1H, CH=), 5.25 (m, 1H, =CH₂), 5.17 (m, 1H, =CH₂), 4.83 (d, 1H, *J*₁₋₂ = 3.6 Hz, H1), 4.13 (m, 1H, OCH₂), 3.94 (m, 1H, OCH₂), 3.82 (dd, 1H, *J*₂₋₃ = 10.7 Hz, H2), 3.78 (dd, 1H, *J*₅₋₆ₐ = 2.2 Hz, *J*_{6a-6b} = 12.0 Hz, H6a), 3.69 (dd, 1H, H6b), 3.67 (dd, 1H, *J*₃-₄ = 9.7 Hz, H3), 3.63 (ddd, 1H, *J*₄-₅ = 9.9 Hz, *J*_{5-6b} = 5.6 Hz, H5), 3.39 (t, 1H, H4), 1.94 (s, 3H, COCH₃); ¹³C NMR (D₂O, 100 MHz) δ (ppm): 174.5 (C=O), 133.7 (CH=), 117.9 (=CH₂), 96.2 (C1), 72.0 (C5), 71.1 (C3), 70.1 (C4), 68.5 (OCH₂), 60.6 (C6), 53.7 (C2), 21.9 (COCH₃). HRMS (ESI⁺) of C₁₁H₁₉NO₆Na ([M+Na]⁺, 284.1110) *m*/*z* 284.1112.

**Allyl** α**-D-glucopyranosyl-(1→4)-**α**-D-glucopyranosyl-(1→4)-2-acetamido-2-deoxy-**α**-D-glucopyranoside (XX₂).** The crude mixture issued from the enzymatic glucosylation of **XX₁** (3.80 g, 14.54 mmol) was purified by silica gel column chromatography (9:1 CH₃CN-H₂O) to give a mixture of allyl α-D-glucopyranosyl-(1→4)-2-acetamido-2-deoxy-α-D-glucopyranoside (**XX₃**) and fructose (4.16 g) and of the pure trisaccharide **XX₂** (2.53 g, 4.32 mmol) resulting from the enzyme-mediated transfer of glucose onto disaccharide **XX₃**. Trisaccharide **XX₂** had Rf = 0.3 (9:1 CH₃CN-H₂O). ¹H NMR (D₂O 400 MHz) δ (ppm): 5.91 (m, 1H, CH=), 5.35-5.32 (m, 2H, H1_{E}, H1_{E'}), 5.29 (m, 1H, =CH₂), 5.20 (m, 1H, =CH₂), 4.86 (d, 1H, *J*₁₋₂ = 3.5 Hz, H1_{D}), 4.15 (m, 1H, OCH₂), 4.01-3.93 (m, 2H, H3_{D}, OCH₂), 3.92-3.85 (m, 2H, H2_{D}, H3_{E}), 3.83-3.58 (m, 12H, H4_{D}, H5_{D}, H6a_{D}, H6b_{D}, H4_{E}, H5_{E}, H6a_{E}, H6b_{E}, H3_{E'}, H5_{E'}, H6a_{E'}, H6b_{E'}), 3.56 (dd overlapped, 1H, *J*₁-₂ = 4.0 Hz, *J*₂₋₃ = 10.0 Hz, H2_{E}), 3.52 (dd overlapped, 1H, *J*₁₋₂ = 3.8 Hz, *J*₂₋₃ = 9.8 Hz, H2_{E'}), 3.35 (pt, 1H, *J*₃-₄ = *J*₄-₅ = 9.3 Hz, H4_{E'}), 1.97 (s, 3H, COCH₃); ¹³C NMR (D₂O, 100 MHz) δ (ppm): 174.5 (C=O), 133.7 (CH=), 118.1 (=CH₂), 99.7 (C1_{E}, *J*_{C1-H1} = 171.6 Hz), 99.9 (C1_{E'}, *J*_{C1-H1} = 172.2 Hz), 96.0 (C1_{D}, *J* _{C1-H1} = 171.5 Hz), 77.7 (C4_{D}), 76.9 (C4_{E}), 73.4 (C3_{E}), 72.9 (C3_{E'}), 72.8 (C5_{E'}), 71.8, 71.6 (2C, C2_{E}, C2_{E'}), 71.5 (C3_{D}), 71.3 (C5_{E}), 70.5 (C5_{D}), 69.4 (C4_{E'}), 68.6 (OCH₂), 60.6 (3C, C6_{D}, C6_{E}, C6_{E'}), 53.5 (C2_{D}), 21.9 (COCH₃). HRMS (ESI⁺) for C₂₃H₃₉NO₁₆Na ([M+Na]⁺, 608.2167) *m*/*z* 608.2172.

**Allyl 2,3,4,6-tetra-*O*-acetyl-**α**-D-glucopyranosyl-(1→4)-2-acetamido-3,6-di-*O*-acetyl-2-deoxy-**α**-D-glucopyranoside (XX₄).** Acetic anhydride (80 mL, 725 mmol) was added dropwise to a solution of **XX₃** in mixture with fructose (4.16 g) in anhydrous pyridine (80 mL). The resulting mixture was stirred at room temperature under argon. After 2 days, TLC showed the complete disappearance of the starting materials. The mixture was concentrated under reduced pressure, and volatiles were eliminated by repeated coevaporation with toluene. The residue was purified by column chromatography (Cyclohexane-EtOAc, 6:4) to give **XX₄** (2.96 g, 30% from **XX₁**) as a colourless amorphous solid. Compound **XX₄** had Rf = 0.6 (95:5 CH₂Cl₂-MeOH). ¹H NMR (CACl₃, 400 MHz) δ (ppm): 5.90 (m, 1H, CH=), 5.71 (d, 1H, *J* = 9.7 Hz, NH), 5.45 (d, 1H, *J*₁₋₂ = 4.0 Hz, H1_{E}), 5.35 (dd, 1H, *J*₃-₄ = 9.6 Hz, H3_{E}), 5.34-5.23 (m, 3H, =CH₂, H3_{D}), 5.04 (pt, 1H, *J*₄-₅ = 9.8 Hz, H4_{E}), 4.85 (dd, 1H, *J*₂-₃ = 10.5 Hz, H2_{E}), 4.79 (d, 1H, *J*₁₋₂ = 3.6 Hz, H1_{D}), 4.42 (dd, 1H, *J*₅₋₆ₐ = 2.0 Hz, *J*_{6a-6b} = 12.0 Hz, H6a_{D}), 4.23-4.15 (m, 4H, OCH₂, H2_{D}, H6b_{D}, H6a_{E}), 4.04-3.91 (m, 5H, OCH₂, H4_{D}, H5_{D}, H5_{E}, H6b_{E}), 2.13, 2.08, 2.01, 2.00, 1.98, 1.91 (7s, 21H, COCH₃); ¹³C NMR (CDCl₃, 100 MHz) δ (ppm): 171.6, 170.6, 170.5, 170.2, 170.0, 169.4 (7C, C=O), 133.0 (CH=), 118.7 (=CH₂), 96.1 (C1_{D}), 95.5 (C1_{E}), 74.1 (C3_{D}), 72.4 (C4_{D}), 70.1 (C2_{E}), 69.4 (C3_{E}), 68.8 (OCH₂), 68.4 (C5_{E}), 68.0 (C5_{D}), 67.9 (C4_{E}), 62.8 (C6_{D}), 61.4 (C6_{E}), 52.2 (C2_{D}), 23.1, 21.0, 20.8, 20.7, 20.6, 20.5 (7C, COCH₃). HRMS (ESI⁺) for C₂₉H₄₁NO₁₇Na ([M+Na]⁺, 698.2272) *m*/*z* 698.2277.

**Allyl** α**-D-glucopyranosyl-(1-4)-2-acetamido-2-deoxy-**α**-D-glucopyranoside (XX₃).** *Method a:* Methanolic sodium methoxide (0.5 M solution, 8 mL, 4 mmol) was added to a stirred solution of **XX₄** (2.68 g, 3.97 mmol) in anhydrous methanol (100 mL). The reaction mixture was stirred for 6.5 h at room temperature by which time all the starting material had been consumed (Rf = 0.3 in 9:1 CH₃CN-H₂O). Excess base was neutralized with Dowex-H⁺ resin. The suspension was filtered, and the filtrate was concentrated under reduced pressure. The residue was purified by silica gel chromatography, eluting with 10% H₂O in CH₃CN to afford compound **XX₃** as a brownish foamy solid (1.65 g, 98%); ¹H NMR (D₂O 400 MHz) δ (ppm): 5.95 (m, 1H, CH=), 5.38 (d, 1H, *J*₁₋₂ = 3.9 Hz, H1_{E}), 5.34 (m, 1H, =CH₂), 5.25 (m, 1H, =CH₂), 4.91 (d, 1H, *J*₁₋₂ = 3.5 Hz, H1_{D}), 4.20 (m, 1H, OCH₂), 4.02 (m overlapped, 1H, OCH₂), 4.01 (dd overlapped, 1H, *J*₃₋₄ = 8.4 Hz, H3_{D}), 3.94 (dd, 1H, *J*₂₋₃ = 10.8 Hz, H2_{D}), 3.89-3.67 (m, 7H, H4_{D}, H5_{D}, H6a_{D}, H6b_{D}, H5_{E}, H6a_{E}, H6b_{E}), 3.66 (pt, 1H, H3_{E}), 3.56 (dd, 1H, *J*₂-₃ = 9.9 Hz, H2_{E}), 3.40 (pt, 1H, *J*₃-₄ = *J*₄-₅ = 9.5 Hz, H4_{E}), 2.02 (s, 3H, COCH₃); ¹³C NMR (D₂O, 100 MHz) δ (ppm): 174.5 (C=O), 133.7 (CH=), 118.1 (=CH₂), 99.9 (C1_{E}, *J*_{C1-H1} = 171.3 Hz), 96.0 (C1_{D}, *J* _{C1-H1} = 171.4 Hz), 77.6 (C4_{D}), 72.9 (C3_{E}), 72.8 (C5_{E}), 71.8 (C2_{E}), 71.5 (C3_{D}), 70.6 (C5_{D}), 69.4 (C4_{E}), 68.6 (OCH₂), 60.6 (2C, C6_{D}, C6_{E}), 53.5 (C2_{D}), 21.9 (COCH₃). HRMS (ESI⁺) for C₁₇H₃₀NO₁₁ ([M+H]⁺, 424.1819) *m*/*z* 424.1821.

*Method b:* Trisaccharide **XX₂** (2.19 g, 3.74 mmol) in solution in 110 mL of acetate buffer (pH 4.8) was incubated with 1120 IU of amyloglucosidase from *Aspergillus niger* with magnetic stirring for 90 min at 50 °C. After freeze-drying, the products were purified by silica gel chromatography eluting with 9:1 CH₃CN-H₂O. Concentration and freeze-drying gave a 1:1.1 mixture of disaccharide **XX₃** and D-glucose (1.43 g, 62%) as seen by NMR.

### EXAMPLE 4: Chemo-enzymatic synthesis of potential acceptor building blocks to oligosaccharide fragments of S. flexneri 1b and/or 1a O-antigens

### Allyl α-D-glucopyranosyl-(1→4)-2-amino-2-deoxy-α-D-glucopyranoside (XX₅).

*Method a:* Disaccharide **XX₃** (784 mg, 1.85 mmol) was treated with Ba(OH)₂.8 H₂O (10 g) in water (50 mL), and the mixture was stirred at 90 °C overnight. When *N*-deacetylation was completed (Rf = 0.5 in 4:1:0.5 *i*PrOH-H₂O-conc. NH₄OH), the cooled mixture was saturated with CO₂, the volume was diminished to ∼5 mL, EtOH (30 mL) was added, and solids were sedimented by centrifugation (0 °C, 666 x g, 2000 r.p.m., 10 min.). The supernatant was decanted, the residue was re-extracted with EtOH, and supernatants were pooled. The residue remaining after solvent removal was taken up in 13:7:2 CH₂Cl₂-MeOH-conc. NH₄OH, and passed through a layer (8 x 3) of silica gel wet with the same solvent. Pooled fractions containing the product were concentrated and freeze-dried to give **XX₅** as a solid (587 mg; 83%). ¹H NMR (D₂O, 400 MHz) δ (ppm): 5.88 (m, 1H, CH=), 5.30 (d overlapped, 1H, H1_{E}), 5.27 (m, 1H, =CH₂), 5.18 (m, 1H, =CH₂), 4.97 (d, 1H, *J*₁₋₂ = 3.7 Hz, H1_{D}), 4.15 (m, 1H, OCH₂), 3.98 (m, 1H, OCH₂), 3.93 (dd, 1H, *J*₂₋₃ = 10.5 Hz, *J*₃₋₄ = 8.8 Hz, H3_{D}), 3.80-3.62 (m, 5H, H5_{D}, H6a_{D}, H6b_{D}, H6a_{E}, H6b_{E}), 3.62-3.57 (m, 2H, H4_{D}, H5_{E}), 3.57 (dd overlapped , 1H, *J*₃₋₄ = 9.1 Hz, H3_{E}), 3.48 (dd, 1H, *J*₁₋₂ = 4.0 Hz, *J*₂-₃ = 9.9 Hz, H2_{E}), 3.31 (pt, 1H, *J*₄-₅ = 9.6 Hz, H4_{E}), 3.03 (dd, 1H, H2_{D}); ¹³C NMR (D₂O, 100 MHz) δ (ppm): 133.4 (CH=), 118.5 (=CH₂), 99.7 (C1_{E}), 95.8 (C1_{D}), 76.7 (C4_{D}), 72.9 (C5_{E}*), 72.8 (C3_{E}*), 72.1 (C3_{D}), 71.7 (C2_{E}), 70.7 (C5_{D}), 69.4 (C4_{E}), 68.8 (OCH₂), 60.5, 60.4 (2C, C6_{D}, C6_{E}), 54.2 (C2_{D}). HRMS (ESI⁺) for C₁₅H₂₈NO₁₀ ([M+H]⁺, 382.1713) *m*/*z* 382.1719.

*Method b:* One-pot removal of *O*-acetyl and N-acetamide groups in disaccharide **XX₄** followed the above mentioned procedure. Briefly, **XX₄** (5.05 g, 7.47 mmol) was treated overnight with Ba(OH)₂.8 H₂O (25 g) in water (50 mL) at 90 °C. After neutralization with dry ice and repeated centrifugations (0 °C, 666 x g, 2000 r.p.m., 10 min.), compound **XX₅** was purified by silica gel chromatography eluting with 13:7:2 CH₂Cl₂-MeOH-conc. NH₄OH (2.39 g, 84%).

**Allyl** α**-D-glucopyranosyl-(1→4)-2-amino-2-*N*,3-*O*-carbonyl-2-deoxy-**α**-D-glucopyranoside (XX₆)**. An ice-cooled solution of p-nitrophenoxycarbonyl chloride (2.50 g, 12.6 mmol) in acetone (25 mL) was added over several minutes to a stirred, ice bath-cooled, mixture of **XX₅** (1.60 g, 4.2 mmol) and methanolic NaOMe (25% w/w solution, 3.5 mL, 12.6 mmol) in 50 mL methanol. The mixture was vigorously stirred with ice-bath cooling for 30 min, then at room temperature for 1 h. Water (100 mL) was added and the resulting aqueous mixture was extracted with diethyl ether. The aqueous layer was made acidic (pH ∼3) by dropwise addition of 10% hydrochloric acid solution. The acidified aqueous phase was extracted with diethyl ether to remove *p*-nitrophenol until the etheral layer failed to turn yellow upon when NaHCO₃ addition. The aqueous solution was then neutralized with Amberlyst-A26 (OH⁻ form) resin, filtered, and evaporated to give crude oxazolidinone **XX₆** (1.70 g). ¹H NMR (CD₃OD, 400 MHz) δ (ppm): 5.98 (m, 1H, CH=), 5.37 (m, 1H, =CH₂), 5.29 (d, 1H, *J*₁₋₂ = 3.8 Hz, H1_{E}), 5.22 (m, 1H, =CH₂), 5.17 (d, 1H, *J*₁₋₂ = 2.9 Hz, H1_{D}), 4.78 (dd, 1H, *J*₂₋₃ = 12.0 Hz, *J*₃₋₄ = 9.8 Hz, H3_{D}), 4.30 (m, 1H, OCH₂), 4.29 (pt overlapped, 1H, H4_{D}), 4.13 (m, 1H, OCH₂), 3.86 (dd, 1H, *J*₅₋₆ₐ = 2.3 Hz, *J*_{6a-6b} = 11.8 Hz, H6a_{E}), 3.83 (dd, 2H, *J*₅-₆ = 3.1 Hz, H6a_{D}, H6b_{D}), 3.77 (dt, 1H, *J*₄₋₅ = 9.3 Hz, H5_{D}), 3.72 (dd, 1H, *J*_{5-6b} = 5.4 Hz, H6b_{E}), 3.68 (dd, 1H, H2_{D}), 3.65-3.59 (m overlapped, 2H, H3_{E}, H5_{E}), 3.46 (dd, 1H, *J*₂₋₃ = 9.8 Hz, H2_{E}), 3.35 (dd, 1H, *J*₃₋₄ = 9.0 Hz, *J*₄₋₅ = 9.8 Hz, H4_{E}); ¹³C NMR (CD₃OD, 100 MHz) δ (ppm): 160.6 (C=O), 133.7 (CH=), 116.3 (=CH₂), 97.4 (C1_{E}), 94.8 (C1_{D}), 79.5 (C3_{D}), 73.3 (C3_{E}*), 73.1 (2C, C5_{D}, C5_{E}*), 71.8 (C2_{E}), 71.0 (C4_{D}), 70.2 (C4_{E}), 68.5 (OCH₂), 61.1 (C6_{E}), 60.2 (C6_{D}), 58.4 (C2_{D}). HRMS (ESI⁺) for C₁₆H₂₅NO₁₁Na ([M+Na]⁺, 430.1325) *m*/*z* 430.1331.

**Allyl 2,4,6-tri-*O*-benzyl-**α**-D-glucopyranosyl-(1→4)-6-*O*-benzyl-2-benzylamino-2-*N*,3-*O*-carbonyl-2-deoxy-**α**-D-glucopyranoside (XX₇).** 60% NaH in oil (57 mg, 1.42 mmol) was added portionwise to an ice-cold mixture of the crude oxazolidinone **XX₆** (108 mg) and benzyl bromide (0.19 mL, 1.60 mmol) in DMF (5 mL). After stirring for 30 min at 0 °C, the reaction mixture was warmed up and stirred for an additional 5.5 h at room temperature. The reaction was quenched by addition of Et₃N, diluted with EtOAc, and the mixture was washed with brine, dried (Na₂SO₄), filtered, and concentrated. The residue was purified by flash-chromatography (4:1 Cyclohexane-EtOAc) to afford **XX₈** (48 mg, 19% over 2 steps) and the corresponding 3-hydroxyl compound **XX₇** (39 mg, 17% over two steps); Rf = 0.2 (3:1 Cyclohexane-EtOAc). ¹H NMR (CDCl₃, 400 MHz) δ (ppm): 7.45-7.22 (m, 25H, H arom.), 5.76 (m, 1H, CH=), 5.54 (d, 1H, *J*₁₋₂ = 3.6 Hz, H1_{E}), 5.30-5.20 (m, 2H, =CH₂), 4.86 (d overlapped, 1H, CH₂Ph), 4.84 (d overlapped, 1H, CH₂Ph), 4.76 (d, 1H, *J*₁₋₂ = 2.8 Hz, H1_{D}), 4.65 (dd overlapped, 1H, H3_{D}), 4.64 (d overlapped, 1H, CH₂Ph), 4.54 (d overlapped, 1H, CH₂Ph), 4.52 (d overlapped, 1H, CH₂Ph), 4.48 (m, 2H, CH₂Ph), 4.41 (m, 2H, CH₂Ph), 4.33 (d overlapped, 1H, CH₂Ph), 4.30 (pt overlapped, 1H, *J*₃₋₄ = *J*₄₋₅ = 9.6 Hz, H4_{D}), 4.02 (m overlapped, 1H, OCH₂), 4.00 (pt overlapped, 1H, H3_{E}), 3.81 (ddd, 1H, H5_{D}), 3.76 (dd, 1H, *J*₅₋₆ₐ = 3.7 Hz, *J*_{6a-6b} = 11.0 Hz, H6a_{D}), 3.70-3.57 (m, 4H, OCH₂, H6b_{D}, H4_{E}, H5_{E}), 3.54 (dd, 1H, *J*₅₋₆ₐ = 2.4 Hz, *J*_{6a-6b} = 10.7 Hz, H6a_{E}), 3.65 (dd 1H, *J*₂₋₃ = 9.7 Hz, H2_{E}), 3.41 (dd overlapped, 1H, *J*_{5-6b} = 1.2 Hz, H6b_{E}), 3.34 (dd, 1H, *J*₂₋₃ = 11.8 Hz, H2_{D}); ¹³C NMR (CDCl₃, 100 MHz) δ (ppm): 158.6 (C=O), 138.6, 138.0, 137.8, 137.6, 135.3 (5C, C quat. arom.), 133.1 (CH=), 128.8, 128.7, 128.6, 128.5, 128.3, 128.2, 128.1, 127.9, 127.8, 127.6, 127.3 (25C, C arom.), 118.4 (=CH₂), 94.8 (C1_{E}), 94.3 (C1_{D}), 78.7 (C2_{E}), 77.2 (C4_{E}), 76.9 (C3_{D}), 74.6, 73.5 (3C, OCH₂Ph), 73.2 (C3_{E}), 72.5 (OCH₂Ph), 72.1 (C5_{D}), 71.0 (C4_{D}), 70.9 (C5_{E}), 69.1 (OCH₂), 68.3 (C6_{D}), 68.1 (C6_{E}), 61.1 (C2_{D}), 48.7 (NCH₂Ph). HRMS (ESI⁺) for C₅₁H₅₅NO₁₁Na ([M+Na]⁺, 880.3673) *m*/*z* 880.3704.

**Allyl 2,3,4,6-tetra-*O*-benzyl-**α**-D-glucopyranosyl-(1→4)-6-*O-*benzyl-2-benzylamino-2-*N*,3-*O*-carbonyl-2-deoxy-**α**-D-glucopyranoside (XX₈).** Methanolic sodium methoxide (0.5 M solution, 1.6 mL, 0.79 mmol) and trichloroethylchloroformate (225 µL, 1.56 mmol) were added to a solution of compound **XX₅** (100 mg, 0.26 mmol) in methanol (3.4 mL) at 0 °C. After 4 h, the reaction mixture was neutralized by addition of Dowex-H⁺ resin, filtered, and concentrated to dryness to afford the crude trichloroethyl carbamate. This material (Rf = 0.7, 85:15 CH₃CN-H₂O) was used directly without further purification.

NaH 60% in oil (63 mg, 2.60 mmol) was added to an ice-cold mixture of the crude trichloroethyl carbamate and benzyl bromide (0.31 mL, 2.60 mmol) in DMF (5 mL). The reaction mixture was stirred for 30 min at 0 °C, then stirred for an additional 4.5 h at room temperature. The reaction was quenched by addition of Et₃N, diluted with EtOAc, poured into saturated aqueous NH₄Cl, and extracted with EtOAc. The combined organic extracts were washed with water and brine, dried (Na₂SO₄), filtered, and concentrated. The residue was purified by flash-chromatography (17:3 Cyclohexane-EtOAc) to afford **XX₈** (81 mg, 34% over 2 steps); Rf = 0.3 (4:1 Cyclohexane-EtOAc). ¹H NMR (CDCl₃, 400 MHz) δ (ppm): 7.46-7.16 (m, 30H, H arom.), 5.79 (m, 1H, CH=), 5.56 (d, 1H, *J*₁₋₂ = 3.5 Hz, H1_{E}), 5.26 (m, 2H, =CH₂), 5.02 (d overlapped, 1H, CH₂Ph), 4.87-4.80 (m, 3H, CH₂Ph), 4.79-4.72 (m overlapped, 3H, H1_{D}, H3_{D}, CH₂Ph), 4.56-4.49 (m, 4H, CH₂Ph), 4.47 (m, 2H, CH₂Ph), 4.35 (pt overlapped, 1H, H4_{D}), 4.34 (d overlapped, 1H, CH₂Ph), 4.05 (m, 1H, OCH₂), 3.92-3.88 (m, 2H, H5_{D}, H3_{E}), 3.81 (dd, 1H, *J*₅₋₆ₐ = 3.5 Hz, *J*_{6a-6b} = 11.0 Hz, H6a_{D}), 3.73-3.66 (m, 4H, OCH₂, H6b_{D}, H4_{E}, H5_{E}), 3.65 (dd overlapped, 1H, H2_{E}), 3.57 (dd, 1H, *J*₅₋₆ₐ = 2.7 Hz, *J*_{6a-6b} = 10.8 Hz, H6a_{E}), 3.43 (dd, 1H, *J*_{5-6b} = 1.4 Hz, H6b_{E}), 3.37 (dd, 1H, *J*₁₋₂ = 2.7 Hz, *J*₂₋₃ = 11.8 Hz, H2_{D}); ¹³C NMR (CDCl₃, 100 MHz) δ (ppm): 158.7 (C=O), 138.9, 138.5, 138.1, 137.9, 137.7, 135.3 (6C, C quat. arom.), 133.1 (CH=), 128.8, 128.7, 128.5, 128.4, 128.3, 128.2, 127.9, 127.8, 127.6, 127.5, 127.3 (30C, C arom.), 118.3 (=CH₂), 95.4 (C1_{E}), 94.4 (C1_{D}), 81.8 (C3_{E}), 79.5 (C2_{E}), 77.4 (C4_{E}), 76.9 (C3_{D}), 75.6, 75.1, 73.5, 73.1 (5C, OCH₂Ph), 72.1 (C5_{D}), 71.3 (C5_{E}), 70.9 (C4_{D}), 69.1 (OCH₂), 68.4 (C6_{D}), 68.2 (C6_{E}), 61.2 (C2_{D}), 48.7 (NCH₂Ph). HRMS (ESI⁺) for C₅₈H₆₁NO₁₁Na ([M+Na]⁺, 970.4142) *m*/*z* 970.4107.

**Allyl 2,3,4,6-tetra-*O*-benzyl-**α**-n-glucopyranosyl-(1→4)-6-*O-*benzyl-2-amino-2-*N*,3-*O*-carbonyl-2-deoxy-**α**-D-glucopyranoside (XX_{8A}).** NaH (60% in oil, 368 mg, 9.21 mmol) was added portionwise to a stirred solution of the crude oxazolidinone **XX₆** (500 mg, 1.23 mmol) in dry DMF (10 mL) at 0 °C. After 1 h, the mixture was treated with benzyl bromide (800 µL, 6.75 mmol), stirred overnight, treated with MeOH, and concentration to dryness. The residue was purified by flash-chromatography (85:15 Toluene-EtOAc) to afford **XX₈** (74 mg, 6% over 2 steps), and the corresponding penta-*O*-benzyl derivative **XX_{8A}** (158 mg, 15%); Rf = 0.3 (85:15 Toluene-EtOAc). ¹H NMR (CDCl₃, 400 MHz) δ (ppm): 7.35-7.17 (m, 25H, H arom.), 5.80 (m, 1H, CH=), 5.57 (d, 1H, *J*₁₋₂ = 3.5 Hz, H1_{E}), 5.21 (m, 1H, =CH₂), 5.13 (m, 2H, =CH₂), 4.95 (d, 1H, CH₂Ph), 4.86 (d overlapped, 1H, CH₂Ph), 4.83 (d overlapped, 1H, CH₂Ph), 4.78 (d overlapped, 1H, CH₂Ph), 4.77 (d, 1H, *J*₁₋₂ = 3.6 Hz, H1_{D}), 4.71-4.49 (m, 5H, CH₂Ph), 4.37 (d overlapped, 1H, CH₂Ph), 4.29 (ddd, 1H, *J*₂₋₃ = 10.1 Hz, H2_{D}), 4.19 (pt, 1H, *J*₃₋₄ = *J*₄₋₅ = 9.2 Hz, H4_{D}), 4.06 (m, 1H, OCH₂), 3.97 (pt overlapped, 1H, H3_{E}), 3.95 (dd overlapped, 1H, H6a_{D}), 3.91-3.81 (m, 3H, H3_{D},H5_{D}, H5_{E}), 3.76-3.64 (m, 3H, OCH₂, H6b_{D}, H4_{E}), 3.57 (dd, 1H, *J*₅₋₆ₐ = 3.2 Hz, *J*_{6a-6b} = 10.8 Hz, H6a_{E}), 3.52 (dd 1H, *J*₂₋₃ = 9.8 Hz, H2_{E}), 3.48 (dd, 1H, *J*_{5-6b} = 1.4 Hz, H6b_{E}); ¹³C NMR (CDCl₃, 100 MHz) δ (ppm): 156.4 (C=O), 138.8, 138.6, 138.4, 138.1, 135.3 (5C, C quat. arom.), 133.8 (CH=), 128.3, 128.1, 128.0, 127.9, 127.8, 127.6, 127.5, 127.4 (25C, C arom.), 117.6 (=CH₂), 97.4 (C1_{D}), 96.7 (C1_{E}), 82.0 (C3_{E}), 80.5 (C3_{D}), 79.5 (C2_{E}), 77.7 (C4_{E}), 75.6, 75.0, 73.2, 72.9 (4C, OCH₂Ph), 72.6 (C4_{D}), 71.7 (OCH₂Ph), 71.1 (C5_{E}), 70.7 (C5_{D}), 69.0 (C6_{D}), 68.4 (C6_{E}), 68.4 (OCH₂), 52.8 (C2_{D}). HRMS (ESI⁺) for C₅₁H₅₅NO₁₁Na ([M+Na]⁺, 880.3673) *m*/*z* 880.3710.

**Allyl 2,3,4,6-tetra-*O*-benzyl-**α**-D-glucopyranosyl-(1-4)-6-*O-*benzyl-2-benzylamino-2-deoxy-**α**-D-glucopyranoside (XX₉).** Perbenzylated **XX₈** (36 mg, 0.04 mmol) was treated with 1 M aqueous NaOH/1,4-dioxane (1:1, v/v, 10 mL) at 65 °C for 2 days. The mixture was diluted with EtOAc and washed with water. The separated aqueous layer was extracted with EtOAc. The combined organic extracts were washed with water and brine, dried (Na₂SO₄), filtered and concentrated. The residue was purified by flash-chromatography (4:1 Cyclohexane-EtOAc) to afford target **XX₉** (32 mg, 91%) as an oil; Rf = 0.1 (4:1 Cyclohexane-EtOAc). ¹H NMR (CDCl₃, 400 MHz) δ (ppm): 7.41-7.16 (m, 30H, H arom.), 5.95 (m, 1H, CH=), 5.33 (m, 1H, =CH₂), 5.24 (d overlapped, 1H, *J*₁₋₂ = 3.4 Hz, H1_{E}), 5.23 (m, 1H, =CH₂), 4.96 (d, 1H, CH₂Ph), 4.90-4.76 (m, 4H, CH₂Ph, H1_{D}), 4.57 (d overlapped, 1H, CH₂Ph), 4.55 (d overlapped, 1H, CH₂Ph), 4.50 (d overlapped, 1H, CH₂Ph), 4.46 (d overlapped, 1H, CH₂Ph), 4.40 (d overlapped, 1H, CH₂Ph), 4.16 (m, 1H, OCH₂), 4.00 (pt overlapped, 1H, H3_{E}), 3.99-3.95 (m, 4H, OCH₂, CH₂Ph, H3_{D}), 3.85-3.77 (m, 2H, H5_{D}, H5_{E}), 3.76-3.63 (m, 4H, H4_{D}, H4_{E}, H6a_{D}, H6b_{D}), 3.62-3.56 (m, 2H, H2_{E}, H6a_{E}), 3.47 (dd, 1H, *J*_{5-6b} = 1.8 Hz, *J*_{6a6b} = 10.6 Hz, H6b_{E}), 2.80 (dd, 1H, *J*₁₋₂ = 3.5 Hz, *J*₂₋₃ = 10.2 Hz, H2_{D}); ¹³C NMR (CDCl₃, 100 MHz) δ (ppm): 138.7, 138.4, 138.3, 137.9, 135.4 (6C, C quat. arom.), 134.0 (CH=), 128.6, 128.5, 128.4, 128.3, 128.1, 127.9, 127.8, 127.7, 127.6, 127.5, 127.4, 127.1 (30C, C arom.), 117.7 (=CH₂), 99.4 (C1_{E}), 96.3 (C1_{D}), 82.1 (C3_{E}), 80.3 (C4_{D}), 79.7 (C2_{E}), 77.7 (C4_{E}), 75.7, 75.0, 73.7, 73.5, 73.2 (5C, OCH₂Ph), 73.2 (C3_{D}), 71.2 (C5_{E}), 69.5 (C5_{D}), 69.1 (C6_{D}), 68.5 (C6_{E}), 68.4 (OCH₂), 61.1 (C2_{D}), 51.9 (NCH₂Ph). HRMS (ESI⁺) for C₅₇H₆₄NO₁₀ ([M+H]⁺, 922.4530) m/z 922.4540.

**Allyl 2,3,4,6-tetra-*O*-benzyl-**α**-D-glucopyranosyl-(1→4)-6-*O-*benzyl-2-benzylacetamido-2-deoxy-**α**-D-glucopyranoside (XX₁₀)**.

*Method a:* To a solution of disaccharide **XX₉** (424 mg, 0.46 mmol) in anhydrous pyridine (5 mL) was added dropwise anhydride acetic (250 µL, 2.65 mmol). The resulting mixture was stirred at room temperature under argon. After 24 h, TLC showed the complete disappearance of the starting materials. The mixture was concentrated under reduced pressure, and volatiles were eliminated by repeated coevaporation with toluene. The residue was purified by column chromatography (Cyclohexane-EtOAc, 2:1) to give **XX₁₀** (391 mg, 88%) as a colourless oil. Compound **XX₁₀** had Rf = 0.2 (2:1 Cyclohexane-EtOAc). NMR analysis indicated an equilibrium between two rotamers.

*Method b:* Perbenzylated **XX₈** (125 mg, 0.13 mmol) was treated with 1 M aqueous NaOH/1,4-dioxane (1:1, v/v, 15 mL) at 65 °C for 2 days. The mixture was diluted with EtOAc and washed with water. The separated aqueous layer was extracted with EtOAc. The combined organic extracts were washed with water and brine, dried (Na₂SO₄), filtered, and concentrated to give crude **XX₉** (109 mg, 0.12 mmol) as an oil.

Anhydride acetic (56 µL, 0.59 mmol) was added to a solution of crude disaccharide **XX₉** (109 mg, 0.12 mmol) in anhydrous pyridine (1 mL). The mixture was stirred at room temperature under argon. After 5 h, TLC showed the complete disappearance of the starting materials. Volatiles were eliminated by repeated coevaporation with toluene under reduced pressure. The residue was purified by column chromatography (Cyclohexane-EtOAc, 2:1) to give **XX₁₀** (98 mg, 77%) as a colourless oil. ¹H NMR (CDCl₃, 400 MHz) δ (ppm): 7.37-7.15 (m, 30H, H arom.), 5.50-5.40 (m, 1H, CH=), 5.10-4.76 (m, 7H, H1_{D}, H1_{E}, H2_{D}, =CH₂, OCH₂Ph), 4.75-4.65 (m, 3H, OCH₂Ph, NCH₂Ph), 4.60-4.38 (m, 5H, OCH₂Ph), 4.27-4.12 (m, 0.9H, H3_{D}, OCH₂), 4.04-3.91 (m, 2.1H, H3_{D}, OCH₂), 3.91-3.81 (m, 1H, H5_{E}), 3.80-3.50 (m, 6H, H2_{E}, H4_{D}, H4_{E}, H6a_{D}, H6b_{D}, H6a_{E}), 3.50-3.38 (m, 1H, H6b_{E}), 2.31 (s, 0.9H, COCH₃), 1.99 (s, 2.1H, COCH₃); ¹³C NMR (CDCl₃, 100 MHz) δ (ppm): 173.9, 172.5 (C=O), 139.8, 139.4, 138.5, 138.2, 137.8, 136.9 (C quat. arom.), 133.5 (CH=), 128.8, 128.7, 128.6, 128.4, 128.3, 128.0, 127.9, 127.7, 127.6, 127.5, 127.4, 126.6, 126.4, 125.9 (C arom.), 117.8, 117.3 (=CH₂), 100.8 (C1_{E}), 98.0, 97.7 (C1_{D}), 83.8 (C4_{D}), 82.2 (C3_{E}), 79.4 (C2_{E}), 77.7 (C4_{E}), 75.7, 75.6, 75.0, 74.2, 73.5, 73.3, 73.1 (OCH₂Ph), 71.3 (C5_{E}), 70.0 (C5_{D}), 69.1 (C6_{D}), 69.0 (C3_{D}), 68.9 (OCH₂), 68.3 (C6_{E}), 56.4 (C2_{D}), 48.5 (NCH₂Ph), 22.6 (COCH₃). HRMS (ESI⁺) for C₅₉H₆₆NO₁₁ ([M+H]+, 964.4636) *m*/*z* 964.4813.

**Allyl 2-*O*-benzoyl-4-*O*-benzyl-3-*O*-chloroacetyl-**α**-L-rhamnopyranosyl-(1→3)-[2,3,4,6-tetra-*O*-benzyl-**α**-D-glucopyranosyl-(1→4)]-6-*O-*benzyl-2-benzylacetamido-2-deoxy-**α**-D-glucopyranoside (XX₁₁)**. TMSOTf (5.5 µL, 0.030 mmol) was added to a solution of known 2-*O*-benzoyl-4-*O*-benzyl-3-*O-*chloroacetyl-α-L-rhamnopyranosyl trichloroacetimidate (ref. 73) (73 mg, 0.126 mmol) and acceptor **XX₁₀** (98 mg, 0.102 mmol) in toluene (2.75 mL) containing activated MS4Å under argon at -10 °C. The mixture was stirred at room temperature and, when TLC monitoring indicated complete consumption of the donor, the reaction was quenched by addition of triethylamine. After filtration through a bed of celite and concentration under vacuum, flash chromatography (85:15 Toluene-EtOAc) gave a mixture of two products (Rf = 0.4, 4:1 Toluene-EtOAc), among which the target trisaccharide **XX₁₁** as indicated by mass spectrometry analysis. Compound **XX₁₁** had HRMS (ESI⁺) for C₈₁H₈₆ClNO₁₇Na ([M+Na]⁺, 1402.5482) *m*/*z* 1402.5525.

**Allyl 2-*O*-acetyl-3,4-di-*O*-benzyl-**α**-L-rhamnopyranosyl-(1→3)-[2,3,4,6-tetra-*O*-benzyl-**α**-D-glucopyranosyl-(1→4)]-6-*O*-benzyl-2-benzylacetamido-2-deoxy-**α**-D-glucopyranoside (XX₁₂).** TMSOTf (4 µL, 0.026 mmol) was added to a solution of known 2-*O*-acetyl-3,4-di-*O*-benzyl-α-L-rhamnopyranosyl trichloroacetimidate (ref. 74) (132 mg, 0.249 mmol) and acceptor **XX₁₀** (76 mg, 0.079 mmol) in toluene (4 mL) containing activated MS4Å, stirred under argon at 0 °C. Following additional stirring for 3 h at 60 °C, TLC monitoring indicated complete consumption of the donor. The reaction was quenched by addition of triethylamine. After filtration through a bed of celite and concentration under vacuum, flash chromatography (9:1 Toluene-EtOAc) gave the expected trisaccharide **XX₁₂** (Rf = 0.4, 4:1 Toluene-EtOAc). ¹H NMR (CDCl₃, 400 MHz) δ (ppm): 7.28-7.12 (m, 41H, NH, H arom.), 5.61 (d, 1H, *J*₁₋₂ = 3.6 Hz, H1_{E}), 5.22 (m, 1H, H2_{D}), 5.14 (pt, 1H, *J*₁₋₂ = *J*₂₋₃ = 2.5 Hz, H2_{C}), 5.08-4.97 (m, 1H, CH=), 4.83 (d overlapped, 1H, H1_{C}), 4.83-4.57 (m, 11H, H1_{d}, 2 =CH₂, 6 OCH₂Ph, 2 NCH₂Ph), 4.50-4.32 (m, 7H, 7 OCH₂Ph), 4.30-4.20 (m, 2H, H3_{D}, OCH₂Ph), 4.09 (dd, 1H, *J*₃₋₄ = 7.2 Hz, *J*₄₋₅ = 9.0 Hz, H4_{D}), 4.01-3.90 (m, 2H, H5_{C}, OCH₂), 3.85 (dd overlapped, 1H, H3_{C}), 3.83-3.60 (m, 6H, H5_{D}, H3_{E}, H4_{E}, H5_{E}, H6a_{D}, OCH₂), 3.56-3.42 (m, 2H, H6b_{D}, H6a_{E}), 3.40 (dd, 1H, *J*₂₋₃ = 9.6 Hz, H2_{E}), 3.33 (pt, 1H, *J*₃₋₄ = *J*₄₋₅ = 9.3 Hz, H4_{C}), 3.09 (m, 1H, *J*₅₋₆ = 5.7 Hz, *J*_{6a-6b} = 11.8 Hz, H6b_{E}), 2.04, 1.98 (2s, 6H, COCH₃), 1.20 (d, 3H, *J*₅₋₆ = 6.1 Hz, H6_{C}); ¹³C NMR (CDCl₃, 100 MHz) δ (ppm): 173.8, 170.4 (C=O), 139.0, 138.9, 138.7, 138.6, 138.4, 138.3 (C quat. arom.), 133.1 (CH=), 128.5, 128.4, 128.3, 128.2, 128.1, 127.9, 127.8, 127.7, 127.6, 127.4, 127.3, 126.6, 125.3 (C arom.), 117.5 (=CH₂), 98.1 (C1_{C}), 97.5 (C1_{D}), 94.2 (C1_{E}), 81.8 (C3_{E}), 80.2 (C2_{E}), 79.8 (C4_{C}), 79.7 (C3_{D}), 77.7 (C4_{E}), 77.6 (C3_{C}), 75.4, 75.0, 74.8, 73.4 (4C, OCH₂Ph), 73.2 (C4_{D}), 73.2, 73.1, 71.6 (3C, OCH₂Ph), 71.3 (C5_{D}*), 70.2 (C2_{C}), 70.1 (C5_{E}*), 69.4 (C6_{D}), 68.8 (OCH₂), 68.7 (C5_{C}), 68.6 (C6_{E}), 54.9 (C2_{D}), 48.3 (NCH₂Ph), 22.7, 21.1 (2C, COCH₃), 18.1 (C6_{C}). HRMS (ESI⁺) for C₈₁H₈₉NO₁₆Na ([M+Na]⁺, 1354.6079) *m*/*z* 1354.5994.

### EXAMPLE 5: Chemo-enzymatic synthesis of potential donor building blocks to oligosaccharide fragments of S. flexneri 1b and/or 1a O-antigens

**Ally** α**-D-glucopyranosyl-(1-4)-2-deoxy-2-trichloroacetamido-**α-**D-glucopyranoside (XX₁₃).** Methanolic sodium methoxide (25% wt solution in MeOH, 1.72 mL, 6.10 mmol) and trichloracetic anhydride (560 µL, 3.07 mmol) were added to a solution of compound **XX₅** (390 mg, 1.02 mmol) in methanol (8 mL) at 0 °C. After 1 h the reaction mixture was neutralized by addition of Dowex-H⁺ resin, filtered, and concentrated to dryness to obtain crude **XX₁₃.** This material was used directly without further purification. Compound **XX₁₃** had Rf = 0.7 (85:15 CH₃CN-H₂O). HRMS (ESI⁺) of C₁₇H₂₆Cl₃NO₁₁Na ([M+Na]⁺, 548.0469) *m*/*z* 548.0524.

**Allyl 2,3,4,6-tetra-*O*-acetyl-**α**-D-glucopyranosyl-(1→4)-3,6-di-*O-*acetyl-2-deoxy-2-trichloroacetamido-**α**-D-glucopyranoside (XX₁₄).** Acetic anhydride (5 mL, 52.9 mmol) was added dropwise to a solution of crude **XX₁₃** (1.02 mmol) in anhydrous pyridine (5 mL) and the resulting mixture was stirred overnight at room temperature. The reaction was quenched at 0 °C by addition of methanol, and the mixture was evaporated to dryness. The residue was purified by flash-chromatography (3:1 Cyclohexane-Acetone) to yield to **XX₁₄** (458 mg, 58% over 2 steps) as an oil. Compound **XX₁₄** had Rf = 0.5 (3:1 Cyclohexane-Acetone). ¹H NMR (CDCl₃, 400 MHz) δ (ppm): 6.93 (d, 1H, *J* = 9.1 Hz, NH), 5.92 (m, 1H, CH=), 5.51 (d, 1H, *J*₁₋₂ = 4.0 Hz, H1_{E}), 5.47-5.27 (m, 4H, =CH₂, H3_{D}, H3_{E}), 5.09 (pt, 1H, *J*₄₋₅ = 10.0 Hz, H4_{E}), 4.95 (d, 1H, *J*₁₋₂ = 3.6 Hz, H1_{D}), 4.89 (dd, 1H, *J*₂₋₃ = 10.5 Hz, H2_{E}), 4.48 (dd, 1H, *J*₅₋₆ₐ = 1.3 Hz, *J*_{6a-6b} = 12.0 Hz, H6a_{D}), 4.31-4.23 (m, 3H, OCH₂, H6b_{D}, H6a_{E}), 4.14 (ddd overlapped, 1H, *J*₂₋₃ = 10.8 Hz, H2_{D}), 4.11-4.05 (m, 4H, OCH₂, H4_{D}, H5_{D}, H6b_{E}), 3.99 (pdt, 1H, *J*₅₋₋₆ₐ = 3.1 Hz, *J*_{5-6b} = 10.0 Hz, H5_{E}), 2.18, 2.13, 2.06, 2.05, 2.02 (6s, 18H, COCH₃); ¹³C NMR (CDCl₃, 100 MHz) δ (ppm): 171.4, 170.5, 169.9, 169.4 (6C, C=O), 161.9 (C=O), 132.7 (CH=), 119.2 (=CH₂), 95.6 (C1_{E}), 95.2 (C1_{D}), 92.0 (CCl₃), 73.1 (C3_{D}), 72.3 (C4_{D}), 70.1 (C2_{E}), 69.4 (C3_{E}), 69.1 (OCH₂), 68.6 (C5_{E}), 68.3 (C5_{D}), 68.0 (C4_{E}), 62.7 (C6_{D}), 61.4 (C6_{E}), 54.4 (C2_{D}), 21.0, 20.8, 20.7, 20.6, 20.5 (6C, COCH₃). HRMS (ESI⁺) of C₂₉H₃₈Cl₃NO₁₇Na ([M+Na]⁺, 800.1103) m/z 800.1112.

**2,3,4,6-tetra-*O*-acetyl-**α**-*D*-glucopyranosyl-(1→4)-3,6-di-*O*-acetyl-2-deoxy-2-trichloroacetamido-**α**-D-glucopyranose (XX₁₅).** A catalytic amount of 1,5-cyclooctadiene-bis[methyldiphenylphosphine]-iridium hexafluorophosphate (50 mg) was dissolved in dry THF (20 mL). The stirred solution was degassed, placed under hydrogen for 10 minutes until the orange colour turned yellow, degassed and placed under nitrogen. A solution of **XX₁₄** (444 mg, 0.57 mmol) in THF/H₂O mixture (5:2, v/v, 7mL), was then poured into the solution of iridium complex. The reaction mixture was stirred for 3 h and I₂ (290 mg, 1.14 mmol) was added. After stirring for another 3 h, the mixture was diluted with dichloromethane, washed with aqueous NaHSO₃ and water, dried and concentrated. The crude material was used as such. Compound **XX₁₅** had Rf = 0.2 (3:2 Cyclohexane-EtOAc). ¹H NMR (CDCl₃, 400 MHz) δ (ppm): 7.03 (d, 1H, *J* = 9.2 Hz, NH), 5.53 (d overlapped, 1H, H1_{E}), 5.50 (dd overlapped, 1H, *J*₂₋₃ = 10.8 Hz, *J*₃₋₋₄ = 9.0 Hz, H3_{D}), 5.39 (pt, 1H, *J*₂-₃ =*J*₃-₄ = 10.0 Hz, H3_{E}), 5.31 (sl, 1H, H1_{D}), 5.10 (pt, 1H, *J*₄-₅ = 10.0 Hz, H4_{E}), 4.89 (dd, 1H, *J*₁₋₂ = 4.0 Hz, H2_{E}), 4.52 (dd, 1H, J₅₋₆ₐ = 3.7 Hz, *J*_{6a-6b} = 13.4 Hz, H6a_{D}), 4.32-4.23 (m, 3H, H5_{D}, H6b_{D}, H6a_{E}), 4.15-3.94 (m, 4H, H2_{D}, H4_{D}, H5_{E}, H6b_{E}), 2.18, 2.13, 2.06, 2.05, 2.02 (6s, 18H, COCH₃). ^{l3}C NMR (CDCl₃, 100 MHz) δ (ppm): 171.4, 170.6, 170.0, 169.4 (6C, C=O), 162.0 (C=O), 95.5 (C1_{E}), 92.0 (CCl₃), 90.6 (C1_{D}), 72.9 (C3_{D}), 72.3 (C4_{D}), 69.9 (C2_{E}), 69.4 (C3_{E}), 68.5 (C5_{E}), 68.0 (2C, C5_{D}, C4_{E}), 62.8 (C6_{D}), 61.4 (C6_{E}), 54.7 (C2_{D}), 21.0, 20.8, 20.7, 20.6, 20.5 (6C, COCH₃). HRMS (ESI⁺) of C₂₆H₃₄Cl₃NO₁₇Na ([M+Na]⁺, 760.0709) *m*/*z* 760.0804.

**2,3,4,6-Tetra-*O*-acetyl-α-D-glucopyranosyl-(1→4)-3,6-di-O-acetyl-2-deoxy-2-trichloroacetamido-α-D-glucopyranosyl trichloroacetimidate (XX₁₆).** DBU (26 µL, 0.17 mmol) was added to a stirred solution of crude **XX₁₅** (0.57 mmol) and CCl₃CN (70 µL, 0.68 mmol) in dichloroethane (6 mL) at -5 °C. After 3 h, the solution was concentrated. Column chromatography (2:1 Cyclohexane-EtOAc) of the residue yielded **XX₁₆** (131 mg, 26%) as a syrup. Compound **XX₁₆** had Rf = 0.4 (3:2 Cyclohexane-EtOAc). ¹H NMR (CDCl₃, 400 MHz) δ (ppm): 6.98 (d, 1H, *J* = 8.7 Hz, NH), 6.46 (d, 1H, *J*₁₋₂ = 3.6 Hz, H1_{D}), 5.56 (d, 1H, *J*₁₋₂ = 4.1 Hz, H1_{E}), 5.51 (dd overlapped, 1H, H3_{D}), 5.41 (dd overlapped, 1H, H3_{E}), 5.10 (pt, 1H, *J*₄₋₅ = 10.0 Hz, H4_{E}), 4.89 (dd, 1H, *J*₂₋₃ = 10.4 Hz, H2_{E}), 4.50 (dd, 1H, *J*₅₋₆ₐ = 1.8 Hz, *J*_{6a-6b} = 12.4 Hz, H6a_{D}), 4.38-4.24 (m, 4H, H2_{D}, H5_{D}, H6b_{D}, H6a_{E}), 4.20-3.94 (m, 3H, H4_{D}, H5_{E}, H6b_{E}), 2.18, 2.13, 2.06, 2.10, 2.05, 2.02 (6s, 18H, COCH₃).

**Allyl 2,3,4,6-tetra-*O*-acetyl-**α**-D-glucopyranosyl-(1→4)-3,6-di-*O-*acetyl-2-deoxy-2-trichloroacetamido-**α**-D-glucopyranosyl-(1→2)-3,4-di-*O*-benzyl-**α-**L-rhamnopyranoside (XX₁₇).** TMSOTf (8 µL, 0.04 mmol) was added to a solution of the trichloroacetimidate donor **XX₁₆** (131 mg, 0.15 mmol) and known allyl 3,4-di-*O-*benzyl-α-L-rhamnopyranoside (ref. 76) (86 mg, 0.22 mmol) in toluene (4 mL) containing activated MS4Å under argon. The mixture was stirred at -60 °C for 60 min and, when TLC monitoring indicated complete consumption of the donor, the reaction was quenched by addition of triethylamine. After filtration through a bed of celite and concentration under vacuum, flash chromatography (2:1 Cyclohexane-EtOAc) gave slightly contaminated trisaccharide **XX₁₇** (Rf = 0.2 in 2:1 Cyclohexane-EtOAc). ¹H NMR (CDCl₃, 400 MHz) δ (ppm): 7.41-7.28 (m, 10H, H arom.), 6.71 (d, 1H, *J* = 8.7 Hz, NH), 5.91 (m, 1H, CH=), 5.45 (d, 1H, *J*₁₋₂ = 4.0 Hz, H1_{E}), 5.43-5.37 (m, 2H, H4_{D}, H3_{E}), 5.27 (m, 1H, =CH₂), 5.19 (m, 1H, =CH₂), 5.11-5.07 (m, 2H, H3_{D}, H4_{E}), 4.89 (dd, 1H, *J*₂₋₃ = 10.1 Hz, H2_{E}), 4.86 (d, 1H, OCH₂Ph), 4.80 (d, 1H, *J*₁₋₂ = 1.4 Hz, H1_{A}), 4.78 (d, 1H, OCH₂Ph), 4.62 (d, 1H, OCH₂Ph), 4.59 (d, 1H, OCH₂Ph), 4.56 (d, 1H, *J*₁₋₂ = 8.5 Hz, H1_{D}), 4.50 (m, 1H, H6a_{D}), 4.31-4.19 (m, 2H, H6b_{D}, H6a_{E}), 4.17-3.95 (m, 6H, 2 OCH₂, H2_{D}, H5_{D}, H5_{E}, H6b_{E}), 3.93 (dd, 1H, *J*₂₋₃ = 5.1 Hz, H2_{A}), 3.89 (dd, 1H, iJ₃₋₄ = 9.2 Hz, H3_{A}), 3.76-3.68 (m, 1H, H5_{A}), 3.38 (pt, 1H, *J*₄₋₅ = 9.2 Hz, H4_{A}), 2.17, 2.12, 2.10, 2.06, 2.05, 2.04 (6s, 18H, 6 COCH₃), 1.30 (d, 1H, *J*₅₋₆ = 5.1 Hz, H6_{A}); ¹³C NMR (CDCl₃, 100 MHz) δ (ppm): 171.4, 170.5, 170.3, 170.3, 169.4, 161.8 (7C, C=O), 138.5, 138.3 (2C, C quat. arom.), 133.8 (CH=), 128.9, 128.5, 128.4, 128.2, 127.7, 127.6 (10C, C arom.), 117.2 (=CH₂), 101.4 (C1_{D}), 98.2 (C1_{A}), 95.5 (C1_{E}), 92.1 (CCl₃), 81.0 (C4_{A}), 80.0 (C3_{A}), 76.8 (C2_{A}), 75.6 (OCH₂Ph), 75.0 (C3_{D}), 73.6 (OCH₂Ph), 73.2 (C4_{D}), 70.0 (C2_{E}), 69.4 (C3_{E}), 68.5 (C5_{E}), 68.0 (3C, C5_{A}, C5_{D}, C4_{E}), 67.8 (OCH₂), 62.5 (C6_{D}), 61.5 (C6_{E}), 55.9 (C2_{D}), 22.5, 21.1, 21.0, 20.8, 20.7 (6C, COCH₃), 17.9 (C6_{A}). HRMS (ESI⁺) for C₄₉H₆₀Cl3NO₂₁Na ([M+Na]⁺, 1126.2621) *m*/*z* 1126.2623.

### EXAMPLE 6: Chemical synthesis of potential acceptor building blocks to oligosaccharide fragments of S. flexneri 1b and/or 1a O-antigens

**Allyl 2-acetamido-3-*O*-acetyl-4,6-*O*-benzylidene-2-deoxy-**α**-D-glucopyranoside (ref. 69) (XX₁₉).** Benzaldehyde dimethylacetal (2.6 mL, 17.55 mmol) and CSA (2.72 g, 11.70 mmol) were added to a solution of compound **XX₁** (1.53 g, 5.85 mmol) in acetonitrile (50 mL). After 45 min, the methanol formed during the reaction was removed under reduced pressure and another 2 mL of benzaldehyde dimethylacetal were added to the mixture. After 60 min, the reaction mixture was cooled to 0 °C, neutralized by addition of triethylamine and concentrated to dryness to afford crude **XX₁₈** (ref. 75) (2.03 g) as a white solid (Rf = 0.6, 1:1 Cyclohexane-Acetone). This material was used directly without further purification.

Ac₂O (20 mL, 211.6 mmol) was added dropwise to a solution of crude **XX₁₈** (2.03 g) in anhydrous pyridine (50 mL) and the resulting mixture was stirred for one night at room temperature. The reaction was then quenched at 0 °C by addition of methanol, and the mixture was evaporated to dryness. The residue was purified by flash-chromatography (Rf = 0.2, 7:3 Cyclohexane-Acetone) to obtain **XX₁₉** (2.27 g, 99% over 2 steps) as a white solid. ¹H NMR (CDCl₃, 400 MHz) δ (ppm): 7.49-7.45 (m, 2H, H arom.), 7.41-7.34 (m, 3H, H arom.), 5.91 (m, 1H, CH=), 5.85 (d, 1H, *J* = 9.6 Hz, NH), 5.55 (s, 1H, H7), 5.35 (dd overlapped, 1H, H3), 5.32 (m overlapped, 1H, =CH₂), 5.27 (m, 1H, =CH₂), 4.90 (d, 1H, *J*₁₋₂ = 3.7 Hz, H1), 4.37 (ddd, 1H, *J*₂-₃ = 10.6 Hz, H2), 4.30 (dd, 1H, *J*₅₋₆ₐ = 4.8 Hz, *J*_{6a-6b} = 10.3 Hz, H6a), 4.22 (m, 1H, OCH₂), 4.02 (m, 1H, OCH₂), 3.95 (ddd, 1H, *J*₄-₅ = 9.5 Hz, *J*₅-_{6b} = 9.8 Hz, H5), 3.80 (pt, 1H, H6b), 3.74 (pt, 1H, *J*₃-₄ = 9.5 Hz, H4), 2.08, 1.97 (2s, 6H, COCH₃); ¹³C NMR (CDCl₃, 100 MHz) δ (ppm): 171.4, 170.0 (2C, C=O), 137.0, 133.2, 129.1, 128.2, 128.1, 126.2 (7C, CH=, C arom.), 118.3 (=CH₂), 101.6 (C7), 97.2 (C1), 79.1 (C4), 70.3 (C3), 68.9, 68.7 (2C, C6, OCH₂), 63.0 (C5), 52.6 (C2), 23.2, 20.9 (2C, COCH₃). HRMS (ESI⁺) for C₂₀H₂₅NO₇Na ([M+Na]⁺, 414.1529) *m*/*z* 414.1525.

**Allyl 2-acetamido-3-*O*-acetyl-6-*O*-benzyl-2-deoxy-**α**-D-glucopyranoside (ref. 69) (XX₂₀)**. Trifluoroacetic acid (1.1 mL, 14.81 mmol) was slowly added to an ice-cold mixture of **XX₁₉** (1.30 g, 3.29 mmol) and triethylsilane (2.6 mL, 16.45 mmol) in CH₂Cl₂ (35 mL). After stirring the mixture for 2 h at 0 °C, then for 4.5 h at room temperature, the reaction was quenched by addition of Et₃N and concentrated. Purification of the residue by flash column chromatography on silica gel (7:3 → 3:2, Cyclohexane-Acetone) gave α-glycoside **XX₂₀** (0.84 g, 65%) and the de-O-acetylated analogue **XX_{20A}** (ref. 77) (284 mg, 21%).

Compound **XX₂₀** had Rf = 0.2 (7:3 Cyclohexane-Acetone): ¹H NMR (CDCl₃, 400 MHz) δ (ppm): 7.38-7.28 (m, 5H, H arom.), 5.89 (m, 1H, CH=), 5.84 (d, 1H, *J* = 9.6 Hz, NH), 5.29 (m, 1H, =CH₂), 5.22 (m, 1H, =CH₂), 5.12 (dd, 1H, *J*₂-₃ = 10.8 Hz, *J*₃-₄ = 8.6 Hz, H3), 4.86 (d, 1H, *J*₁₋₂ = 3.6 Hz, H1), 4.63 (d, 1H, OCH₂Ph), 4.57 (d, 1H, OCH₂Ph), 4.26 (ddd, 1H, H2), 4.19 (m, 1H, OCH₂), 4.00 (m, 1H, OCH₂), 3.85-3.76 (m, 3H, H4, H5, H6a), 3.73 (dd, 1H, *J*_{5-6b} = 3.9 Hz, *J*_{6a-6b} = 10.2 Hz, H6b), 2.09, 1.95 (2s, 6H, COCH₃); ¹³C NMR (CDCl₃, 100 MHz) δ (ppm): 172.2, 170.1 (2C, C=O), 137.7 (C quat. arom.), 133.4 (CH=), 128.5, 127.8, 127.6 (5C, C arom.), 118.0 (=CH₂), 96.5 (C1), 74.1 (C3), 73.7 (OCH₂Ph), 70.3 (C5*), 70.0 (C4*), 69.9 (C6), 68.4 (OCH₂), 51.8 (C2), 23.2, 21.0 (2C, COCH₃). HRMS (ESP) for C₂₀H₂₇NO₇Na ([M+Na]⁺, 416.1685) *m*/*z* 416.1670.

Compound **XX_{20A}** had Rf = 0.2 (1 : 1 Cyclohexane-Acetone). ¹H NMR (CDCl₃, 400 MHz) δ (ppm): 6.03 (d, 1H, *J* = 9.0 Hz, NH), 5.92 (m, 1H, CH=), 5.30 (m, 1H, =CH₂), 5.24 (m, 1H, =CH₂), 4.84 (d, 1H, *J*₁₋₂ = 3.8 Hz, H1), 4.60 (d, 1H, OCH₂Ph), 4.57 (d, 1H, OCH₂Ph), 4.20 (m overlapped, 1H, OCH₂), 4.14 (ddd overlapped, 1H, H2), 4.00 (m, 1H, OCH₂), 3.82-3.71 (m, 4H, H3, H5, H6a, H6b), 3.60 (pt, 1H, *J*₃₋₄ = *J*₄₋₅ = 8.8 Hz, H4), 1.97 (s, 3H, COCH₃); ¹³C NMR (CDCl₃, 100 MHz) δ (ppm): 172.5 (C=O), 138.1 (C quat. arom.), 133.4 (CH=), 128.4, 127.8, 127.7 (5C, C arom.), 118.1 (=CH₂), 96.5 (C1), 74.0 (C3), 73.6 (OCH₂Ph), 71.7 (C4), 70.6 (C5), 69.6 (C6), 68.3 (OCH₂), 53.3 (C2), 23.3 (COCH₃).

**Phenyl 2,3,4,6-tetra-O-acetyl-1-thio-β-D-glucopyranoside (ref. 78) (XX₂₁).** Ethanethiol (7.9 ml, 76.9 mmol) and boron trifluoride etherate (6.3 ml, 51.2 mmol) were successively added at 0 °C to a solution of β-D-glucose pentaacetate (10 g, 25.6 mmol) in CH₂Cl₂ (100 mL). After 4 h stirring at this temperature, the organic solution was washed with saturated aqueous NaHCO₃ and water, then dried (Na₂SO₄) and concentrated to give **XX₂₁** as a white foam (10.28 g, 91%). Compound **XX₂₁** had Rf = 0.5 (4:1 Cyclohexane-EtOAc). ¹H NMR (CDCl₃, 400 MHz) δ (ppm): 7.53-7.51 (m, 2H, H arom.), 7.36-7.32 (m, 3H, H arom.), 5.25 (pt, 1H, *J*₂-₃ = *J*₃-₄ = 9.4 Hz, H3), 5.06 (pt, 1H, *J*₄-₅ = 9.4 Hz, H4), 5.00 (dd, 1H, *J*₁₋₂ = 10.1 Hz, H2), 4.73 (d, 1H, H1), 4.25 (dd, 1H, *J*₅-₆ₐ = 5.0 Hz, *J*₆ₐ-_{6b} = 12.3 Hz, H6a), 4.20 (dd, 1H, *J*₅-_{6b} = 2.7 Hz, H6b), 3.75 (ddd, 1H, H5), 2.11, 2.04, 2.01 (4s, 12H, COCH₃); ¹³C NMR (CDCl₃, 100 MHz) δ (ppm): 170.9, 170.5, 169.7, 169.6 (4C, C=O), 133.5, 132.1, 129.3, 128.8 (6C, C arom.), 86.1 (C1), 76.2 (C5), 74.4 (C3), 70.4 (C2), 68.7 (C4), 62.6 (C6), 21.1, 21.0, 20.9 (4C, COCH₃).

**Phenyl 2,3,4,6-tetra-*O*-benzyl-1-thio-**β**-D-glucopyranoside (ref. 79) (XX₂₃).** Methanolic sodium methoxide (0.5 M solution, 20 mL, 10.0 mmol) was added to a solution of peracetylated **XX₂₁** (10.27 g, 23.3 mmol) in methanol (50 mL). After 7 h at room temperature, the reaction was quenched with Dowex-H⁺ resin, filtered, and concentrated. The crude tetraol (ref. 80) **XX₂₂** was dissolved in *N*,*N*-dimethylformamide (100 mL) and a 60% suspension of NaH in oil (5.71 g, 143 mmol), and benzyl bromide (28.3 mL, 238 mmol), were added at 0°C. The mixture was allowed to reach room temperature and was stirred overnight. Excess NaH was neutralized with MeOH. After concentration to dryness, the perbenzylated product **XX₂₃** (10.94 g, 72% over 2 steps) was purified by flash chromatography (9:1 Cyclohexane-EtOAc). Compound **XX₂₃** had Rf = 0.5 (9:1 Cyclohexane-EtOAc). ¹H NMR (CDCl₃, 400 MHz) δ (ppm): 7.64-7.59 (m, 2H, H arom.), 7.43-7.21 (m, 23H, H arom.), 4.94-4.84 (m, 4H, OCH₂Ph), 4.76 (d, 1H, OCH₂Ph), 4.70 (d overlapped, 1H, H1), 4.65-4.56 (m, 3H, OCH₂Ph), 3.82 (dd, 1H, *J*₅-₆ₐ = 2.0 Hz, *J*₆ₐ-_{6b} = 10.9 Hz, H6a), 3.76 (dd overlapped, 1H, H6b), 3.74 (pt overlapped, 1H, H3), 3.68 (pt, 1H, *J*₃-₄ = *J*₄-₅ = 9.4 Hz, H4), 3.54 (dd, 1H, *J*₁-₂ = 9.8 Hz, *J*₂₋₃ = 8.6 Hz, H2), 3.53 (ddd, 1H, H5); ¹³C NMR (CDCl₃, 100 MHz) δ (ppm): 138.8, 138.5, 134.3 (4C, C quat. arom.), 132.3, 129.3, 128.8, 128.7, 128.6, 128.3, 128.2, 128.1, 128.0, 127.9, 127.8 (20C, C arom.), 87.9 (C1), 87.2 (C3), 81.3 (C2), 79.5 (C5), 78.3 (C4), 76.2, 75.8, 75.4, 73.8 (4C, OCH₂Ph), 69.5 (C6). HRMS (ESI⁺) for C₄₀H₄₀O₅SNa ([M+Na]⁺, 655.2494) *m*/*z* 655.2475.

**Allyl 2,3,4,6-tetra-*O*-benzyl-**α**-D-glucopyranosyl-(1**→**4)-2-acetamido-3-*O*-acetyl-6-*O*-benzyl-2-deoxy-**α**-D-glucopyranoside (XX₂₄).** NIS (353 mg, 1.57 mmol) and TMSOTf (65 µL, 0.36 mmol) were successively added to a solution of thioglycoside donor **XX₂₃** (917 mg, 1.45 mmol) and glycosyl acceptor **XX₂₀** (475 mg, 1.21 mmol) in 5:2 Et₂O-CH₂Cl₂ (14 mL) containing activated MS4Å under argon. The mixture was stirred at 0 °C for 60 min. When TLC monitoring indicated reaction completion (Rf = 0.3, 1:1 Cyclohexane-EtOAc), triethylamine was added. After filtration through a bed of celite and concentration under vacuum, the residue was purified by flash chromatography (7:3 Cyclohexane-Acetone) to give slightly contaminated **XX₂₄** (780 mg). ¹H NMR (CDCl₃, 400 MHz) δ (ppm): 7.38-7.13 (m, 25H, H arom.), 5.93 (m, 1H, CH=), 5.86 (d, 1H, *J* = 9.7 Hz, NH), 5.44 (dd, 1H, *J*₂₋₃ = 10.7 Hz, *J*₃₋₄ = 9.1 Hz, H3_{D}), 5.32 (m, 1H, =CH₂), 5.25 (m, 1H, =CH₂), 5.17 (d, 1H, *J*₁₋₂ = 3.3 Hz, H1_{E}), 4.91 (d, 1H, OCH₂Ph), 4.90 (d, 1H, *J*₁₋₂ = 4.0 Hz, H1_{D}), 4.83 (d, 1H, OCH₂Ph), 4.80 (d, 1H, OCH₂Ph), 4.67 (s, 2H, OCH₂Ph), 4.62-4.53 (m, 3H, OCH₂Ph), 4.45 (d, 1H, OCH₂Ph), 4.37 (ddd, 1H, H2_{D}), 4.35 (d, 1H, OCH₂Ph), 4.20 (m, 1H, OCH₂), 4.17 (pt, 1H, *J*₄₋₅ = 9.4 Hz, H4_{D}), 4.05-3.97 (m, 2H, OCH₂, H6a_{D}), 3.90 (pt, 1H, *J*₃₋₄ = 9.3 Hz, H3_{E}), 3.89 (m overlapped, 1H, H5_{D}), 3.89 (ddd, 1H, *J*₄-₅ = 9.9 Hz, H5_{E}), 3.70 (dd, 1H, *J*₆ₐ-_{6b} = 10.8 Hz, H6b_{D}), 3.65 (dd, 1H, *J*₃₋₄ = 9.2 Hz, *J*₄₋₅ = 9.9 Hz, H4_{E}), 3.54 (dd, 1H, *J*₅-₆ₐ = 3.3 Hz, H6a_{E}), 3.52 (dd, 1H, *J*₂₋₃ = 9.7 Hz, H2_{E}), 3.44 (dd, 1H, *J*₅-_{6b} = 1.8 Hz, *J*_{6a-6b} = 10.6 Hz, H6b_{E}), 1.99, 1.96 (2s, 6H, 2 COCH₃); ¹³C NMR (CDCl₃, 100 MHz) δ (ppm): 171.7, 169.8 (2C, C=O), 138.4, 138.2, 137.9, 137.5 (5C, C quat. arom.), 133.1 (CH=), 128.2, 128.1, 128.0, 127.9, 127.8, 127.6, 127.5, 127.4, 127.3, 127.2, 127.1, 127.0 (25C, C arom.), 117.6 (=CH₂), 97.0 (C1_{E}, J_{C-H} = 170 Hz), 96.1 (C1_{D}), 81.3 (C3_{E}), 79.5 (C2_{E}), 77.2 (C4_{E}), 75.3, 74.6 (2C, OCH₂Ph), 73.1 (C3_{D}), 73.0, 72.9, 72.8 (3C, OCH₂Ph), 72.7 (C4_{D}), 70.8 (C5_{E}), 70.2 (C5_{D}), 68.1 (2C, OCH₂, C6_{D}), 67.9 (C6_{E}), 51.9 (C2_{D}), 22.9, 20.9 (2C, COCH₃).

**Allyl 2,3,4,6-tetra-*O*-benzyl-**α**-D-glucopyranosyl-(1**→**4)-2-acetamido-6-*O*-benzyl-2-deoxy-**α**-D-glucopyranoside (XX₂₅).** Methanolic sodium methoxide (0.5 M solution, 0.4 mL, 0.2 mmol) was added to a stirred solution of **XX₂₄** (742 mg, 0.92 mmol) in anhydrous methanol (7.5 mL). The reaction mixture was stirred for 1 day at room temperature by which time all the starting material had been consumed (Rf = 0.4, 1:1 Cyclohexane-EtOAc). Excess base was neutralized with Dowex-H⁺ resin. After removal of the resin by filtration, the filtrate was concentrated, and the residue was purified by silica gel chromatography, eluting with 1:1 Cyclohexane-EtOAc to obtain compound **XX₂₅** as a colourless oil (555 mg, 69%); ¹H NMR (CDCl₃, 400 MHz) δ (ppm): 7.39-7.18 (m, 25H, H arom.), 5.93 (m, 1H, CH=), 5.87 (d, 1H, *J* = 8.4 Hz, NH), 5.32 (m, 1H, =CH₂), 5.24 (m, 1H, =CH₂), 5.03 (d, 1H, *J*₁₋₂ = 3.5 Hz, H1_{E}), 5.00 (d, 1H, *J*₁₋₂ = 3.7 Hz, H1_{D}), 4.96-4.83 (m, 5H, OCH₂Ph), 4.77 (d, 1H, OCH₂Ph), 4.62-4.43 (m, 4H, OCH₂Ph), 4.25-4.18 (m, 2H, 1H OCH₂, H2_{D}), 4.05-3.99 (m, 2H, 1H OCH₂, H3_{E}), 3.96 (dd, 1H, *J*₂₋₃ = 10.7 Hz, *J*₃₋₄ = 8.6 Hz, H3_{D}), 3.88 (ddd, 1H, *J*₄-₅ = 10.0 Hz, *J*₅-₆ₐ = 3.5 Hz, H5_{E}), 3.81-3.60 (m, 6H, H4_{D}, H5_{D}, H6a_{D}, H6b_{D}, H4_{E}, H6a_{E}), 3.59 (dd, 1H, *J*₂₋₃ = 10.0 Hz, H2_{E}), 3.50 (dd, 1H, *J*₅-_{6b} = 1.7 Hz, *J*_{6a-6b} = 10.6 Hz, H6b_{E}), 2.06 (s, 3H, COCH₃); ¹³C NMR (CDCl₃, 100 MHz) δ (ppm): 170.5 (C=O), 138.6, 138.5, 138.2, 137.9, 137.0 (7C, C quat. arom.), 133.8 (CH=), 128.7, 128.6, 128.5, 128.4, 128.3, 127.9, 127.8 (25C, C arom.), 117.6 (=CH₂), 100.6 (C1_{E}, J_{C-H} = 172 Hz), 96.5 (C1_{D}), 82.2 (C3_{E}), 82.0 (C4_{D}), 79.6 (C2_{E}), 77.8 (C4_{E}), 75.7, 75.0, 74.1, 73.5, 73.2 (5C, OCH₂Ph), 72.0 (C3_{D}), 71.5 (C5_{E}), 70.2 (C5_{D}), 69.0 (C6_{D}), 68.5 (C6_{E}), 68.4 (OCH₂), 53.3 (C2_{D}), 23.4 (COCH₃). HRMS (ESI⁺) for C₅₂H₅₉NO₁₁Na ([M+Na]⁺, 896.3986) *m*/*z* 896.4020.

### EXAMPLE 7: Synthesis and characterization of α-D-glucopyranosyl-(1→4)-N-acetyl-D-glucosamine (P2)

Glucosylation of N-acetyl-D-glucosamine (acceptor) α-D-glucopyranosyl-(1→4)-N-acetyl-D-glucosamine (P2) is the main acceptor reaction product obtained by action of 1G5A amylosucrase (AS) (Recombinant form of *Neisseria polysaccharea* amylosucrase EC2.4.1.4 (GH13)) using sucrose as donor and D-GlcpNAc as acceptor in a molar ratio of 1.

### 7.1. Materials

Recombinant enzyme were produced in *E.coli* as reported elsewhere. Purified AS, conserved at -20°C or -80°C, served for enzymatic reaction.

*N*-acetyl-D-glucosamine was purchased from Sigma-Aldrich.

### 7.2. Acceptor reaction assay

The glucosylation reaction was performed in the enzyme optimal buffer: in Tris-HCl (50 mM, pH = 7.5) for AS assay. The reaction mixture was carried out at 30 °C with sucrose and acceptor in equimolar ratio (146 mM). AS were used at 1 U/mL. Activity one unit is defined as the amount of enzyme that catalyzes the formation of 1µmol of fructose/min at 30°C, in enzyme buffer and sucrose at a concentration of 146 mM. The reaction was stopped by heating at 95°C for 5 min. The final mixture was centrifuged at 18 000 g for 10 min and filtered on a 0.22 µm membrane before HPLC analysis.

### 7.3. Glucosyl acceptor production

In order to characterize glucosylated products of *N*-acetyl-D-glucosamine, acceptor reactions were conducted at preparative scale.

P2 from N-acetyl-D-glucosamine glucosylation by purified AS (1 U/mL) was produced in 100 mL mixture reaction (292 mM in sucrose, 730 mM in acceptor).

After a 24 h reaction time at 30°C, the media were centrifugated at 4800 rpm, for 20 min at 4°C to remove proteins and filtered for a better clarification. The purification of the glucosylated products was performed on a preparative octadecyl reverse-phase chromatography column (C18 column) (Bischoff Chromatography). Ultra pure water was used as eluent at a constant flow rate of 50 mL/min. Glucosyl detection was carried out with a refractometer, and each peak was collected separately, concentrated and reinjected into an analytical HPLC system to check the purity of the compounds.

### 7.4. Analytical methods

### 7.4.a. High Performance Liquid Chromatography (HPLC)

HPLC analysis device consisted in a Dionex P 680 series pump, a Shodex RI 101 series refractometer, a Dionex UVD 340 UV/Vis detector and an autosampler HTC PAL. Five columns were employed to separate the acceptor reaction products and to determine the acceptor conversion rate and product yields (i) a Biorad HPLC Carbohydrate Analysis columns: AMINEX HPX-87C at 80°C (elution with ultra-pure water at 0.6 mL/min) (ii) HPX-87K columns (300 x 7.8 mm) at 65°C (elution with ultra-pure water at 0.6 mL/min) (iii) C 18 column Bischoff Prontosil Eurobond, 5 µm (elution with ultra pure water at room temperature and 1mL/min) (iv) C30: Bischoff Prontosil Eurobond, 5 µm, 250 x 4.0 mm (elution with ultra pure water at room temperature and 1mL/min) (v) C18RP: Sinergi Fusion RP Phenomenex, 4µm, 250 x 4.6 mm (elution with ultra pure water at room temperature and 1 mL/min).

### 7.4.b. High Resolution Mass Spectrometry (HRMS) and Nuclear Magnetic Resonance (NMR)

Accurate mass determination was carried out using an Autospec mass spectrometer arranged in an EBE geometry (Micromass, Manchester, UK). The instrument was operated at 8 kV accelerating voltage in positive mode. The caesium gun was set to 35 keV energy and 1 µL of sample was mixed in the tip of the probe with a glycerol or dithiothreitol/dithioerythritol matrix.

NMR analyses: ¹H (400.130 MHz), ¹³C (100.612 MHz), HSQC and HMBC were registered on a Bruker-ARX 400 spectrometer equipped with an ultrashim system. Samples were disolved in deuterium oxide at c.a. 80 g/L and experiments were performed at 300K.

### REFERENCES

1. Gabius, H.J. et al., Chembiochem 5, 740-764 (2004).
2. Ruffing, A. & Chen, R.R. Microbial cell factories 5, 25 (2006).
3. Persidis, A. Nature biotechnology 15, 479-480 (1997).
4. Seeberger, P.H. & Werz, D.B. Nature 446, 1046-1051 (2007).
5. Kilcoyne, M. & Joshi, L. Cardiovascular & hematological agents in medicinal chemistry 5, 186-197 (2007).
6. Lee, J.C. et al., Nature protocols 1, 3143-3152 (2006).
7. Plante, O.J. et al., Science (New York, N.Y) 291, 1523-1527 (2001).
8. Wang, C.C. et al., Nature 446, 896-899 (2007).
9. Joe, M. et al., Journal of the American Chemical Society 129, 9885-9901 (2007).
10. Drouillard, S. et al., Angewandte Chemie (International ed) 45, 1778-1780 (2006).
11. Lindberg, A.A., Vaccine 17 Suppl 2, S28-36 (1999).
12. Ada, G. & Isaacs, Clin Microbiol Infect 9, 79-85 (2003).
13. Pirofski, L.A., Trends in microbiology 9, 445-451 (2001).
14. Nyame, A.K. et al., Archives of biochemistry and biophysics 426, 182-200 (2004).
15. Ragupathi, G., Cancer Immunol Immunother 43, 152-157 (1996).
16. Pozsgay, V., Advances in carbohydrate chemistry and biochemistry 56, 153-199 (2000).
17. Verez-Bencomo, V. et al., Science (New York, N.Y) 305, 522-525 (2004).
18. Thiem, J., FEMS microbiology reviews 16, 193-211 (1995).
19. Koeller, K.M. & Wong, C.H., Nature 409, 232-240 (2001).
20. Yan, F. et al., Carbohydrate research 328, 3-16 (2000).
21. Hanson, S. et al., Trends Biochem Sci 29, 656-663 (2004).
22. Crout, D.H. & Vic, G., Current opinion in chemical biology 2, 98-111 (1998).
23. Schroven, A. et al., Chemistry (2007).
24. Schmidt, D. et al., J Org Chem 65, 8518-8526 (2000).
25. Yoon, J.H. & Ajisaka, K., Carbohydrate research 292, 153-163 (1996).
26. Hancock, S.M. et al., Current opinion in chemical biology 10, 509-519 (2006).
27. Jank, T. et al., The Journal of biological chemistry 280, 37833-37838 (2005).
28. Zhang, J.H. et al., Proceedings of the National Academy of Sciences of the United States of America 94, 4504-4509 (1997).
29. Parikh, M.R. & Matsumura, I., Journal of molecular biology 352, 621-628 (2005).
30. Osanjo, G. et al., Biochemistry 46, 1022-1033 (2007).
31. Hancock, S.M. et al., Chembiochem 6, 866-875 (2005).
32. Kim, Y.W. et al., The Journal of biological chemistry 279, 42787-42793 (2004).
33. Honda, Y. & Kitaoka, M., The Journal of biological chemistry 281, 1426-1431 (2006).
34. Niggemann, J. et al., Bioorg Med Chem 6, 1605-1612 (1998).
35. Eichler, E. et al., Carbohydrate research 319, 1-16 (1999).
36. Mehta, S. et al., Org Lett 2, 751-753 (2000).
37. Yan, F. et al., J Org Chem 68, 2426-2431 (2003).
38. Yan, F. et al., Org Lett 3, 3265-3268 (2001).
39. Kotloff, K.L. et al., Bulletin of the World Health Organization 77, 651-666 (1999).
40. Levine, M.M. et al., Nature reviews 5, 540-553 (2007).
41. Lindberg, A.A. et al., Reviews of infectious diseases 13 Suppl 4, S279-284 (1991).
42. Jennison, A.V. & Verma, N.K., FEMS microbiology reviews 28, 43-58 (2004).
43. Phalipon, A. et al., J Immunol 176, 1686-1694 (2006).
44. Simmons, D.A., Biochemical Society transactions 18, 1271-1272 (1990).
45. De Montalk, G.P. et al., Journal of bacteriology 181, 375-381 (1999).
46. Albenne, C. et al., The Journal of biological chemistry 279, 726-734 (2004).
47. Yoon, S.H. & Robyt, J.F., Carbohydrate research 337, 2427-2435 (2002).
48. Cote, G.L. et al., Carbohydrate research 340, 257-262 (2005).
49. Richard, G. et al., Carbohydrate research 340, 395-401 (2005).
50. Demuth, K. et al., Carbohydrate research 337, 1811-1820 (2002).
51. Coutinho, P.M. & Henrissat, B., in Recent Advances in Carbohydrate Bioengineering. (eds. H.J. Gilbert, G. Davies, B. Henrissat & B. Svensson) 3-12 (The Royal Society of Chemistry, Cambridge; 1999).
53. Skov, L.K. et al., Acta Crystallogr D Biol Crystallogr 56, 203-205 (2000).
54. Mirza, O. et al., Biochemistry 40, 9032-9039 (2001).
55. Skov, L.K. et al., The Journal of biological chemistry 277, 47741-47747 (2002).
56. Bradford, M.M., Analytical biochemistry 72, 248-254 (1976).
57. Sumner, J. & Howell, S.A., J. Biol. Chem 108, 51-54 (1935).
58. Clement, M.J. et al., The Journal of biological chemistry 278, 47928-47936 (2003).
59. West, N.P. et al., Science 307, 1313-1317 (2005).
60. Allison, G.E. & Verma, N.K., Trends in microbiology 8, 17-23 (2000).
61. Ovodov, Y.S., Biochemistry 71, 937-954 (2006).
62. Davies, G. & Henrissat, B., Structure 3, 853-9 (1995).
63. Henrissat, B., Biochem J 280, 309-16 (1991).
64. Henrissat, B. & Bairoch, A., Biochem J 293, 781-8 (1993).
65. Stam, M. R. et al., Protein Eng Des Sel. 19, 555-62 (2006).
66. Lemanski, G. & Ziegler, T., Eur. J Org. Chem. 2618-2630 (2006).
67. Rye C.S. & Withers S.G., Curr Opin Chem Biol 4, 573-80 (2000).
68. Skov, L. et al., J Biol Chem 276, 25273-8 (2001).
69. Ghosh M. et al., JOC 65, 8387-8390 (2000).
70. Cai, Y. et al., Org. Lett. 7, 4021-4024 (2005).
71. McGeary, R. P. et al., J Org. Chem. 66, 5102 - 5105 (2001).
72. Blatter, G. & Beau, J.M., Carbohydr. Res. 260, 189-202 (1994).
73. Poszgay, V., J. Org. Chem. 63, 5983-5999 (1998).
74. Palomino, J. et al., J. Carbohydr. Chem. 15, 137-146 (1996).
75. Feng, F. et al., Org. Biomol. Chem. 2, 1617-1623 (2004).
76. Westerduin, P. et al., Carbohydr. Res. 180, 195-206 (1988).
77. Cai, J. et al., Carbohydr. Res. 300, 109-118 (1997).
78. Cheng, M. S. et al., J Org. Chem. 68, 3658-3662 (2003).
79. Damager, I. et al., Carbohydr. Res. 320, 19- 30 (1999).
80. Blom, P. et al., J Org. Chem. 70, 10109-10112 (2005).

## Claims

1. A method for preparing the building block corresponding to a disaccharide α-D-glucopyranosyl-(1→4)-*N*-acetyl-β-D-glucopyranosaminyl of formula (Ia): 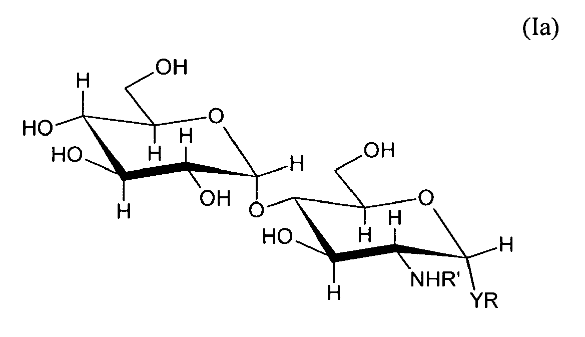 said method being **characterized in that** it comprises the step of reacting:
A) a mutant of a glycoside hydrolase,
• wherein said wild type glycoside hydrolase has 450 to 850 amino acids and comprises eleven motifs defined by the following consensus motifs:
(1) the amino acid sequence LGVNYLHLMPL (SEQ ID NO: 1), which is located in the β-strand 2 of said wild type glycoside hydrolase;
(2) the amino acid sequence DGGYAV (SEQ ID NO: 2), which is located in the loop 2 of the (β/α)₈-barrel of said wild type glycoside hydrolase;
(3) the amino acid sequence DFVFNH (SEQ ID NO: 3) which is located in the β-strand 3 of said wild type glycoside hydrolase;
(4) the amino acid sequence LREIFPDTAPGNF (SEQ ID NO: 4), which is located in the domain B of said wild type glycoside hydrolase;
(5) the amino acid sequence FNSYQWDLN (SEQ ID NO: 5), which is located in the C-terminal part of the domain B of said wild type glycoside hydrolase;
(6) the amino acid sequence ILRLDAVAFLWK (SEQ ID NO: 6), which is located in the β-strand 4 of said wild type glycoside hydrolase;
(7) the amino acid sequence EAIV (SEQ ID NO: 7), which is located in the β-stand 5 of said wild type glycoside hydrolase;
(8) the amino acid sequence YVRCHDDI (SEQ ID NO: 8), which is located in the β-strand 7 of said wild type glycoside hydrolase;
(9) the amino acid sequence RISGTLASLAG (SEQ ID NO: 9), which is located in the domain B' of said wild type glycoside hydrolase;
(10) the amino acid sequence GIPLIYLGDE (SEQ ID NO: 10), which is located in the β-strand 8 of said wild type glycoside hydrolase;
(11) the amino acid sequence RWVHRP (SEQ ID NO: 11), which is located in the loop 8 of the (β/α)₈-barrel,
and the sequence formed by said eleven motifs from said wild type glycoside hydrolase joined end-to-end from motif (1) to motif (11) has at least 65% sequence identity or at least 80% sequence similarity with the amino acid sequence SEQ
ID NO: 12;
• wherein said mutant has a mutation consisting of:
- the substitution of the amino acid residue at position 4 in said motif (4) with any amino acid selected from the group consisting of alanine (A), cysteine (C), glutamic acid (E), glycine (G), histidine (H), leucine (L), methionine (M), asparagine (N), proline (P), glutamine (Q), serine (S), threonine (T), valine (V) with the provisio that said wild type glycoside hydrolase does not contain a valine at this position, tryptophan (W) and tyrosine (Y), or
- the substitution of the amino acid residue at position 8 in said motif (6) with any amino acid selected from the group consisting of glutamic acid (E), phenylalanine (F), glycine (G), lysine (K), leucine (L), methionine (M), proline (P), glutamine (Q), arginine (R) and valine (V), or
- the substitution of the amino acid residue at position 9 in said motif (6) with any amino acid selected from the group consisting of alanine (A), cysteine (C), aspartic acid (D), glutamic acid (E), glycine (G), histidine (H), isoleucine (I), lysine (K), leucine (L), methionine (M), proline (P), glutamine (Q), arginine (R), serine (S), threonine (T), valine (V) and tryptophan (W), or
- the substitution of the amino acid residue at position 4 in said motif (7) with any amino acid selected from the group consisting of alanine (A), cysteine (C), aspartic acid (D), glycine (G), histidine (H), isoleucine (I), leucine (L), methionine (M), asparagine (N), serine (S), threonine (T) and tyrosine (Y), or
- the substitution of the amino acid residue at position 7 in said motif (8) with any amino acid selected from the group consisting of alanine (A) and valine (V), or
- the substitution of the amino acid residue at position 1 in said motif (9) with any amino acid selected from the group consisting of alanine (A), cysteine (C), phenylalanine (F), glycine (G) with the provisio that said wild type glycoside hydrolase does not contain a glycine at this position, lysine (K), asparagine (N), glutamine (Q), serine (S) with the provisio that said wild type glycoside hydrolase does not contain a serine at this position, threonine (T) and tryptophan (W);
B) with the acceptor of formula (IIa): 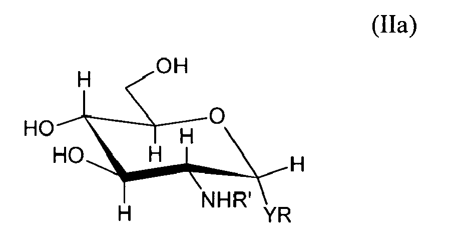 wherein Y is selected from -O- and -S- and R is selected from the group consisting of: C₁-C₆ alkyl, C₁-C₆ alkenyl, aryl, allyl, -CO-alkyl (C₁-C₆), -CO-alkenyl (C₁-C₆), -CO-aryl,
R' designates a group selected from: acetyl, trichloroacetyl, trifluoroacetyl,
and
C) with a donor of formula (IIIa) : 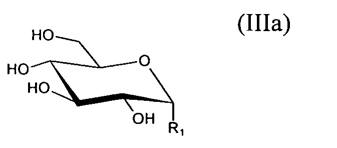
wherein R₁ represents a group selected from:

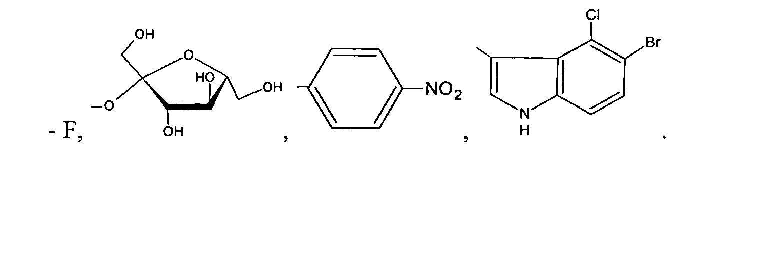

2. A method according to claim 1, **characterized in that** said wild type glycoside hydrolase is an amylosucrase (EC 2.4.1.4) or sucrose hydrolase (EC 3.2.1.-).

3. A method according to claim 2, **characterized in that** said wild type glycoside hydrolase is an amylosucrase from *Neisseria polysaccharea*, and is preferably selected from the group consisting of 1G5A (SEQ ID NO: 13), 1ZS2, 1MVY, 1MW0, 1S46, 1JGI, 1MW2, 1MW3, 1MW1 and 1JG9 proteins.

4. A method according to anyone of claims 1 to 3, for the preparation of the disaccharide α-D-glucopyranosyl-(1→4)-*N*-acetyl-β-D-glucopyranosaminyl of formula (I): 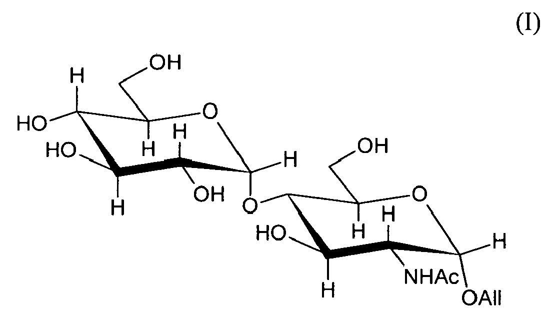 said method being **characterized in that** it comprises the step of reacting a mutant of a glycoside hydrolase as disclosed in anyone of claims 1 to 3, with the acceptor of formula (II): 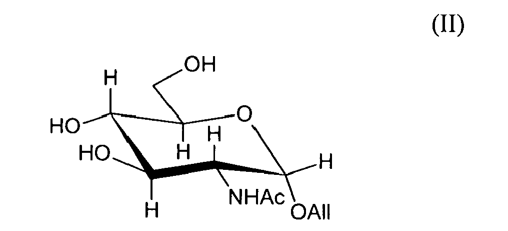 and with a donor sucrose of formula (III):

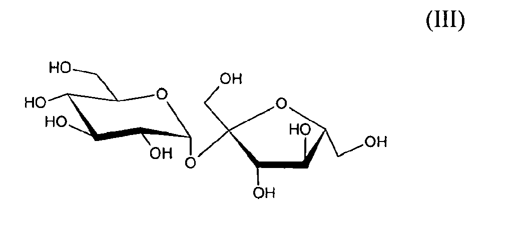

5. A method for the preparation of the building block corresponding to the disaccharide of formula (XX_{3B}) in which R₂ represents a group selected from AcBn 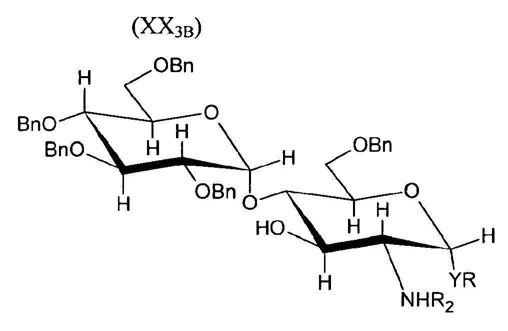 comprising at least one step according to claim 1 or claim 4.

6. A method according to claim 5, for the preparation of the disaccharide of formula (XX_{3A})

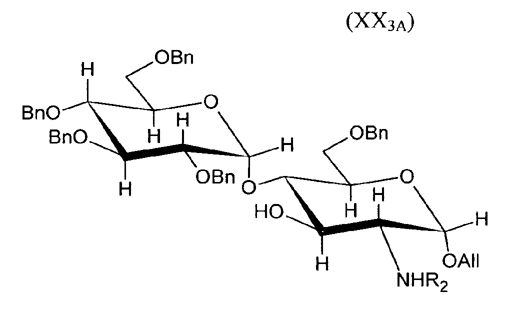

7. A mutant of a wild type glycoside hydrolase, wherein said wild type glycoside hydrolase has 450 to 850 amino acids and comprises eleven motifs defined by the following consensus motifs:
(1) the amino acid sequence LGVNYLHLMPL (SEQ ID NO: 1), which is located in the β-strand 2 of said wild type glycoside hydrolase;
(2) the amino acid sequence DGGYAV (SEQ ID NO: 2), which is located in the loop 2 of the (β/α)₈-barrel of said wild type glycoside hydrolase;
(3) the amino acid sequence DFVFNH (SEQ ID NO: 3) which is located in the β-strand 3 of said wild type glycoside hydrolase;
(4) the amino acid sequence LREIFPDTAPGNF (SEQ ID NO: 4), which is located in the domain B of said wild type glycoside hydrolase;
(5) the amino acid sequence FNSYQWDLN (SEQ ID NO: 5), which is located in the C-terminal part of the domain B of said wild type glycoside hydrolase;
(6) the amino acid sequence ILRLDAVAFLWK (SEQ ID NO: 6), which is located in the β-stand 4 of said wild type glycoside hydrolase;
(7) the amino acid sequence EAIV (SEQ ID NO: 7), which is located in the β-strand 5 of said wild type glycoside hydrolase;
(8) the amino acid sequence YVRCHDDI (SEQ ID NO: 8), which is located in the β-strand 7 of said wild type glycoside hydrolase;
(9) the amino acid sequence RISGTLASLAG (SEQ ID NO: 9), which is located in the domain B' of said wild type glycoside hydrolase;
(10) the amino acid sequence GIPLIYLGDE (SEQ ID NO: 10), which is located in the β-strand 8 of said wild type glycoside hydrolase;
(11) the amino acid sequence RWVHRP (SEQ ID NO: 11), which is located in the loop 8 of the (β/α)₈-barrel,
and the sequence formed by said eleven motifs from said wild type glycoside hydrolase joined end-to-end from motif (1) to motif (11) has at least 65% sequence identity or at least 80% sequence similarity with the amino acid sequence SEQ ID NO: 12;
**characterized in that** said mutant has a mutation consisting of:
- the substitution of the amino acid residue at position 4 in said motif (4) with any amino acid selected from the group consisting of alanine (A), cysteine (C), glutamic acid (E), glycine (G), histidine (H), leucine (L), methionine (M), asparagine (N), proline (P), glutamine (Q), serine (S), threonine (T), valine (V), tryptophan (W) and tyrosine (Y), or
- the substitution of the amino acid residue at position 8 in said motif (6) with any amino acid selected from the group consisting of glutamic acid (E), phenylalanine (F), glycine (G), lysine (K), leucine (L), methionine (M), proline (P), glutamine (Q), arginine (R) and valine (V), or
- the substitution of the amino acid residue at position 9 in said motif (6) with any amino acid selected from the group consisting of alanine (A), cysteine (C), aspartic acid (D), glutamic acid (E), glycine (G), histidine (H), isoleucine (I), lysine (K), leucine (L), methionine (M), proline (P), glutamine (Q), arginine (R), serine (S), threonine (T), valine (V) and tryptophan (W), or
- the substitution of the amino acid residue at position 4 in said motif (7) with any amino acid selected from the group consisting of alanine (A), cysteine (C), aspartic acid (D), glycine (G), histidine (H), isoleucine (I), leucine (L), methionine (M), asparagine (N), serine (S), threonine (T) and tyrosine (Y), or
- the substitution of the amino acid residue at position 7 in said motif (8) with any amino acid selected from the group consisting of alanine (A) and valine (V), or
- the substitution of the amino acid residue at position 1 in said motif 9 with any amino acid selected from the group consisting of alanine (A), cysteine (C), phenylalanine (F), glycine (G), lysine (K), asparagine (N), glutamine (Q), serine (S), threonine (T) and tryptophan (W).

8. A mutant according to claim 7, **characterised in that** the amino acid residue at position 9 in said motif (6) is substituted with any amino acid selected from the group consisting of cysteine (C), aspartic acid (D), isoleucine (I), lysine (K) and glutamine (Q), and more preferably with any amino acid selected from the group consisting of aspartic acid (D) and lysine (K).

9. A mutant according to claim 7 or claim 8, **characterized in that** said wild type glycoside hydrolase is an amylosucrase (EC 2.4.1.4) or sucrose hydrolase (EC 3.2.1.-).

10. A mutant according to claim 9, **characterized in that** said wild type glycoside hydrolase is an amylosucrase from *Neisseria polysaccharea*, and is preferably selected from the group consisting of 1G5A, 1ZS2, 1MVY, 1MW0, 1S46, 1JGI, 1MW2, 1MW3, 1MW1 and 1JG9 proteins.

11. A polynucleotide encoding a mutant of a glycoside hydrolase of anyone of claims 7 to 10.

12. A recombinant vector comprising a polynucleotide of claim 11.

13. A method for determining whether a wild type protein is a wild type glycoside hydrolase, said method comprising the steps of:
a) determining the amino acid sequence of said protein,
b) identifying in the amino acid sequence of said protein, preferably from the N- to C-terminus, eleven motifs defined by the following consensus motifs:
(1) the amino acid sequence LGVNYLHLMPL (SEQ ID NO: 1);
(2) the amino acid sequence DGGYAV (SEQ ID NO: 2);
(3) the amino acid sequence DFVFNH (SEQ ID NO: 3);
(4) the amino acid sequence LREIFPDTAPGNF (SEQ ID NO: 4);
(5) the amino acid sequence FNSYQWDLN (SEQ ID NO: 5);
(6) the amino acid sequence ILRLDAVAFLWK (SEQ ID NO: 6);
(7) the amino acid sequence EAIV (SEQ ID NO: 7);
(8) the amino acid sequence YVRCHDDI (SEQ ID NO: 8);
(9) the amino acid sequence RISGTLASLAG (SEQ ID NO: 9)
(10) the amino acid sequence GIPLIYLGDE (SEQ ID NO: 10);
(11) the amino acid sequence RWVHRP (SEQ ID NO: 11); determining the sequence identity percent or sequence similarity percent between the sequence formed by said eleven motifs joined end-to-end from motif (1) to motif (11) with the amino acid sequence SEQ ID NO: 12, and if the sequence identity percent is at least 65%, or if the sequence similarity percent is at least 80%, then the wild type protein is a wild type glycoside hydrolase.

14. A molecule selected from the following list:
- Allyl α-D-glucopyranosyl-(1→4)-α-D-glucopyranosyl-(1→4)-2-acetamido-2-deoxy-α-D-glucopyranoside (XX₂),
- Allyl α-D-glucopyranosyl-(1→4)-2-acetamido-2-deoxy-α-D-glucopyranoside (XX₃),
- Allyl 2,3,4,6-tetra-*O*-acetyl-a-D-glucopyranosyl-(1→4)-2-acetamido-3,6-di-*O*-acetyl-2-deoxy-α-D-glucopyranoside (XX₄),
- Allyl α-D-glucopyranosyl-(1→4)-2-amino-2-deoxy-α-D-glucopyranoside (XX₅),
- Allyl α-D-glucopyranosyl-(1→4)-2-amino-2-N,3-*O*-carbonyl-2-deoxy-α-D-glucopyranoside (XX₆),
- Allyl 2,4,6-tri-*O*-benzyl-α-D-glucopyranosyl-(1→4)-6-*O*-benzyl-2-benzylamino-2-*N*,3-*O*-carbonyl-2-deoxy-α-D-glucopyranoside (XX₇),
- Allyl 2,3,4,6-tetra-*O*-benzyl-α-D-glucopyranosyl-(1→4)-6-*O*-benzyl-2-benzylamino-2-*N*,3-*O*-carbonyl-2-deoxy-α-D-glucopyranoside (XX₈),
- Allyl 2,3,4,6-tetra-*O*-benzyl-α-D-glucopyranosyl-(1→4)-6-*O*-benzyl-2-amino-2-*N*,3-*O*-carbonyl-2-deoxy-α-D-glucopyranoside (XX_{8A}),
- Allyl 2,3,4,6-tetra-*O*-benzyl-α-D-glucopyranosyl-(1→4)-6-*O*-benzyl-2-benzylamino-2-deoxy-α-D-glucopyranoside (XX₉),
- Allyl 2,3,4,6-tetra-*O*-benzyl-α-D-glucopyranosyl-(1→4)-6-*O*-benzyl-2-benzylacetamido-2-deoxy-α-D-glucopyranoside (XX₁₀),
- Allyl 2-*O*-benzoyl-4-*O*-benzyl-3-*O*-chloroacetyl-α-L-rhamnopyranosyl-(1→3)-[2,3,4,6-tetra-*O*-benzyl-α-D-glucopyranosyl-(1→4)]-6-*O-*benzyl-2-benzylacetamido-2-deoxy-α-D-glucopyranoside (XX₁₁),
- Allyl 2-*O*-acetyl-3,4-di-*O*-benzyl-α-L-rhamnopyranosyl-(1→3)-[2,3,4,6-tetra-*O*-benzyl-α-D-glucopyranosyl-(1→4)]-6-*O*-benzyl-2-benzylacetamido-2-deoxy-α-D-glucopyranoside (XX₁₂),
- Allyl α-D-glucopyranosyl-(1→4)-2-deoxy-2-trichloroacetamido-α-D-glucopyranoside (XX₁₃),
- Allyl 2,3,4,6-tetra-*O*-acetyl-α-D-glucopyranosyl-(1→4)-3,6-di-*O-*acetyl-2-deoxy-2-trichloroacetamido-α-D-glucopyranoside (XX₁₄),
- 2,3,4,6-tetra-*O*-acetyl-α-D-glucopyranosyl-(1→4)-3,6-di-*O*-acetyl-2-deoxy-2-trichloroacetamido-α-D-glucopyranose (XX₁₅),
- 2,3,4,6-Tetra-*O*-acetyl-α-D-glucopyranosyl-(1→4)-3,6-di-*O*-acetyl-2-deoxy-2-trichloroacetamido-α-D-glucopyranosyl trichloroacetimidate (XX₁₆),
- Allyl 2,3,4,6-tetra-O-acetyl-α-D-glucopyranosyl-(1→4)-3,6-di-*O-*acetyl-2-deoxy-2-trichloroacetamido-α-D-glucopyranosyl-(1→2)-3,4-di-*O*-benzyl-α-L-rhamnopyranoside (XX₁₇),
- Allyl 2,3,4,6-tetra-*O*-benzyl-α-D-glucopyranosyl-(1→4)-2-acetamido-3-*O*-acetyl-6-*O*-benzyl-2-deoxy-α-D-glucopyranoside (XX₂₄),
- Allyl 2,3,4,6-tetra-*O*-benzyl-α-D-glucopyranosyl-(1→4)-2-acetamido-6-*O*-benzyl-2-deoxy-α-D-glucopyranoside (XX₂₅).
